(19) Europäisches Patentamt
European Patent Office
Office européen des brevets

(11) EP 2 638 153 B1

(12) **EUROPEAN PATENT SPECIFICATION**

(45) Date of publication and mention of the grant of the patent:
**12.07.2017 Bulletin 2017/28**

(21) Application number: **11839617.5**

(22) Date of filing: **11.11.2011**

(51) Int Cl.:
***C12N 9/46*** (2006.01) ***C12N 15/56*** (2006.01)
***C12N 15/64*** (2006.01) ***C12P 19/00*** (2006.01)
***C12N 9/42*** (2006.01) ***C12P 19/14*** (2006.01)
***C12P 7/14*** (2006.01)

(86) International application number:
**PCT/CN2011/082094**

(87) International publication number:
**WO 2012/062220 (18.05.2012 Gazette 2012/20)**

(54) **POLYPEPTIDES HAVING ENDOGLUCANASE ACTIVITY AND POLYNUCLEOTIDES ENCODING SAME**

POLYPEPTIDE MIT ENDOGLUCANASE-AKTIVITÄT UND POLYNUKLEOTIDE ZU IHRER KODIERUNG

POLYPEPTIDES AYANT UNE ACTIVITÉ ENDOGLUCANASE ET POLYNUCLÉOTIDES CODANT POUR CEUX-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.11.2010 PCT/CN2010/078675**

(43) Date of publication of application:
**18.09.2013 Bulletin 2013/38**

(73) Proprietor: **Novozymes Inc.**
**Davis, CA 95616 (US)**

(72) Inventors:
- **LIU, Ye**
**Beijing 100044 (CN)**
- **DUAN, Junxin**
**Beijing 100089 (CN)**
- **TANG, Lan**
**Beijing 100080 (CN)**

(74) Representative: **Rasmussen, Preben**
**Novozymes A/S**
**Patents**
**Krogshoejvej 36**
**2880 Bagsvaerd (DK)**

(56) References cited:
**WO-A1-2009/111706 JP-A- 2010 148 427**

- **DATABASE GENBANK [Online] 29 October 2008 XP003031824 Database accession no. EEA18584**
- **DATABASE GENBANK [Online] 22 December 2008 XP003031825 Database accession no. EED20864**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 638 153 B1

SEQUENCE LISTING

<110> Novozymes A/S
Tang, Lan
Liu, Ye
Duan, Junxin

<120> Polypeptides having endoglucanase activity and polynucleotides encoding same

<130> 12038-WO-PCT[2]

<150> PCT/CN2010/078675
<151> 2010-11-12

<150> US 61/434,159
<151> 2011-01-19

<160> 34

<170> PatentIn version 3.5

<210> 1
<211> 1497
<212> DNA
<213> Penicillium pinophilum

<400> 1

atgtctaaca tggcgactag accattggct tttgcagcta ttgctgctct tttccaccat 60
gccgcctcac agcaggcccc taccccagat aatttagctt ctctaccgac ctggaaatgt 120
acaacttccg gcggctgtgt tcaacagtcg acctctattg tcgtggattg ggtgtatcac 180
tggatccaca cagtcaatgg gagcacatcg tgcaccacat ctagcggatt ggacccaact 240
ttatgtggaa cggaagagga atgctataca aactgtgaaa tctcacctgc aacctacgat 300
ggcctcggta taaaaacttc tggaaacgct ttaaccctca atcaatacgt cacaagcaac 360
ggaacgacaa gtaacgcctc tccgcgtgta tatcttttgg atcccgccgg caagaattat 420
gagatgctgc agctcctcgg tcaagagatt agctttgacg tagatgcctc caatttacca 480
tgtggcgaaa acggggctct ttatctctct gagatggatg cgactggagg tcgaagccag 540
tacaaccctg ccggagcttc atacggttcc ggttactgtg atgctcagtg tggaagtagc 600
agctggttta atggctcgat taatagcgct ggccttggtt cttgctgtaa cgaaatggat 660
ctctgggaag caaatggcga ggcaactgct ttgacacctc atccatgcag tgtcgatggt 720
ccttatggct gctctggtag cgcctgtggt tcgactggag tgtgtgacaa gaatggttgc 780
ggattcaatc catatgccct tggaaatcac agctactacg gcccaggtct tacagtggac 840
acaagcaagc cctttacagt tacgacacag tttgtgacca acgatggcac caagaccggc 900
accctgaccg aaattcgtcg atcttacact cagaatggca aggttattgc gaatgccgtt 960
gcatcctctt cgtcggggtt ttcaggtcaa agttctatca cagagtcctt ctgtactgcg 1020
atggactccg aagccgggac actgggtggt ctgactacaa tgggtgaggc ccttggccgt 1080
ggcatggttc ttatcttcag catttggaat gatgcaggtg gatacatgaa ctggctggat 1140
agtggtagct cgggcccttg cagtagtact gcaggaattc cgtccaccat tcaggcgaat 1200
gaccccggta cttcggttac tttctcaaac atcaagtggg gtgatattgg atctacaggg 1260
tctggcactg gaggaagcag ttcatcatcg tcgtcaactt cgacctcacc aaaaactacc 1320
agcaccacaa caacatcagc aacgaccaaa acatcagcaa cgacaactac aaccagcaca 1380
ggggtaactc agactcacta tggtcaatgt ggaggcatgt attatactgg tcctactgtt 1440
tgtgcctctc cgtacacctg tcaagtacag aatccgtact attcacagtg cctttag 1497

<210> 2
<211> 498
<212> PRT
<213> Penicillium pinophilum

<400> 2

```
Met Ser Asn Met Ala Thr Arg Pro Leu Ala Phe Ala Ala Ile Ala Ala
1               5                   10                  15

Leu Phe His His Ala Ala Ser Gln Gln Ala Pro Thr Pro Asp Asn Leu
            20                  25                  30

Ala Ser Leu Pro Thr Trp Lys Cys Thr Thr Ser Gly Gly Cys Val Gln
        35                  40                  45

Gln Ser Thr Ser Ile Val Val Asp Trp Val Tyr His Trp Ile His Thr
    50                  55                  60

Val Asn Gly Ser Thr Ser Cys Thr Thr Ser Ser Gly Leu Asp Pro Thr
65                  70                  75                  80

Leu Cys Gly Thr Glu Glu Glu Cys Tyr Thr Asn Cys Glu Ile Ser Pro
                85                  90                  95

Ala Thr Tyr Asp Gly Leu Gly Ile Lys Thr Ser Gly Asn Ala Leu Thr
            100                 105                 110

Leu Asn Gln Tyr Val Thr Ser Asn Gly Thr Thr Ser Asn Ala Ser Pro
        115                 120                 125

Arg Val Tyr Leu Leu Asp Pro Ala Gly Lys Asn Tyr Glu Met Leu Gln
    130                 135                 140

Leu Leu Gly Gln Glu Ile Ser Phe Asp Val Asp Ala Ser Asn Leu Pro
145                 150                 155                 160
```

```
Cys Gly Glu Asn Gly Ala Leu Tyr Leu Ser Glu Met Asp Ala Thr Gly
                165                 170                 175

Gly Arg Ser Gln Tyr Asn Pro Ala Gly Ala Ser Tyr Gly Ser Gly Tyr
            180                 185                 190

Cys Asp Ala Gln Cys Gly Ser Ser Ser Trp Phe Asn Gly Ser Ile Asn
        195                 200                 205

Ser Ala Gly Leu Gly Ser Cys Cys Asn Glu Met Asp Leu Trp Glu Ala
    210                 215                 220

Asn Gly Glu Ala Thr Ala Leu Thr Pro His Pro Cys Ser Val Asp Gly
225                 230                 235                 240

Pro Tyr Gly Cys Ser Gly Ser Ala Cys Gly Ser Thr Gly Val Cys Asp
                245                 250                 255

Lys Asn Gly Cys Gly Phe Asn Pro Tyr Ala Leu Gly Asn His Ser Tyr
            260                 265                 270

Tyr Gly Pro Gly Leu Thr Val Asp Thr Ser Lys Pro Phe Thr Val Thr
        275                 280                 285

Thr Gln Phe Val Thr Asn Asp Gly Thr Lys Thr Gly Thr Leu Thr Glu
    290                 295                 300

Ile Arg Arg Ser Tyr Thr Gln Asn Gly Lys Val Ile Ala Asn Ala Val
305                 310                 315                 320

Ala Ser Ser Ser Ser Gly Phe Ser Gly Gln Ser Ser Ile Thr Glu Ser
                325                 330                 335

Phe Cys Thr Ala Met Asp Ser Glu Ala Gly Thr Leu Gly Gly Leu Thr
            340                 345                 350

Thr Met Gly Glu Ala Leu Gly Arg Gly Met Val Leu Ile Phe Ser Ile
        355                 360                 365

Trp Asn Asp Ala Gly Gly Tyr Met Asn Trp Leu Asp Ser Gly Ser Ser
    370                 375                 380

Gly Pro Cys Ser Ser Thr Ala Gly Ile Pro Ser Thr Ile Gln Ala Asn
385                 390                 395                 400

Asp Pro Gly Thr Ser Val Thr Phe Ser Asn Ile Lys Trp Gly Asp Ile
                405                 410                 415
```

```
Gly Ser Thr Gly Ser Gly Thr Gly Gly Ser Ser Ser Ser Ser Ser Ser
            420                     425                     430

Thr Ser Thr Ser Pro Lys Thr Thr Ser Thr Thr Thr Thr Ser Ala Thr
        435                     440                     445

Thr Lys Thr Ser Ala Thr Thr Thr Thr Thr Ser Thr Gly Val Thr Gln
    450                     455                     460

Thr His Tyr Gly Gln Cys Gly Gly Met Tyr Tyr Thr Gly Pro Thr Val
465                     470                     475                     480

Cys Ala Ser Pro Tyr Thr Cys Gln Val Gln Asn Pro Tyr Tyr Ser Gln
                485                     490                     495

Cys Leu
```

<210> 3
<211> 34
<212> DNA
<213> Artificial Sequence

<220>
<223> Synthetic Construct

<400> 3
tcgcgatccg ttttcgcatt tatcgtgaaa cgct 34

<210> 4
<211> 33
<212> DNA
<213> Artificial Sequence

<220>
<223> Synthetic Construct

<400> 4
ccgcaaacgc tggtgaaagt aaaagatgct gaa 33

<210> 5
<211> 20
<212> DNA
<213> Artificial Sequence

<220>
<223> Synthetic Construct

<400> 5
agcgtttgcg gccgcgatcc 20

<210> 6
<211> 21
<212> DNA
<213> Artificial Sequence

<220>
<223> Synthetic Construct

<400> 6
ttattcggtc gaaaaggatc c 21

<210> 7
<211> 46
<212> DNA
<213> Artificial Sequence

<220>
<223> Synthetic Construct

<400> 7
acacaactgg ggatccacca tgtctaacat ggcgactaga ccattg 46

<210> 8
<211> 48
<212> DNA
<213> Artificial Sequence

<220>
<223> Synthetic Construct

<400> 8
gtcaccctct agatcttcaa aggcactgtg aatagtacgg attctgta 48

<210> 9
<211> 1599
<212> DNA
<213> Aspergillus fumigatus

<400> 9

```
atgctggcct ccaccttctc ctaccgcatg tacaagaccg cgctcatcct ggccgccctt      60
ctgggctctg gccaggctca gcaggtcggt acttcccagg cggaagtgca tccgtccatg     120
acctggcaga gctgcacggc tggcggcagc tgcaccacca acaacggcaa ggtggtcatc     180
gacgcgaact ggcgttgggt gcacaaagtc ggcgactaca ccaactgcta caccggcaac     240
acctgggaca cgactatctg ccctgacgat gcgacctgcg catccaactg cgcccttgag     300
ggtgccaact acgaatccac ctatggtgtg accgccagcg gcaattccct ccgcctcaac     360
ttcgtcacca ccagccagca gaagaacatt ggctcgcgtc tgtacatgat gaaggacgac     420
tcgacctacg agatgtttaa gctgctgaac caggagttca ccttcgatgt cgatgtctcc     480
aacctcccct gcggtctcaa cggtgctctg tactttgtcg ccatggacgc cgacggtggc     540
atgtccaagt acccaaccaa caaggccggt gccaagtacg gtactggata ctgtgactcg     600
cagtgccctc gcgacctcaa gttcatcaac ggtcaggcca acgtcgaagg gtggcagccc     660
tcctccaacg atgccaatgc gggtaccggc aaccacgggt cctgctgcgc ggagatggat     720
```

```
atctgggagg ccaacagcat ctccacggcc ttcaccccc atccgtgcga cacgcccggc      780
caggtgatgt gcaccggtga tgcctgcggt ggcacctaca gctccgaccg ctacggcggc      840
acctgcgacc ccgacggatg tgatttcaac tccttccgcc agggcaacaa gaccttctac      900
ggccctggca tgaccgtcga caccaagagc aagtttaccg tcgtcaccca gttcatcacc      960
gacgacggca cctccagcgg caccctcaag gagatcaagc gcttctacgt gcagaacggc     1020
aaggtgatcc ccaactcgga gtcgacctgg accggcgtca gcggcaactc catcaccacc     1080
gagtactgca ccgcccagaa gagcctgttc caggaccaga acgtcttcga aaagcacggc     1140
ggcctcgagg gcatgggtgc tgccctcgcc cagggtatgg ttctcgtcat gtccctgtgg     1200
gatgatcact cggccaacat gctctggctc gacagcaact acccgaccac tgcctcttcc     1260
accactcccg gcgtcgcccg tggtacctgc gacatctcct ccggcgtccc tgcggatgtc     1320
gaggcgaacc accccgacgc ctacgtcgtc tactccaaca tcaaggtcgg ccccatcggc     1380
tcgaccttca acagcggtgg ctcgaacccc ggtggcggaa ccaccacgac aactaccacc     1440
cagcctacta ccaccacgac cacggctgga aaccctggcg gcaccggagt cgcacagcac     1500
tatggccagt gtggtggaat cggatggacc ggacccacaa cctgtgccag cccttatacc     1560
tgccagaagc tgaatgatta ttactctcag tgcctgtag                            1599
```

<210> 10
<211> 532
<212> PRT
<213> Aspergillus fumigatus

<400> 10

```
Met Leu Ala Ser Thr Phe Ser Tyr Arg Met Tyr Lys Thr Ala Leu Ile
1               5                   10                  15

Leu Ala Ala Leu Leu Gly Ser Gly Gln Ala Gln Gln Val Gly Thr Ser
            20                  25                  30

Gln Ala Glu Val His Pro Ser Met Thr Trp Gln Ser Cys Thr Ala Gly
        35                  40                  45

Gly Ser Cys Thr Thr Asn Asn Gly Lys Val Val Ile Asp Ala Asn Trp
    50                  55                  60

Arg Trp Val His Lys Val Gly Asp Tyr Thr Asn Cys Tyr Thr Gly Asn
65                  70                  75                  80

Thr Trp Asp Thr Thr Ile Cys Pro Asp Asp Ala Thr Cys Ala Ser Asn
                85                  90                  95
```

```
Cys Ala Leu Glu Gly Ala Asn Tyr Glu Ser Thr Tyr Gly Val Thr Ala
            100                 105                 110

Ser Gly Asn Ser Leu Arg Leu Asn Phe Val Thr Thr Ser Gln Gln Lys
        115                 120                 125

Asn Ile Gly Ser Arg Leu Tyr Met Met Lys Asp Asp Ser Thr Tyr Glu
    130                 135                 140

Met Phe Lys Leu Leu Asn Gln Glu Phe Thr Phe Asp Val Asp Val Ser
145                 150                 155                 160

Asn Leu Pro Cys Gly Leu Asn Gly Ala Leu Tyr Phe Val Ala Met Asp
                165                 170                 175

Ala Asp Gly Gly Met Ser Lys Tyr Pro Thr Asn Lys Ala Gly Ala Lys
            180                 185                 190

Tyr Gly Thr Gly Tyr Cys Asp Ser Gln Cys Pro Arg Asp Leu Lys Phe
        195                 200                 205

Ile Asn Gly Gln Ala Asn Val Glu Gly Trp Gln Pro Ser Ser Asn Asp
    210                 215                 220

Ala Asn Ala Gly Thr Gly Asn His Gly Ser Cys Cys Ala Glu Met Asp
225                 230                 235                 240

Ile Trp Glu Ala Asn Ser Ile Ser Thr Ala Phe Thr Pro His Pro Cys
                245                 250                 255

Asp Thr Pro Gly Gln Val Met Cys Thr Gly Asp Ala Cys Gly Gly Thr
            260                 265                 270

Tyr Ser Ser Asp Arg Tyr Gly Gly Thr Cys Asp Pro Asp Gly Cys Asp
        275                 280                 285

Phe Asn Ser Phe Arg Gln Gly Asn Lys Thr Phe Tyr Gly Pro Gly Met
    290                 295                 300

Thr Val Asp Thr Lys Ser Lys Phe Thr Val Val Thr Gln Phe Ile Thr
305                 310                 315                 320

Asp Asp Gly Thr Ser Ser Gly Thr Leu Lys Glu Ile Lys Arg Phe Tyr
                325                 330                 335

Val Gln Asn Gly Lys Val Ile Pro Asn Ser Glu Ser Thr Trp Thr Gly
            340                 345                 350
```

```
Val Ser Gly Asn Ser Ile Thr Thr Glu Tyr Cys Thr Ala Gln Lys Ser
        355                 360                 365

Leu Phe Gln Asp Gln Asn Val Phe Glu Lys His Gly Gly Leu Glu Gly
    370                 375                 380

Met Gly Ala Ala Leu Ala Gln Gly Met Val Leu Val Met Ser Leu Trp
385                 390                 395                 400

Asp Asp His Ser Ala Asn Met Leu Trp Leu Asp Ser Asn Tyr Pro Thr
                405                 410                 415

Thr Ala Ser Ser Thr Thr Pro Gly Val Ala Arg Gly Thr Cys Asp Ile
            420                 425                 430

Ser Ser Gly Val Pro Ala Asp Val Glu Ala Asn His Pro Asp Ala Tyr
        435                 440                 445

Val Val Tyr Ser Asn Ile Lys Val Gly Pro Ile Gly Ser Thr Phe Asn
    450                 455                 460

Ser Gly Gly Ser Asn Pro Gly Gly Gly Thr Thr Thr Thr Thr Thr Thr
465                 470                 475                 480

Gln Pro Thr Thr Thr Thr Thr Thr Ala Gly Asn Pro Gly Gly Thr Gly
                485                 490                 495

Val Ala Gln His Tyr Gly Gln Cys Gly Gly Ile Gly Trp Thr Gly Pro
            500                 505                 510

Thr Thr Cys Ala Ser Pro Tyr Thr Cys Gln Lys Leu Asn Asp Tyr Tyr
        515                 520                 525

Ser Gln Cys Leu
    530
```

<210> 11
<211> 1713
<212> DNA
<213> Aspergillus fumigatus

<400> 11

```
atgaagcacc ttgcatcttc catcgcattg actctactgt tgcctgccgt gcaggcccag      60

cagaccgtat ggggccaatg tatgttctgg ctgtcactgg aataagactg tatcaactgc     120

tgatatgctt ctaggtggcg gccaaggctg gtctggcccg acgagctgtg ttgccggcgc     180

agcctgtagc acactgaatc cctgtatgtt agatatcgtc ctgagtggag acttatactg     240
```

```
acttccttag actacgctca gtgtatcccg ggagccaccg cgacgtccac caccctcacg      300
acgacgacgg cggcgacgac gacatcccag accaccacca aacctaccac gactggtcca      360
actacatccg cacccaccgt gaccgcatcc ggtaaccctt tcagcggcta ccagctgtat      420
gccaacccct actactcctc cgaggtccat actctggcca tgccttctct gcccagctcg      480
ctgcagccca aggctagtgc tgttgctgaa gtgccctcat ttgtttggct gtaagtggcc      540
ttatcccaat actgagacca actctctgac agtcgtagcg acgttgccgc caaggtgccc      600
actatgggaa cctacctggc cgacattcag gccaagaaca aggccggcgc caaccctcct      660
atcgctggta tcttcgtggt ctacgacttg ccggaccgtg actgcgccgc tctggccagt      720
aatggcgagt actcaattgc caacaacggt gtggccaact acaaggcgta cattgacgcc      780
atccgtgctc agctggtgaa gtactctgac gttcacacca tcctcgtcat cggtaggccg      840
tacacctccg ttgcgcgccg cctttctctg acatcttgca gaacccgaca gcttggccaa      900
cctggtgacc aacctcaacg tcgccaaatg cgccaatgcg cagagcgcct acctggagtg      960
tgtcgactat gctctgaagc agctcaacct gcccaacgtc gccatgtacc tcgacgcagg     1020
tatgcctcac ttcccgcatt ctgtatccct tccagacact aactcatcag gccatgcggg     1080
ctggctcgga tggcccgcca acttgggccc cgccgcaaca ctcttcgcca aagtctacac     1140
cgacgcgggt tcccccgcgg ctgttcgtgg cctggccacc aacgtcgcca actacaacgc     1200
ctggtcgctc agtacctgcc cctcctacac ccagggagac cccaactgcg acgagaagaa     1260
gtacatcaac gccatggcgc ctcttctcaa ggaagccggc ttcgatgccc acttcatcat     1320
ggatacctgt aagtgcttat tccaatcgcc gatgtgtgcc gactaatcaa tgtttcagcc     1380
cggaatggcg tccagcccac gaagcaaaac gcctggggtg actggtgcaa cgtcatcggc     1440
accggcttcg gtgttcgccc ctcgactaac accggcgatc cgctccagga tgcctttgtg     1500
tggatcaagc ccggtggaga gagtgatggc acgtccaact cgacttcccc ccggtatgac     1560
gcgcactgcg gatatagtga tgctctgcag cctgctcctg aggctggtac ttggttccag     1620
gtatgtcatc cattagccag atgagggata agtgactgac ggacctaggc ctactttgag     1680
cagcttctga ccaacgctaa cccgtccttt taa                                 1713
```

<210> 12
<211> 454
<212> PRT
<213> Aspergillus fumigatus

<400> 12

```
Met Lys His Leu Ala Ser Ser Ile Ala Leu Thr Leu Leu Leu Pro Ala
1               5                   10                  15

Val Gln Ala Gln Gln Thr Val Trp Gly Gln Cys Gly Gly Gln Gly Trp
```

```
            20                  25                  30

Ser Gly Pro Thr Ser Cys Val Ala Gly Ala Ala Cys Ser Thr Leu Asn
        35                  40                  45

Pro Tyr Tyr Ala Gln Cys Ile Pro Gly Ala Thr Ala Thr Ser Thr Thr
    50                  55                  60

Leu Thr Thr Thr Thr Ala Ala Thr Thr Thr Ser Gln Thr Thr Thr Lys
65                  70                  75                  80

Pro Thr Thr Thr Gly Pro Thr Thr Ser Ala Pro Thr Val Thr Ala Ser
                85                  90                  95

Gly Asn Pro Phe Ser Gly Tyr Gln Leu Tyr Ala Asn Pro Tyr Tyr Ser
            100                 105                 110

Ser Glu Val His Thr Leu Ala Met Pro Ser Leu Pro Ser Ser Leu Gln
        115                 120                 125

Pro Lys Ala Ser Ala Val Ala Glu Val Pro Ser Phe Val Trp Leu Asp
    130                 135                 140

Val Ala Ala Lys Val Pro Thr Met Gly Thr Tyr Leu Ala Asp Ile Gln
145                 150                 155                 160

Ala Lys Asn Lys Ala Gly Ala Asn Pro Pro Ile Ala Gly Ile Phe Val
                165                 170                 175

Val Tyr Asp Leu Pro Asp Arg Asp Cys Ala Ala Leu Ala Ser Asn Gly
            180                 185                 190

Glu Tyr Ser Ile Ala Asn Asn Gly Val Ala Asn Tyr Lys Ala Tyr Ile
        195                 200                 205

Asp Ala Ile Arg Ala Gln Leu Val Lys Tyr Ser Asp Val His Thr Ile
    210                 215                 220

Leu Val Ile Glu Pro Asp Ser Leu Ala Asn Leu Val Thr Asn Leu Asn
225                 230                 235                 240

Val Ala Lys Cys Ala Asn Ala Gln Ser Ala Tyr Leu Glu Cys Val Asp
                245                 250                 255

Tyr Ala Leu Lys Gln Leu Asn Leu Pro Asn Val Ala Met Tyr Leu Asp
            260                 265                 270
```

```
Ala Gly His Ala Gly Trp Leu Gly Trp Pro Ala Asn Leu Gly Pro Ala
            275                 280                 285

Ala Thr Leu Phe Ala Lys Val Tyr Thr Asp Ala Gly Ser Pro Ala Ala
    290                 295                 300

Val Arg Gly Leu Ala Thr Asn Val Ala Asn Tyr Asn Ala Trp Ser Leu
305                 310                 315                 320

Ser Thr Cys Pro Ser Tyr Thr Gln Gly Asp Pro Asn Cys Asp Glu Lys
                325                 330                 335

Lys Tyr Ile Asn Ala Met Ala Pro Leu Leu Lys Glu Ala Gly Phe Asp
            340                 345                 350

Ala His Phe Ile Met Asp Thr Ser Arg Asn Gly Val Gln Pro Thr Lys
        355                 360                 365

Gln Asn Ala Trp Gly Asp Trp Cys Asn Val Ile Gly Thr Gly Phe Gly
    370                 375                 380

Val Arg Pro Ser Thr Asn Thr Gly Asp Pro Leu Gln Asp Ala Phe Val
385                 390                 395                 400

Trp Ile Lys Pro Gly Gly Glu Ser Asp Gly Thr Ser Asn Ser Thr Ser
                405                 410                 415

Pro Arg Tyr Asp Ala His Cys Gly Tyr Ser Asp Ala Leu Gln Pro Ala
            420                 425                 430

Pro Glu Ala Gly Thr Trp Phe Gln Ala Tyr Phe Glu Gln Leu Leu Thr
        435                 440                 445

Asn Ala Asn Pro Ser Phe
    450
```

<210> 13
<211> 799
<212> DNA
<213> Thermoascus aurantiacus

<400> 13

```
atgtcctttt ccaagataat tgctactgcc ggcgttcttg cctctgcttc tctagtggct        60
ggccatggct tcgttcagaa catcgtgatt gatggtaaaa agtatgtcat tgcaagacgc       120
acataagcgg caacagctga caatcgacag ttatggcggg tatctagtga accagtatcc       180
atacatgtcc aatcctccag aggtcatcgc ctggtctact acggcaactg atcttggatt       240
tgtggacggt actggatacc aaaccccaga tatcatctgc catagggggcg ccaagcctgg      300
agccctgact gctccagtct ctccaggagg aactgttgag cttcaatgga ctccatggcc       360
tgattctcac catggcccag ttatcaacta ccttgctccg tgcaatggtg attgttccac       420
tgtggataag acccaattag aattcttcaa aattgccgag agcggtctca tcaatgatga       480
caatcctcct gggatctggg cttcagacaa tctgatagca gccaacaaca gctggactgt       540
caccattcca accacaattg cacctggaaa ctatgttctg aggcatgaga ttattgctct       600
tcactcagct cagaaccagg atggtgccca gaactatccc cagtgcatca atctgcaggt       660
cactggaggt ggttctgata accctgctgg aactcttgga acggcactct accacgatac       720
cgatcctgga attctgatca acatctatca gaaactttcc agctatatca tccctggtcc       780
tcctctgtat actggttaa                                                    799
```

<210> 14
<211> 250
<212> PRT
<213> Thermoascus aurantiacus

<400> 14

```
Met Ser Phe Ser Lys Ile Ile Ala Thr Ala Gly Val Leu Ala Ser Ala
1               5                   10                  15

Ser Leu Val Ala Gly His Gly Phe Val Gln Asn Ile Val Ile Asp Gly
            20                  25                  30

Lys Lys Tyr Tyr Gly Gly Tyr Leu Val Asn Gln Tyr Pro Tyr Met Ser
        35                  40                  45

Asn Pro Pro Glu Val Ile Ala Trp Ser Thr Thr Ala Thr Asp Leu Gly
    50                  55                  60

Phe Val Asp Gly Thr Gly Tyr Gln Thr Pro Asp Ile Ile Cys His Arg
65                  70                  75                  80

Gly Ala Lys Pro Gly Ala Leu Thr Ala Pro Val Ser Pro Gly Gly Thr
                85                  90                  95

Val Glu Leu Gln Trp Thr Pro Trp Pro Asp Ser His His Gly Pro Val
            100                 105                 110

Ile Asn Tyr Leu Ala Pro Cys Asn Gly Asp Cys Ser Thr Val Asp Lys
        115                 120                 125

Thr Gln Leu Glu Phe Phe Lys Ile Ala Glu Ser Gly Leu Ile Asn Asp
    130                 135                 140

Asp Asn Pro Pro Gly Ile Trp Ala Ser Asp Asn Leu Ile Ala Ala Asn
145                 150                 155                 160

Asn Ser Trp Thr Val Thr Ile Pro Thr Thr Ile Ala Pro Gly Asn Tyr
                165                 170                 175

Val Leu Arg His Glu Ile Ile Ala Leu His Ser Ala Gln Asn Gln Asp
            180                 185                 190

Gly Ala Gln Asn Tyr Pro Gln Cys Ile Asn Leu Gln Val Thr Gly Gly
        195                 200                 205

Gly Ser Asp Asn Pro Ala Gly Thr Leu Gly Thr Ala Leu Tyr His Asp
    210                 215                 220

Thr Asp Pro Gly Ile Leu Ile Asn Ile Tyr Gln Lys Leu Ser Ser Tyr
225                 230                 235                 240

Ile Ile Pro Gly Pro Pro Leu Tyr Thr Gly
                245                 250
```

<210> 15
<211> 3060
<212> DNA
<213> Aspergillus fumigatus

<400> 15

```
atgagattcg gttggctcga ggtggccgct ctgacggccg cttctgtagc caatgcccag      60
gtttgtgatg ctttcccgtc attgtttcgg atatagttga caatagtcat ggaaataatc     120
aggaattggc tttctctcca ccattctacc cttcgccttg ggctgatggc cagggagagt     180
gggcagatgc ccatcgacgc gccgtcgaga tcgtttctca gatgacactg gcggagaagg     240
ttaaccttac aacgggtact gggtgggttg cgactttttt gttgacagtg agctttcttc     300
actgaccatc tacacagatg ggaaatggac cgatgcgtcg gtcaaaccgg cagcgttccc     360
aggtaagctt gcaattctgc aacaacgtgc aagtgtagtt gctaaaacgc ggtggtgcag     420
acttggtatc aactggggtc tttgtggcca ggattcccct ttgggtatcc gtttctgtga     480
gctatacccg cggagtcttt cagtccttgt attatgtgct gatgattgtc tctgtatagc     540
tgacctcaac tccgccttcc ctgctggtac taatgtcgcc gcgacatggg acaagacact     600
cgcctacctt cgtggcaagg ccatgggtga ggaattcaac gacaagggcg tggacatttt     660
gctggggcct gctgctggtc ctctcggcaa atacccggac ggcggcagaa tctgggaagg     720
cttctctcct gatccggttc tcactggtgt acttttcgcc gaaactatca agggtatcca     780
agacgcgggt gtgattgcta ctgccaagca ttacattctg aatgaacagg agcatttccg     840
acaggttggc gaggcccagg gatatggtta caacatcacg gagacgatca gctccaacgt     900
```

```
ggatgacaag accatgcacg agttgtacct ttggtgagta gttgacactg caaatgagga      960
ccttgattga tttgactgac ctggaatgca ggccctttgc agatgctgtg cgcggtaaga     1020
ttttccgtag acttgacctc gcgacgaaga aatcgctgac gaaccatcgt agctggcgtt     1080
ggcgctgtca tgtgttccta caatcaaatc aacaacagct acggttgtca aaacagtcaa     1140
actctcaaca agctcctcaa ggctgagctg ggcttccaag gcttcgtcat gagtgactgg     1200
agcgctcacc acagcggtgt cggcgctgcc ctcgctgggt tggatatgtc gatgcctgga     1260
gacatttcct tcgacgacgg actctccttc tggggcacga acctaactgt cagtgttctt     1320
aacggcaccg ttccagcctg gcgtgtcgat gacatggctg ttcgtatcat gaccgcgtac     1380
tacaaggttg gtcgtgaccg tcttcgtatt ccccctaact tcagctcctg gacccgggat     1440
gagtacggct gggagcattc tgctgtctcc gagggagcct ggaccaaggt gaacgacttc     1500
gtcaatgtgc agcgcagtca ctctcagatc atccgtgaga ttggtgccgc tagtacagtg     1560
ctcttgaaga acacgggtgc tcttcctttg accggcaagg aggttaaagt gggtgttctc     1620
ggtgaagacg ctggttccaa cccgtggggt gctaacggct gccccgaccg cggctgtgat     1680
aacggcactc ttgctatggc ctggggtagt ggtactgcca acttccctta ccttgtcacc     1740
cccgagcagg ctatccagcg agaggtcatc agcaacggcg gcaatgtctt tgctgtgact     1800
gataacgggg ctctcagcca gatggcagat gttgcatctc aatccaggtg agtgcgggct     1860
cttagaaaaa gaacgttctc tgaatgaagt tttttaacca ttgcgaacag cgtgtctttg     1920
gtgtttgtca acgccgactc tggagagggt ttcatcagtg tcgacggcaa cgagggtgac     1980
cgcaaaaatc tcactctgtg gaagaacggc gaggccgtca ttgacactgt tgtcagccac     2040
tgcaacaaca cgattgtggt tattcacagt gttgggcccg tcttgatcga ccggtggtat     2100
gataacccca acgtcactgc catcatctgg gccggcttgc ccggtcagga gagtggcaac     2160
tccctggtcg acgtgctcta tggccgcgtc aaccccagcg ccaagacccc gttcacctgg     2220
ggcaagactc gggagtctta cggggctccc ttgctcaccg agcctaacaa tggcaatggt     2280
gctccccagg atgatttcaa cgagggcgtc ttcattgact accgtcactt tgacaagcgc     2340
aatgagaccc ccatttatga gtttggccat ggcttgagct acaccacctt tggttactct     2400
caccttcggg ttcaggccct caatagttcg agttcggcat atgtcccgac tagcggagag     2460
accaagcctg cgccaaccta tggtgagatc ggtagtgccg ccgactacct gtatcccgag     2520
ggtctcaaaa gaattaccaa gtttatttac ccttggctca actcgaccga cctcgaggat     2580
tcttctgacg acccgaacta cggctgggag gactcggagt acattcccga aggcgctagg     2640
gatgggtctc ctcaacccct cctgaaggct ggcggcgctc ctggtggtaa ccctaccctt     2700
tatcaggatc ttgttagggt gtcggccacc ataaccaaca ctggtaacgt cgccggttat     2760
```

```
gaagtccctc aattggtgag tgacccgcat gttccttgcg ttgcaatttg gctaactcgc      2820
ttctagtatg tttcactggg cggaccgaac gagcctcggg tcgttctgcg caagttcgac      2880
cgaatcttcc tggctcctgg ggagcaaaag gtttggacca cgactcttaa ccgtcgtgat      2940
ctcgccaatt gggatgtgga ggctcaggac tgggtcatca caaagtaccc caagaaagtg      3000
cacgtcggca gctcctcgcg taagctgcct ctgagagcgc ctctgccccg tgtctactag      3060
```

<210> 16
<211> 863
<212> PRT
<213> Aspergillus fumigatus

<400> 16

```
Met Arg Phe Gly Trp Leu Glu Val Ala Ala Leu Thr Ala Ala Ser Val
1               5                   10                  15

Ala Asn Ala Gln Glu Leu Ala Phe Ser Pro Pro Phe Tyr Pro Ser Pro
            20                  25                  30

Trp Ala Asp Gly Gln Gly Glu Trp Ala Asp Ala His Arg Arg Ala Val
        35                  40                  45

Glu Ile Val Ser Gln Met Thr Leu Ala Glu Lys Val Asn Leu Thr Thr
    50                  55                  60

Gly Thr Gly Trp Glu Met Asp Arg Cys Val Gly Gln Thr Gly Ser Val
65                  70                  75                  80

Pro Arg Leu Gly Ile Asn Trp Gly Leu Cys Gly Gln Asp Ser Pro Leu
                85                  90                  95

Gly Ile Arg Phe Ser Asp Leu Asn Ser Ala Phe Pro Ala Gly Thr Asn
            100                 105                 110

Val Ala Ala Thr Trp Asp Lys Thr Leu Ala Tyr Leu Arg Gly Lys Ala
        115                 120                 125

Met Gly Glu Glu Phe Asn Asp Lys Gly Val Asp Ile Leu Leu Gly Pro
    130                 135                 140

Ala Ala Gly Pro Leu Gly Lys Tyr Pro Asp Gly Gly Arg Ile Trp Glu
145                 150                 155                 160

Gly Phe Ser Pro Asp Pro Val Leu Thr Gly Val Leu Phe Ala Glu Thr
                165                 170                 175

Ile Lys Gly Ile Gln Asp Ala Gly Val Ile Ala Thr Ala Lys His Tyr
```

```
            180                 185                 190

Ile Leu Asn Glu Gln Glu His Phe Arg Gln Val Gly Glu Ala Gln Gly
        195                 200                 205

Tyr Gly Tyr Asn Ile Thr Glu Thr Ile Ser Ser Asn Val Asp Asp Lys
    210                 215                 220

Thr Met His Glu Leu Tyr Leu Trp Pro Phe Ala Asp Ala Val Arg Ala
225                 230                 235                 240

Gly Val Gly Ala Val Met Cys Ser Tyr Asn Gln Ile Asn Asn Ser Tyr
                245                 250                 255

Gly Cys Gln Asn Ser Gln Thr Leu Asn Lys Leu Leu Lys Ala Glu Leu
            260                 265                 270

Gly Phe Gln Gly Phe Val Met Ser Asp Trp Ser Ala His His Ser Gly
        275                 280                 285

Val Gly Ala Ala Leu Ala Gly Leu Asp Met Ser Met Pro Gly Asp Ile
    290                 295                 300

Ser Phe Asp Asp Gly Leu Ser Phe Trp Gly Thr Asn Leu Thr Val Ser
305                 310                 315                 320

Val Leu Asn Gly Thr Val Pro Ala Trp Arg Val Asp Asp Met Ala Val
                325                 330                 335

Arg Ile Met Thr Ala Tyr Tyr Lys Val Gly Arg Asp Arg Leu Arg Ile
            340                 345                 350

Pro Pro Asn Phe Ser Ser Trp Thr Arg Asp Glu Tyr Gly Trp Glu His
        355                 360                 365

Ser Ala Val Ser Glu Gly Ala Trp Thr Lys Val Asn Asp Phe Val Asn
    370                 375                 380

Val Gln Arg Ser His Ser Gln Ile Ile Arg Glu Ile Gly Ala Ala Ser
385                 390                 395                 400

Thr Val Leu Leu Lys Asn Thr Gly Ala Leu Pro Leu Thr Gly Lys Glu
                405                 410                 415

Val Lys Val Gly Val Leu Gly Glu Asp Ala Gly Ser Asn Pro Trp Gly
            420                 425                 430
```

```
Ala Asn Gly Cys Pro Asp Arg Gly Cys Asp Asn Gly Thr Leu Ala Met
        435                 440                 445

Ala Trp Gly Ser Gly Thr Ala Asn Phe Pro Tyr Leu Val Thr Pro Glu
    450                 455                 460

Gln Ala Ile Gln Arg Glu Val Ile Ser Asn Gly Gly Asn Val Phe Ala
465                 470                 475                 480

Val Thr Asp Asn Gly Ala Leu Ser Gln Met Ala Asp Val Ala Ser Gln
                485                 490                 495

Ser Ser Val Ser Leu Val Phe Val Asn Ala Asp Ser Gly Glu Gly Phe
            500                 505                 510

Ile Ser Val Asp Gly Asn Glu Gly Asp Arg Lys Asn Leu Thr Leu Trp
        515                 520                 525

Lys Asn Gly Glu Ala Val Ile Asp Thr Val Val Ser His Cys Asn Asn
    530                 535                 540

Thr Ile Val Val Ile His Ser Val Gly Pro Val Leu Ile Asp Arg Trp
545                 550                 555                 560

Tyr Asp Asn Pro Asn Val Thr Ala Ile Ile Trp Ala Gly Leu Pro Gly
                565                 570                 575

Gln Glu Ser Gly Asn Ser Leu Val Asp Val Leu Tyr Gly Arg Val Asn
            580                 585                 590

Pro Ser Ala Lys Thr Pro Phe Thr Trp Gly Lys Thr Arg Glu Ser Tyr
        595                 600                 605

Gly Ala Pro Leu Leu Thr Glu Pro Asn Asn Gly Asn Gly Ala Pro Gln
    610                 615                 620

Asp Asp Phe Asn Glu Gly Val Phe Ile Asp Tyr Arg His Phe Asp Lys
625                 630                 635                 640

Arg Asn Glu Thr Pro Ile Tyr Glu Phe Gly His Gly Leu Ser Tyr Thr
                645                 650                 655

Thr Phe Gly Tyr Ser His Leu Arg Val Gln Ala Leu Asn Ser Ser Ser
            660                 665                 670

Ser Ala Tyr Val Pro Thr Ser Gly Glu Thr Lys Pro Ala Pro Thr Tyr
        675                 680                 685
```

Gly Glu Ile Gly Ser Ala Ala Asp Tyr Leu Tyr Pro Glu Gly Leu Lys
690 695 700

Arg Ile Thr Lys Phe Ile Tyr Pro Trp Leu Asn Ser Thr Asp Leu Glu
705 710 715 720

Asp Ser Ser Asp Asp Pro Asn Tyr Gly Trp Glu Asp Ser Glu Tyr Ile
725 730 735

Pro Glu Gly Ala Arg Asp Gly Ser Pro Gln Pro Leu Leu Lys Ala Gly
740 745 750

Gly Ala Pro Gly Gly Asn Pro Thr Leu Tyr Gln Asp Leu Val Arg Val
755 760 765

Ser Ala Thr Ile Thr Asn Thr Gly Asn Val Ala Gly Tyr Glu Val Pro
770 775 780

Gln Leu Tyr Val Ser Leu Gly Gly Pro Asn Glu Pro Arg Val Val Leu
785 790 795 800

Arg Lys Phe Asp Arg Ile Phe Leu Ala Pro Gly Glu Gln Lys Val Trp
805 810 815

Thr Thr Thr Leu Asn Arg Arg Asp Leu Ala Asn Trp Asp Val Glu Ala
820 825 830

Gln Asp Trp Val Ile Thr Lys Tyr Pro Lys Lys Val His Val Gly Ser
835 840 845

Ser Ser Arg Lys Leu Pro Leu Arg Ala Pro Leu Pro Arg Val Tyr
850 855 860

<210> 17
<211> 1415
<212> DNA
<213> Aspergillus fumigatus

<400> 17

```
atggtccatc tatcttcatt ggcagcagcc ctggctgctc tgcctctgta tgtttaccca      60
ctcacgagag gaggaacagc tttgacattg ctatagtgta tatggagctg gcctgaacac     120
agcagccaaa gccaaaggac taaagtactt tggttccgcc acggacaatc cagagctcac     180
ggactctgcg tatgtcgcgc aactgagcaa caccgatgat tttggtcaaa tcacacccgg     240
aaactccatg aaggtttgct tacgtctgcc tccctggagc attgcctcaa aagctaattg     300
gttgttttgt ttggatagtg ggatgccacc gagccttctc agaattcttt ttcgttcgca     360
aatggagacg ccgtggtcaa tctggcgaac aagaatggcc agctgatgcg atgccatact     420
ctggtctggc acagtcagct accgaactgg ggtatgtaaa cgtcttgtct attctcaaat     480
actctctaac agttgacagt ctctagcggg tcatggacca atgcgaccct tttggcggcc     540
atgaagaatc atatcaccaa tgtggttact cactacaagg ggaagtgcta cgcctgggat     600
gttgtcaatg aaggtttgtt gctccatcta tcctcaatag ttcttttgaa actgacaagc     660
ctgtcaatct agccctgaac gaggacggta ctttccgtaa ctctgtcttc taccagatca     720
tcggcccagc atacattcct attgcgttcg ccacggctgc tgccgcagat cccgacgtga     780
aactctacta caacgactac aacattgaat actcaggcgc caaagcgact gctgcgcaga     840
atatcgtcaa gatgatcaag gcctacggcg cgaagatcga cggcgtcggc ctccaggcac     900
actttatcgt cggcagcact ccgagtcaat cggatctgac gaccgtcttg aagggctaca     960
ctgctctcgg cgttgaggtg gcctataccg aacttgacat ccgcatgcag ctgccctcga    1020
ccgccgcaaa gctggcccag cagtccactg acttccaagg cgtggccgca gcatgcgtta    1080
gcaccactgg ctgcgtgggt gtcactatct gggactggac cgacaagtac tcctgggtcc    1140
ccagcgtgtt ccaaggctac ggcgccccat tgccttggga tgagaactat gtgaagaagc    1200
cagcgtacga tggcctgatg gcgggtcttg gagcaagcgg ctccggcacc acaacgacca    1260
ctactactac ttctactacg acaggaggta cggaccctac tggagtcgct cagaaatggg    1320
gacagtgtgg cggtattggc tggaccgggc caacaacttg tgtcagtggt accacttgcc    1380
aaaagctgaa tgactggtac tcacagtgcc tgtaa                               1415
```

<210> 18
<211> 397
<212> PRT
<213> Aspergillus fumigatus

<400> 18

```
Met Val His Leu Ser Ser Leu Ala Ala Ala Leu Ala Ala Leu Pro Leu
1               5                   10                  15

Val Tyr Gly Ala Gly Leu Asn Thr Ala Ala Lys Ala Lys Gly Leu Lys
            20                  25                  30

Tyr Phe Gly Ser Ala Thr Asp Asn Pro Glu Leu Thr Asp Ser Ala Tyr
        35                  40                  45

Val Ala Gln Leu Ser Asn Thr Asp Asp Phe Gly Gln Ile Thr Pro Gly
    50                  55                  60

Asn Ser Met Lys Trp Asp Ala Thr Glu Pro Ser Gln Asn Ser Phe Ser
65                  70                  75                  80
```

```
Phe Ala Asn Gly Asp Ala Val Val Asn Leu Ala Asn Lys Asn Gly Gln
                85                  90                  95

Leu Met Arg Cys His Thr Leu Val Trp His Ser Gln Leu Pro Asn Trp
            100                 105                 110

Val Ser Ser Gly Ser Trp Thr Asn Ala Thr Leu Leu Ala Ala Met Lys
        115                 120                 125

Asn His Ile Thr Asn Val Val Thr His Tyr Lys Gly Lys Cys Tyr Ala
    130                 135                 140

Trp Asp Val Val Asn Glu Ala Leu Asn Glu Asp Gly Thr Phe Arg Asn
145                 150                 155                 160

Ser Val Phe Tyr Gln Ile Ile Gly Pro Ala Tyr Ile Pro Ile Ala Phe
                165                 170                 175

Ala Thr Ala Ala Ala Ala Asp Pro Asp Val Lys Leu Tyr Tyr Asn Asp
            180                 185                 190

Tyr Asn Ile Glu Tyr Ser Gly Ala Lys Ala Thr Ala Ala Gln Asn Ile
        195                 200                 205

Val Lys Met Ile Lys Ala Tyr Gly Ala Lys Ile Asp Gly Val Gly Leu
    210                 215                 220

Gln Ala His Phe Ile Val Gly Ser Thr Pro Ser Gln Ser Asp Leu Thr
225                 230                 235                 240

Thr Val Leu Lys Gly Tyr Thr Ala Leu Gly Val Glu Val Ala Tyr Thr
                245                 250                 255

Glu Leu Asp Ile Arg Met Gln Leu Pro Ser Thr Ala Ala Lys Leu Ala
            260                 265                 270

Gln Gln Ser Thr Asp Phe Gln Gly Val Ala Ala Ala Cys Val Ser Thr
        275                 280                 285

Thr Gly Cys Val Gly Val Thr Ile Trp Asp Trp Thr Asp Lys Tyr Ser
    290                 295                 300

Trp Val Pro Ser Val Phe Gln Gly Tyr Gly Ala Pro Leu Pro Trp Asp
305                 310                 315                 320

Glu Asn Tyr Val Lys Lys Pro Ala Tyr Asp Gly Leu Met Ala Gly Leu
                325                 330                 335
```

Gly Ala Ser Gly Ser Gly Thr Thr Thr Thr Thr Thr Thr Thr Ser Thr
340 345 350

Thr Thr Gly Gly Thr Asp Pro Thr Gly Val Ala Gln Lys Trp Gly Gln
355 360 365

Cys Gly Gly Ile Gly Trp Thr Gly Pro Thr Thr Cys Val Ser Gly Thr
370 375 380

Thr Cys Gln Lys Leu Asn Asp Trp Tyr Ser Gln Cys Leu
385 390 395

<210> 19
<211> 19
<212> PRT
<213> Thielavia terrestris

<220>
<221> MISC_FEATURE
<222> (1)..(1)
<223> X=I, L, M, OR V

<220>
<221> misc_feature
<222> (3)..(6)
<223> Xaa can be any naturally occurring amino acid

<220>
<221> misc_feature
<222> (8)..(8)
<223> Xaa can be any naturally occurring amino acid

<220>
<221> MISC_FEATURE
<222> (10)..(10)
<223> X=I, L, M, OR V

<220>
<221> misc_feature
<222> (11)..(11)
<223> Xaa can be any naturally occurring amino acid

<220>
<221> misc_feature
<222> (13)..(13)
<223> Xaa can be any naturally occurring amino acid

<220>
<221> MISC_FEATURE
<222> (14)..(14)
<223> X=E OR Q

<220>
<221> misc_feature
<222> (15)..(18)

<223> Xaa can be any naturally occurring amino acid

<220>
<221> MISC_FEATURE
<222> (19)..(19)
<223> X=H, N, OR Q

<400> 19

```
Xaa Pro Xaa Xaa Xaa Xaa Gly Xaa Tyr Xaa Xaa Arg Xaa Xaa Xaa Xaa
1               5                   10                  15


Xaa Xaa Xaa
```

<210> 20
<211> 20
<212> PRT
<213> Thielavia terrestris

<220>
<221> MISC_FEATURE
<222> (1)..(1)
<223> X=I, L, M, OR V

<220>
<221> misc_feature
<222> (3)..(7)
<223> Xaa can be any naturally occurring amino acid

<220>
<221> misc_feature
<222> (9)..(9)
<223> Xaa can be any naturally occurring amino acid

<220>
<221> MISC_FEATURE
<222> (11)..(11)
<223> X=I, L, M, OR V

<220>
<221> misc_feature
<222> (12)..(12)
<223> Xaa can be any naturally occurring amino acid

<220>
<221> misc_feature
<222> (14)..(14)
<223> Xaa can be any naturally occurring amino acid

<220>
<221> MISC_FEATURE
<222> (15)..(15)
<223> X=E OR Q

<220>
<221> misc_feature
<222> (16)..(19)

<223> Xaa can be any naturally occurring amino acid

<220>
<221> MISC_FEATURE
<222> (20)..(20)
<223> X=H, N, OR Q

<400> 20

```
Xaa Pro Xaa Xaa Xaa Xaa Xaa Gly Xaa Tyr Xaa Xaa Arg Xaa Xaa Xaa
1               5                   10                  15

Xaa Xaa Xaa Xaa
            20
```

<210> 21
<211> 9
<212> PRT
<213> Thielavia terrestris

<220>
<221> misc_feature
<222> (2)..(2)
<223> Xaa can be any naturally occurring amino acid

<220>
<221> misc_feature
<222> (5)..(7)
<223> Xaa can be any naturally occurring amino acid

<220>
<221> MISC_FEATURE
<222> (8)..(8)
<223> X= Y OR W

<220>
<221> MISC_FEATURE
<222> (9)..(9)
<223> X= A,I,L,M OR V

<400> 21

```
His Xaa Gly Pro Xaa Xaa Xaa Xaa Xaa
1               5
```

<210> 22
<211> 10
<212> PRT
<213> Thielavia terrestris

<220>
<221> misc_feature
<222> (2)..(3)
<223> Xaa can be any naturally occurring amino acid

<220>
<221> misc_feature

<222> (6)..(8)
<223> Xaa can be any naturally occurring amino acid

<220>
<221> MISC_FEATURE
<222> (9) .. (9)
<223> X= Y OR W

<220>
<221> MISC_FEATURE
<222> (10)..(10)
<223> X= A, I, L, M OR V

<400> 22

```
His Xaa Xaa Gly Pro Xaa Xaa Xaa Xaa Xaa
1               5                   10
```

<210> 23
<211> 11
<212> PRT
<213> Thielavia terrestris

<220>
<221> MISC_FEATURE
<222> (1)..(1)
<223> X= E OR Q

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<220>
<221> misc_feature
<222> (7)..(7)
<223> Xaa can be any naturally occurring amino acid

<220>
<221> MISC_FEATURE
<222> (8)..(8)
<223> X= E, H, Q OR N

<220>
<221> MISC_FEATURE
<222> (9) .. (9)
<223> X=F, I, L, OR V

<220>
<221> misc_feature
<222> (10)..(10)
<223> Xaa can be any naturally occurring amino acid

<220>
<221> MISC_FEATURE
<222> (11)..(11)
<223> X=I, L, OR V

<400> 23

```
Xaa Xaa Tyr Xaa Xaa Cys Xaa Xaa Xaa Xaa Xaa
1               5                   10
```

<210> 24
<211> 9
<212> PRT
<213> Thielavia terrestris

<220>
<221> misc_feature
<222> (2)..(2)
<223> Xaa can be any naturally occurring amino acid

<220>
<221> misc_feature
<222> (5)..(7)
<223> Xaa can be any naturally occurring amino acid

<220>
<221> MISC_FEATURE
<222> (8)..(8)
<223> X= Y OR W

<220>
<221> MISC_FEATURE
<222> (9)..(9)
<223> X= A, I, L, M OR V

<400> 24

```
His Xaa Gly Pro Xaa Xaa Xaa Xaa Xaa
1               5
```

<210> 25
<211> 10
<212> PRT
<213> Thielavia terrestris

<220>
<221> misc_feature
<222> (2)..(3)
<223> Xaa can be any naturally occurring amino acid

<220>
<221> misc_feature
<222> (6)..(8)
<223> Xaa can be any naturally occurring amino acid

<220>
<221> MISC_FEATURE

<222> (9) .. (9)
<223> X= Y OR W

<220>
<221> MISC_FEATURE
<222> (10)..(10)
<223> X= A, I, L, M OR V

<400> 25

```
His Xaa Xaa Gly Pro Xaa Xaa Xaa Xaa Xaa
1               5                   10
```

<210> 26
<211> 11
<212> PRT
<213> Thielavia terrestris

<220>
<221> MISC_FEATURE
<222> (1)..(1)
<223> X= E OR Q

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<220>
<221> misc_feature
<222> (7)..(7)
<223> Xaa can be any naturally occurring amino acid

<220>
<221> MISC_FEATURE
<222> (8)..(8)
<223> X= E, H, Q OR N

<220>
<221> MISC_FEATURE
<222> (9) .. (9)
<223> X=F, I, L, OR V

<220>
<221> misc_feature
<222> (10)..(10)
<223> Xaa can be any naturally occurring amino acid

<220>
<221> MISC_FEATURE
<222> (11)..(11)
<223> X=I, L, OR V

<400> 26

Xaa Xaa Tyr Xaa Xaa Cys Xaa Xaa Xaa Xaa Xaa
1               5                   10

<210> 27
<211> 9
<212> PRT
<213> Thielavia terrestris

<220>
<221> misc_feature
<222> (2)..(2)
<223> Xaa can be any naturally occurring amino acid

<220>
<221> misc_feature
<222> (5)..(7)
<223> Xaa can be any naturally occurring amino acid

<220>
<221> MISC_FEATURE
<222> (8)..(8)
<223> X= Y OR W

<220>
<221> MISC_FEATURE
<222> (9) .. (9)
<223> X= A, I, L, M OR V

<400> 27

His Xaa Gly Pro Xaa Xaa Xaa Xaa Xaa
1               5

<210> 28
<211> 10
<212> PRT
<213> Thielavia terrestris

<220>
<221> misc_feature
<222> (2)..(3)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9)..(9)
<223> X= Y OR W

<220>

<221> MISC_FEATURE
<222> (10)..(10)
<223> X= A, I, L, M OR V

<400> 28

```
His Xaa Xaa Gly Pro Xaa Xaa Xaa Xaa Xaa
1               5                   10
```

<210> 29
<211> 11
<212> PRT
<213> Thielavia terrestris

<220>
<221> MISC_FEATURE
<222> (1)..(1)
<223> X= E OR Q

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<220>
<221> misc_feature
<222> (7)..(7)
<223> Xaa can be any naturally occurring amino acid

<220>
<221> MISC_FEATURE
<222> (8)..(8)
<223> X= E, H, Q OR N

<220>
<221> MISC_FEATURE
<222> (9) .. (9)
<223> X=F, I, L, OR V

<220>
<221> misc_feature
<222> (10)..(10)
<223> Xaa can be any naturally occurring amino acid

<220>
<221> MISC_FEATURE
<222> (11)..(11)
<223> X=I, L, OR V

<400> 29

```
Xaa Xaa Tyr Xaa Xaa Cys Xaa Xaa Xaa Xaa Xaa
1               5                   10
```

<210> 30
<211> 9
<212> PRT
<213> Thielavia terrestris

<220>
<221> misc_feature
<222> (2)..(2)
<223> Xaa can be any naturally occurring amino acid

<220>
<221> misc_feature
<222> (5)..(7)
<223> Xaa can be any naturally occurring amino acid

<220>
<221> MISC_FEATURE
<222> (8)..(8)
<223> X= Y OR W

<220>
<221> MISC_FEATURE
<222> (9) .. (9)
<223> X= A, I, L, M OR V

<400> 30

```
His Xaa Gly Pro Xaa Xaa Xaa Xaa Xaa
1               5
```

<210> 31
<211> 10
<212> PRT
<213> Thielavia terrestris

<220>
<221> misc_feature
<222> (2)..(3)
<223> Xaa can be any naturally occurring amino acid

<220>
<221> misc_feature
<222> (6)..(8)
<223> Xaa can be any naturally occurring amino acid

<220>
<221> MISC_FEATURE
<222> (9) .. (9)
<223> X= Y OR W

<220>
<221> MISC_FEATURE
<222> (10)..(10)
<223> X= A, I, L, M OR V

<400> 31

```
His Xaa Xaa Gly Pro Xaa Xaa Xaa Xaa Xaa
1               5                   10
```

<210> 32
<211> 11
<212> PRT
<213> Thielavia terrestris

<220>
<221> MISC_FEATURE
<222> (1)..(1)
<223> X= E OR Q

<220>
<221> misc_feature
<222> (2)..(2)
<223> Xaa can be any naturally occurring amino acid

<220>
<221> misc_feature
<222> (4)..(5)
<223> Xaa can be any naturally occurring amino acid

<220>
<221> misc_feature
<222> (7)..(7)
<223> Xaa can be any naturally occurring amino acid

<220>
<221> MISC_FEATURE
<222> (8)..(8)
<223> X= E, H, Q OR N

<220>
<221> MISC_FEATURE
<222> (9)..(9)
<223> X=F, I, L, OR V

<220>
<221> misc_feature
<222> (10)..(10)
<223> Xaa can be any naturally occurring amino acid

<220>
<221> MISC_FEATURE
<222> (11)..(11)
<223> X=I, L, OR V

<400> 32

```
Xaa Xaa Tyr Xaa Xaa Cys Xaa Xaa Xaa Xaa Xaa
1               5                   10
```

<210> 33
<211> 19
<212> PRT

<213> Thielavia terrestris

<220>
<221> MISC_FEATURE
<222> (1)..(1)
<223> X=I, L, M OR V

<220>
<221> misc_feature
<222> (3)..(6)
<223> Xaa can be any naturally occurring amino acid

<220>
<221> misc_feature
<222> (8)..(8)
<223> Xaa can be any naturally occurring amino acid

<220>
<221> MISC_FEATURE
<222> (10)..(10)
<223> X=I, L, M OR V

<220>
<221> misc_feature
<222> (11)..(11)
<223> Xaa can be any naturally occurring amino acid

<220>
<221> misc_feature
<222> (13)..(13)
<223> Xaa can be any naturally occurring amino acid

<220>
<221> MISC_FEATURE
<222> (14)..(14)
<223> X= E OR Q

<220>
<221> misc_feature
<222> (15)..(17)
<223> Xaa can be any naturally occurring amino acid

<220>
<221> MISC_FEATURE
<222> (19)..(19)
<223> X= H,N, OR Q

<400> 33

```
Xaa Pro Xaa Xaa Xaa Xaa Gly Xaa Tyr Xaa Xaa Arg Xaa Xaa Xaa Xaa
1               5                   10                  15

Xaa Ala Xaa
```

<210> 34
<211> 20
<212> PRT

<213> Thielavia terrestris

<220>
<221> MISC_FEATURE
<222> (1)..(1)
<223> X=I, L, M OR V

<220>
<221> misc_feature
<222> (3)..(7)
<223> Xaa can be any naturally occurring amino acid

<220>
<221> misc_feature
<222> (9)..(9)
<223> Xaa can be any naturally occurring amino acid

<220>
<221> MISC_FEATURE
<222> (11)..(11)
<223> X=I, L, M OR V

<220>
<221> misc_feature
<222> (12)..(12)
<223> Xaa can be any naturally occurring amino acid

<220>
<221> misc_feature
<222> (14)..(14)
<223> Xaa can be any naturally occurring amino acid

<220>
<221> MISC_FEATURE
<222> (15)..(15)
<223> X=E OR Q

<220>
<221> misc_feature
<222> (16)..(18)
<223> Xaa can be any naturally occurring amino acid

<220>
<221> MISC_FEATURE
<222> (20)..(20)
<223> X= H, N, OR Q

<400> 34

```
Xaa Pro Xaa Xaa Xaa Xaa Xaa Gly Xaa Tyr Xaa Xaa Arg Xaa Xaa Xaa
1               5                   10                  15

Xaa Xaa Ala Xaa
            20
```

## Description

### Reference to a Sequence Listing

This application contains a Sequence Listing in computer readable form.

### Reference to a Deposit of Biological Material

This application contains a reference to a deposit of biological material.

### Background of the Invention

### Field of the Invention

The present invention relates to methods for degrading or converting a cellulosic material comprising treating the cellulosic material with an enzyme composition in the presence of polypeptides having endoglucanase activity, catalytic domains, and cellulose binding domains. The present invention also relates to methods of producing a fermentation product and fermenting cellulosic materials.

### Description of the Related Art

Cellulose is a polymer of the simple sugar glucose covalently linked by beta-1,4-bonds. Many microorganisms produce enzymes that hydrolyze beta-linked glucans. These enzymes include endoglucanases, cellobiohydrolases, and beta-glucosidases. Endoglucanases digest the cellulose polymer at random locations, opening it to attack by cellobiohydrolases. Cellobiohydrolases sequentially release molecules of cellobiose from the ends of the cellulose polymer. Cellobiose is a water-soluble beta-1,4-linked dimer of glucose. Beta-glucosidases hydrolyze cellobiose to glucose.

The conversion of lignocellulosic feedstocks into ethanol has the advantages of the ready availability of large amounts of feedstock, the desirability of avoiding burning or land filling the materials, and the cleanliness of the ethanol fuel. Wood, agricultural residues, herbaceous crops, and municipal solid wastes have been considered as feedstocks for ethanol production. These materials primarily consist of cellulose, hemicellulose, and lignin. Once the lignocellulose is converted to fermentable sugars, *e.g.,* glucose, the fermentable sugars are easily fermented by yeast into ethanol.

The present disclosure provides polypeptides having endoglucanase activity and polynucleotides encoding the polypeptides.

### Summary of the Invention

The present invention relates to a method for degrading or converting a cellulosic material, comprising treating the cellulosic material with an enzyme composition in the presence of isolated polypeptides having endoglucanase activity selected from the group consisting of:

(a) a polypeptide having at least 90% sequence identity to the mature polypeptide of SEQ ID NO: 2;
(b) a polypeptide encoded by a polynucleotide having at least 90% sequence identity to the mature polypeptide coding sequence of SEQ ID NO: 1;
(c) a variant of the mature polypeptide of SEQ ID NO: 2 comprising a substitution, deletion, and/or insertion at one or more (e.g., several) positions wherein the variant has endoglucanase activity and wherein the number of amino acid substitutions, deletions and/or insertions introduced into the mature polypeptide of SEQ ID NO: 2 is up to 10, e.g., 1, 2, 3, 4, 5, 6, 7, 8, 9, or 10; and
(d) a fragment of the polypeptide of (a), (b), or (c) that has endoglucanase activity, wherein at 50°C, 55°C, 60°C, and 65°C the degree of cellulose conversion to glucose is higher than that of the enzyme composition without endoglucanase.

The present disclosure also relates to isolated polypeptides comprising a catalytic domain selected from the group consisting of:

(a) a catalytic domain having at least 91% sequence identity to amino acids 24 to 419 of SEQ ID NO: 2;
(b) a catalytic domain encoded by a polynucleotide having at least 91% sequence identity to nucleotides 70 to 1257 of SEQ ID NO: 1;
(c) a variant of amino acids 24 to 419 of SEQ ID NO: 2 comprising a substitution, deletion, and/or insertion at one or more (*e.g.*, several) positions; and
(d) a fragment of the catalytic domain of (a), (b), or (c), which has endoglucanase activity.

The present disclosure also relates to isolated polypeptides comprising a cellulose binding domain selected from the group consisting of:

(a) a cellulose binding domain having at least 91% sequence identity to amino acids 463 to 498 of SEQ ID NO: 2;
(b) a cellulose binding domain encoded by a polynucleotide having at least 91% sequence identity to nucleotides 1387 to 1494 of SEQ ID NO: 1;
(c) a variant of amino acids 463 to 498 of SEQ ID NO: 2 comprising a substitution, deletion, and/or insertion at one or more (*e.g.*, several) positions; and
(d) a fragment of the cellulose binding domain of (a), (b), or (c) that has cellulose binding activity.

The present disclosure also relates to isolated polynucleotides encoding the polypeptides of the present disclosure; nucleic acid constructs, recombinant expression vectors, and recombinant host cells comprising the polynucleotides; and methods of producing the polypeptides.

The present invention relates to methods for degrading or converting a cellulosic material, comprising: treating the cellulosic material with an enzyme composition in the presence of a polypeptide having endoglucanase activity of the present disclosure. In one aspect, the method further comprises recovering the degraded or converted cellulosic material.

The present invention also relates to methods of producing a fermentation product, comprising: (a) saccharifying a cellulosic material with an enzyme composition in the presence of a polypeptide having endoglucanase activity of the present invention; (b) fermenting the saccharified cellulosic material with one or more (*e.g.*, several) fermenting microorganisms to produce the fermentation product; and (c) recovering the fermentation product from the fermentation.

The present invention also relates to methods of fermenting a cellulosic material, comprising: fermenting the cellulosic material with one or more (*e.g.*, several) fermenting microorganisms, wherein the cellulosic material is saccharified with an enzyme composition in the presence of a polypeptide having endoglucanase activity of the present invention. In one aspect, the fermenting of the cellulosic material produces a fermentation product. In another aspect, the method further comprises recovering the fermentation product from the fermentation.

The present disclosure also relates to a polynucleotide encoding a signal peptide comprising or consisting of amino acids 1 to 23 of SEQ ID NO: 2, which is operably linked to a gene encoding a protein, wherein the gene is foreign to the polynucleotide encoding the signal peptide; nucleic acid constructs, expression vectors, and recombinant host cells comprising the polynucleotide; and methods of producing a protein.

## Brief Description of the Figures

Figure 1 shows the genomic DNA sequence (SEQ ID NO: 1) and the deduced amino acid sequence (SEQ ID NO: 2) of a *Penicillium pinophilum* GH7 endoglucanase gene.
Figure 2 shows a restriction map of pPpin13.
Figure 3 shows the effect of the *Penicillium pinophilum* GH7 endoglucanase on hydrolysis of milled unwashed PCS by a high-temperature enzyme composition at 50-65°C.

## Definitions

**Acetylxylan esterase:** The term "acetylxylan esterase" means a carboxylesterase (EC 3.1.1.72) that catalyzes the hydrolysis of acetyl groups from polymeric xylan, acetylated xylose, acetylated glucose, alpha-napthyl acetate, and p-nitrophenyl acetate. For purposes of the present disclosure, acetylxylan esterase activity is determined using 0.5 mM p-nitrophenylacetate as substrate in 50 mM sodium acetate pH 5.0 containing 0.01% TWEEN™ 20 (polyoxyethylene sorbitan monolaurate). One unit of acetylxylan esterase is defined as the amount of enzyme capable of releasing 1 μmole of p-nitrophenolate anion per minute at pH 5, 25°C.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Alpha-L-arabinofuranosidase:** The term "alpha-L-arabinofuranosidase" means an alpha-L-arabinofuranoside arabinofuranohydrolase (EC 3.2.1.55) that catalyzes the hydrolysis of terminal non-reducing alpha-L-arabinofuranoside residues in alpha-L-arabinosides. The enzyme acts on alpha-L-arabinofuranosides, alpha-L-arabinans containing (1,3)- and/or (1,5)-linkages, arabinoxylans, and arabinogalactans. Alpha-L-arabinofuranosidase is also known as arabinosidase, alpha-arabinosidase, alpha-L-arabinosidase, alpha-arabinofuranosidase, polysaccharide alpha-L-arabinofuranosidase, alpha-L-arabinofuranoside hydrolase, L-arabinosidase, or alpha-L-arabinanase. For purposes of the present disclosure, alpha-L-arabinofuranosidase activity is determined using 5 mg of medium viscosity wheat arabinoxylan (Megazyme International Ireland, Ltd., Bray, Co. Wicklow, Ireland) per ml of 100 mM sodium acetate pH 5 in a total volume of 200 μl for 30 minutes at 40°C followed by arabinose analysis by AMINEX® HPX-87H column chromatography (Bio-Rad Laboratories, Inc., Hercules, CA, USA).

**Alpha-glucuronidase:** The term "alpha-glucuronidase" means an alpha-D-glucosiduronate glucuronohydrolase (EC 3.2.1.139) that catalyzes the hydrolysis of an alpha-D-glucuronoside to D-glucuronate and an alcohol. For purposes of the present disclosure, alpha-glucuronidase activity is determined according to de Vries, 1998, J. Bacteriol. 180: 243-249. One unit of alpha-glucuronidase equals the amount of enzyme capable of releasing 1 μmole of glucuronic or 4-O-methylglucuronic acid per minute at pH 5, 40°C.

**Beta-glucosidase:** The term "beta-glucosidase" means a beta-D-glucoside glucohydrolase (E.C. 3.2.1.21) that catalyzes the hydrolysis of terminal non-reducing beta-D-glucose residues with the release of beta-D-glucose. For purposes of the present disclosure, beta-glucosidase activity is determined using *p*-nitrophenyl-beta-D-glucopyranoside as substrate according to the procedure of Venturi et al., 2002, Extracellular beta-D-glucosidase from Chaetomium thermophilum var. coprophilum: production, purification and some biochemical properties, J. Basic Microbiol. 42: 55-66. One unit of beta-glucosidase is defined as 1.0 μmole of *p*-nitrophenolate anion produced per minute at 25°C, pH 4.8 from 1 mM *p*-nitrophenyl-beta-D-glucopyranoside as substrate in 50 mM sodium citrate containing 0.01% TWEEN® 20.

**Beta-xylosidase:** The term "beta-xylosidase" means a beta-D-xyloside xylohydrolase (E.C. 3.2.1.37) that catalyzes the exo-hydrolysis of short beta (1→4)-xylooligosaccharides to remove successive D-xylose residues from non-reducing termini. For purposes of the present disclosure, one unit of beta-xylosidase is defined as 1.0 μmole of *p*-nitrophenolate anion produced per minute at 40°C, pH 5 from 1 mM *p*-nitrophenyl-beta-D-xyloside as substrate in 100 mM sodium citrate containing 0.01% TWEEN® 20.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Cellulose binding domain:** The term "cellulose binding domain" means the region of an enzyme that mediates binding of the enzyme to amorphous regions of a cellulose substrate. The cellulose binding domain (CBD) is typically found either at the N-terminal or at the C-terminal extremity of an enzyme.

**Cellobiohydrolase:** The term "cellobiohydrolase" means a 1,4-beta-D-glucan cellobiohydrolase (E.C. 3.2.1.91 and E.C. 3.2.1.176) that catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain (Teeri, 1997, Crystalline cellulose degradation: New insight into the function of cellobiohydrolases, Trends in Biotechnology 15: 160-167; Teeri et al., 1998, Trichoderma reesei cellobiohydrolases: why so efficient on crystalline cellulose?, Biochem. Soc. Trans. 26: 173-178). For purposes of the present disclosure, cellobiohydrolase activity is determined according to the procedures described by Lever et al., 1972, Anal. Biochem. 47: 273-279; van Tilbeurgh et al., 1982, FEBS Letters, 149: 152-156; van Tilbeurgh and Claeyssens, 1985, FEBS Letters, 187: 283-288; and Tomme et al., 1988, Eur. J. Biochem. 170: 575-581. In the present disclosure, the Lever *et al.* method can be employed to assess hydrolysis of cellulose in corn stover, while the methods of van Tilbeurgh *et al.* and Tomme *et al.* can be used to determine the cellobiohydrolase activity on a fluorescent disaccharide derivative, 4-methylumbelliferyl-β-D-lactoside.

**Cellulolytic enzyme or cellulase:** The term "cellulolytic enzyme" or "cellulase" means one or more (*e.g.*, several) enzymes that hydrolyze a cellulosic material. Such enzymes include endoglucanase(s), cellobiohydrolase(s), beta-glucosidase(s), or combinations thereof. The two basic approaches for measuring cellulolytic activity include: (1) measuring the total cellulolytic activity, and (2) measuring the individual cellulolytic activities (endoglucanases, cellobiohydrolases, and beta-glucosidases) as reviewed in Zhang et al., Outlook for cellulase improvement: Screening and selection strategies, 2006, Biotechnology Advances 24: 452-481. Total cellulolytic activity is usually measured using insoluble substrates, including Whatman №1 filter paper, microcrystalline cellulose, bacterial cellulose, algal cellulose, cotton, pretreated lignocellulose, *etc.* The most common total cellulolytic activity assay is the filter paper assay using Whatman №1 filter paper as the substrate. The assay was established by the International Union of Pure and Applied Chemistry (IUPAC) (Ghose, 1987, Measurement of cellulase activities, Pure Appl. Chem. 59: 257-68).

For purposes of the present disclosure, cellulolytic enzyme activity is determined by measuring the increase in hydrolysis of a cellulosic material by cellulolytic enzyme(s) under the following conditions: 1-50 mg of cellulolytic enzyme protein/g of cellulose in PCS (or other pretreated cellulosic material) for 3-7 days at a suitable temperature, *e.g.,*

50°C, 55°C, or 60°C, compared to a control hydrolysis without addition of cellulolytic enzyme protein. Typical conditions are 1 ml reactions, washed or unwashed PCS, 5% insoluble solids, 50 mM sodium acetate pH 5, 1 mM $MnSO_4$, 50°C, 55°C, or 60°C, 72 hours, sugar analysis by AMINEX® HPX-87H column (Bio-Rad Laboratories, Inc., Hercules, CA, USA).

**Cellulosic material:** The term "cellulosic material" means any material containing cellulose. The predominant polysaccharide in the primary cell wall of biomass is cellulose, the second most abundant is hemicellulose, and the third is pectin. The secondary cell wall, produced after the cell has stopped growing, also contains polysaccharides and is strengthened by polymeric lignin covalently cross-linked to hemicellulose. Cellulose is a homopolymer of anhydrocellobiose and thus a linear beta-(1-4)-D-glucan, while hemicelluloses include a variety of compounds, such as xylans, xyloglucans, arabinoxylans, and mannans in complex branched structures with a spectrum of substituents. Although generally polymorphous, cellulose is found in plant tissue primarily as an insoluble crystalline matrix of parallel glucan chains. Hemicelluloses usually hydrogen bond to cellulose, as well as to other hemicelluloses, which help stabilize the cell wall matrix.

Cellulose is generally found, for example, in the stems, leaves, hulls, husks, and cobs of plants or leaves, branches, and wood of trees. The cellulosic material can be, but is not limited to, agricultural residue, herbaceous material (including energy crops), municipal solid waste, pulp and paper mill residue, waste paper, and wood (including forestry residue) (see, for example, Wiselogel et al., 1995, in Handbook on Bioethanol (Charles E. Wyman, editor), pp.105-118, Taylor & Francis, Washington D.C.; Wyman, 1994, Bioresource Technology 50: 3-16; Lynd, 1990, Applied Biochemistry and Biotechnology 24/25: 695-719; Mosier et al., 1999, Recent Progress in Bioconversion of Lignocellulosics, in Advances in Biochemical Engineering/Biotechnology, T. Scheper, managing editor, Volume 65, pp.23-40, Springer-Verlag, New York). It is understood herein that the cellulose may be in the form of lignocellulose, a plant cell wall material containing lignin, cellulose, and hemicellulose in a mixed matrix. In a preferred aspect, the cellulosic material is any biomass material. In another preferred aspect, the cellulosic material is lignocellulose, which comprises cellulose, hemicelluloses, and lignin.

In one aspect, the cellulosic material is agricultural residue. In another aspect, the cellulosic material is herbaceous material (including energy crops). In another aspect, the cellulosic material is municipal solid waste. In another aspect, the cellulosic material is pulp and paper mill residue. In another aspect, the cellulosic material is waste paper. In another aspect, the cellulosic material is wood (including forestry residue).

In another aspect, the cellulosic material is arundo. In another aspect, the cellulosic material is bagasse. In another aspect, the cellulosic material is bamboo. In another aspect, the cellulosic material is corn cob. In another aspect, the cellulosic material is corn fiber. In another aspect, the cellulosic material is corn stover. In another aspect, the cellulosic material is miscanthus. In another aspect, the cellulosic material is orange peel. In another aspect, the cellulosic material is rice straw. In another aspect, the cellulosic material is switchgrass. In another aspect, the cellulosic material is wheat straw.

In another aspect, the cellulosic material is aspen. In another aspect, the cellulosic material is eucalyptus. In another aspect, the cellulosic material is fir. In another aspect, the cellulosic material is pine. In another aspect, the cellulosic material is poplar. In another aspect, the cellulosic material is spruce. In another aspect, the cellulosic material is willow.

In another aspect, the cellulosic material is algal cellulose. In another aspect, the cellulosic material is bacterial cellulose. In another aspect, the cellulosic material is cotton linter. In another aspect, the cellulosic material is filter paper. In another aspect, the cellulosic material is microcrystalline cellulose. In another aspect, the cellulosic material is phosphoric-acid treated cellulose.

In another aspect, the cellulosic material is an aquatic biomass. As used herein the term "aquatic biomass" means biomass produced in an aquatic environment by a photosynthesis process. The aquatic biomass can be algae, emergent plants, floating-leaf plants, or submerged plants.

The cellulosic material may be used as is or may be subjected to pretreatment, using conventional methods known in the art, as described herein. In a preferred aspect, the cellulosic material is pretreated.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present disclosure. Each control sequence may be native (*i.e.*, from the same gene) or foreign (*i.e.*, from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Endoglucanase:** The term "endoglucanase" means an endo-1,4-(1,3;1,4)-beta-D-glucan 4-glucanohydrolase (E.C. 3.2.1.4) that catalyzes endohydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity can be determined by measuring reduction in substrate viscosity or increase in reducing ends determined by a reducing sugar assay (Zhang et al., 2006, Biotechnology Advances 24: 452-481). Endoglucanase activity may be determined using carboxymethyl cellulose (CMC) as substrate according to the procedure of Ghose, 1987, Pure and Appl. Chem. 59: 257-268, at pH 5, 40°C. In the present invention, the assay described in Example 10 can be used to measure endoglucanase activity.

The polypeptides of the present disclosure have at least 20%, e.g., at least 40%, at least 50%, at least 60%, at least 70%, at least 80%, at least 90%, at least 95%, and at least 100% of the endoglucanase activity of the mature polypeptide of SEQ ID NO: 2.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Family 61 glycoside hydrolase:** The term "Family 61 glycoside hydrolase" or "Family GH61" or "GH61" means a polypeptide falling into the glycoside hydrolase Family 61 according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696. The enzymes in this family were originally classified as a glycoside hydrolase family based on measurement of very weak endo-1,4-beta-D-glucanase activity in one family member. The structure and mode of action of these enzymes are non-canonical and they cannot be considered as bona fide glycosidases. However, they are kept in the CAZy classification on the basis of their capacity to enhance the breakdown of lignocellulose when used in conjunction with a cellulase or a mixture of cellulases.

**Feruloyl esterase:** The term "feruloyl esterase" means a 4-hydroxy-3-methoxycinnamoyl-sugar hydrolase (EC 3.1.1.73) that catalyzes the hydrolysis of 4-hydroxy-3-methoxycinnamoyl (feruloyl) groups from esterified sugar, which is usually arabinose in "natural" substrates, to produce ferulate (4-hydroxy-3-methoxycinnamate). Feruloyl esterase is also known as ferulic acid esterase, hydroxycinnamoyl esterase, FAE-III, cinnamoyl ester hydrolase, FAEA, cinnAE, FAE-I, or FAE-II. For purposes of the present disclosure, feruloyl esterase activity is determined using 0.5 mM *p*-nitrophenylferulate as substrate in 50 mM sodium acetate pH 5.0. One unit of feruloyl esterase equals the amount of enzyme capable of releasing 1 μmole of p-nitrophenolate anion per minute at pH 5, 25°C.

**Fragment:** The term "fragment" means a polypeptide or a domain having one or more (*e.g.*, several) amino acids absent from the amino and/or carboxyl terminus of a mature polypeptide or domain; wherein the fragment has endoglucanase or cellulose binding activity. In one aspect, a fragment contains at least 395 amino acid residues, *e.g.*, at least 420 amino acid residues or at least 445 amino acid residues.

**Hemicellulolytic enzyme or hemicellulase:** The term "hemicellulolytic enzyme" or "hemicellulase" means one or more (e.g., several) enzymes that hydrolyze a hemicellulosic material. See, for example, Shallom, D. and Shoham, Y. Microbial hemicellulases. Current Opinion In Microbiology, 2003, 6(3): 219-228). Hemicellulases are key components in the degradation of plant biomass. Examples of hemicellulases include, but are not limited to, an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. The substrates of these enzymes, the hemicelluloses, are a heterogeneous group of branched and linear polysaccharides that are bound via hydrogen bonds to the cellulose microfibrils in the plant cell wall, crosslinking them into a robust network. Hemicelluloses are also covalently attached to lignin, forming together with cellulose a highly complex structure. The variable structure and organization of hemicelluloses require the concerted action of many enzymes for its complete degradation. The catalytic modules of hemicellulases are either glycoside hydrolases (GHs) that hydrolyze glycosidic bonds, or carbohydrate esterases (CEs), which hydrolyze ester linkages of acetate or ferulic acid side groups. These catalytic modules, based on homology of their primary sequence, can be assigned into GH and CE families. Some families, with an overall similar fold, can be further grouped into clans, marked alphabetically (*e.g.,* GH-A). A most informative and updated classification of these and other carbohydrate active enzymes is available in the Carbohydrate-Active Enzymes (CAZy) database. Hemicellulolytic enzyme activities can be measured according to Ghose and Bisaria, 1987, Pure & Appl. Chem. 59: 1739-1752, at a suitable temperature, *e.g.*, 50°C, 55°C, or 60°C, and pH, *e.g.*, 5.0 or 5.5.

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

**Host cell:** The term "host cell" means any cell type that is susceptible to transformation, transfection, transduction, or the like with a nucleic acid construct or expression vector comprising a polynucleotide of the present disclosure. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., multiple copies of a gene encoding the substance; use of a stronger promoter than the promoter naturally associated with the gene encoding the substance). The polypeptide of the present disclosure may be used in industrial applications in the form of a fermentation broth product, that is, the polypeptide of the present disclosure is a component of a fermentation broth used as a product in industrial applications (*e.g.,* ethanol production). The fermentation broth product will in addition to the polypeptide of the present disclosure comprise additional ingredients used in the fermentation process, such as, for example, cells (including, the host cells containing the gene encoding the polypeptide of the present disclosure which are used to produce the polypeptide of interest), cell debris, biomass, fermentation media and/or fermentation products. The fermentation broth may optionally be subjected to one or more purification (including filtration) steps to remove or reduce one more components of a fermentation process. Accordingly, an isolated substance may be present in such a fermentation broth product.

**Low stringency conditions:** The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide is amino acids 24 to 498 of SEQ ID NO: 2 based on the SignalP program (Nielsen et al., 1997, Protein Engineering 10: 1-6) that predicts amino acids 1 to 23 of SEQ ID NO: 2 are a signal peptide. It is known in the art that a host cell may produce a mixture of two of more different mature polypeptides (*i.e*., with a different C-terminal and/or N-terminal amino acid) expressed by the same polynucleotide. It is also known in the art that different host cells process polypeptides differently, and thus, one host cell expressing a polynucleotide may produce a different mature polypeptide (*e.g.*, having a different C-terminal and/or N-terminal amino acid) as compared to another host cell expressing the same polynucleotide.

**Mature polypeptide coding sequence:** The term "mature polypeptide coding sequence" means a polynucleotide that encodes a mature polypeptide having endoglucanase activity. In one aspect, the mature polypeptide coding sequence is nucleotides 70 to 1494 of SEQ ID NO: 1 based on the SignalP program (Nielsen *et al*., 1997, *supra*) that predicts nucleotides 1 to 69 of SEQ ID NO: 1 encode a signal peptide.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Polypeptide having cellulolytic enhancing activity:** The term "polypeptide having cellulolytic enhancing activity" means a GH61 polypeptide that catalyzes the enhancement of the hydrolysis of a cellulosic material by enzyme having cellulolytic activity. For purposes of the present disclosure, cellulolytic enhancing activity is determined by measuring the increase in reducing sugars or the increase of the total of cellobiose and glucose from the hydrolysis of a cellulosic material by cellulolytic enzyme under the following conditions: 1-50 mg of total protein/g of cellulose in PCS, wherein total protein is comprised of 50-99.5% w/w cellulolytic enzyme protein and 0.5-50% w/w protein of a GH61 polypeptide having cellulolytic enhancing activity for 1-7 days at a suitable temperature, *e.g.,* 50°C, 55°C, or 60°C, and pH, *e.g.,* 5.0 or 5.5, compared to a control hydrolysis with equal total protein loading without cellulolytic enhancing activity (1-50 mg of cellulolytic protein/g of cellulose in PCS). In a preferred aspect, a mixture of CELLUCLAST® 1.5L (Novozymes A/S, Bagsvaerd, Denmark) in the presence of 2-3% of total protein weight *Aspergillus oryzae* beta-glucosidase (recombinantly produced in *Aspergillus oryzae* according to WO 02/095014) or 2-3% of total protein weight *Aspergillus fumigatus* beta-glucosidase (recombinantly produced in *Aspergillus oryzae* as described in WO 2002/095014) of cellulase protein loading is used as the source of the cellulolytic activity.

The GH61 polypeptides having cellulolytic enhancing activity enhance the hydrolysis of a cellulosic material catalyzed by enzyme having cellulolytic activity by reducing the amount of cellulolytic enzyme required to reach the same degree of hydrolysis preferably at least 1.01-fold, *e.g.,* at least 1.05-fold, at least 1.10-fold, at least 1.25-fold, at least 1.5-fold, at least 2-fold, at least 3-fold, at least 4-fold, at least 5-fold, at least 10-fold, or at least 20-fold.

**Pretreated corn stover:** The term "PCS" or "Pretreated Corn Stover" means a cellulosic material derived from corn stover by treatment with heat and dilute sulfuric acid, alkaline pretreatment, or neutral pretreatment.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Subsequence:** The term "subsequence" means a polynucleotide having one or more (*e.g.,* several) nucleotides absent from the 5' and/or 3' end of a mature polypeptide coding sequence or domain; wherein the subsequence encodes a fragment having endoglucanase activity or cellulose binding activity. In one aspect, a subsequence contains at least 1185 nucleotides, e.g., at least 1260 nucleotides or at least 1335 nucleotides

**Variant:** The term "variant" means a polypeptide having endoglucanase activity comprising an alteration, *i.e.*, a substitution, insertion, and/or deletion, at one or more (*e.g.,* several) positions. A substitution means replacement of the amino acid occupying a position with a different amino acid; a deletion means removal of the amino acid occupying a position; and an insertion means adding an amino acid adjacent to and immediately following the amino acid occupying a position.

**Very high stringency conditions:** The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

**Very low stringency conditions:** The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

**Xylan degrading activity or xylanolytic activity:** The term "xylan degrading activity" or "xylanolytic activity" means a biological activity that hydrolyzes xylan-containing material. The two basic approaches for measuring xylanolytic activity include: (1) measuring the total xylanolytic activity, and (2) measuring the individual xylanolytic activities (*e.g.,* endoxylanases, beta-xylosidases, arabinofuranosidases, alpha-glucuronidases, acetylxylan esterases, feruloyl esterases, and alpha-glucuronyl esterases). Recent progress in assays of xylanolytic enzymes was summarized in several publications including Biely and Puchard, Recent progress in the assays of xylanolytic enzymes, 2006, Journal of the Science of Food and Agriculture 86(11): 1636-1647; Spanikova and Biely, 2006, Glucuronoyl esterase - Novel carbohydrate esterase produced by Schizophyllum commune, FEBS Letters 580(19): 4597-4601; Herrmann, Vrsanska, Jurickova, Hirsch, Biely, and Kubicek, 1997, The beta-D-xylosidase of Trichoderma reesei is a multifunctional beta-D-xylan xylohydrolase, Biochemical Journal 321: 375-381.

Total xylan degrading activity can be measured by determining the reducing sugars formed from various types of xylan, including, for example, oat spelt, beechwood, and larchwood xylans, or by photometric determination of dyed xylan fragments released from various covalently dyed xylans. The most common total xylanolytic activity assay is based on production of reducing sugars from polymeric 4-O-methyl glucuronoxylan as described in Bailey, Biely, Poutanen, 1992, Interlaboratory testing of methods for assay of xylanase activity, Journal of Biotechnology 23(3): 257-270. Xylanase activity can also be determined with 0.2% AZCL-arabinoxylan as substrate in 0.01% TRITON® X-100 (4-(1,1,3,3-tetramethylbutyl)phenyl-polyethylene glycol) and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

For purposes of the present disclosure, xylan degrading activity is determined by measuring the increase in hydrolysis of birchwood xylan (Sigma Chemical Co., Inc., St. Louis, MO, USA) by xylan-degrading enzyme(s) under the following typical conditions: 1 ml reactions, 5 mg/ml substrate (total solids), 5 mg of xylanolytic protein/g of substrate, 50 mM sodium acetate pH 5, 50°C, 24 hours, sugar analysis using *p*-hydroxybenzoic acid hydrazide (PHBAH) assay as described by Lever, 1972, A new reaction for colorimetric determination of carbohydrates, Anal. Biochem 47: 273-279.

**Xylanase:** The term "xylanase" means a 1,4-beta-D-xylan-xylohydrolase (E.C. 3.2.1.8) that catalyzes the endohydrolysis of 1,4-beta-D-xylosidic linkages in xylans. For purposes of the present disclosure, xylanase activity is determined with 0.2% AZCL-arabinoxylan as substrate in 0.01% TRITON® X-100 and 200 mM sodium phosphate buffer pH 6 at 37°C. One unit of xylanase activity is defined as 1.0 μmole of azurine produced per minute at 37°C, pH 6 from 0.2% AZCL-arabinoxylan as substrate in 200 mM sodium phosphate pH 6 buffer.

## Detailed Description of the Invention

### Polypeptides Having Endoglucanase Activity

In one aspect, the present disclosure relates to isolated polypeptides having a sequence identity to the mature polypeptide of SEQ ID NO: 2 of at least 90%, e.g., at least 91%, at least 92%, at least 93%, at least 94%, at least 95%, at least 96%, at least 97%, at least 98%, at least 99%, or 100%, which have endoglucanase activity. In one aspect, the polypeptides differ by up to 10 amino acids, *e.g.,* 1, 2, 3, 4, 5, 6, 7, 8, 9, or 10, from the mature polypeptide of SEQ ID NO: 2.

A polypeptide of the present disclosure preferably comprises or consists of the amino acid sequence of SEQ ID NO: 2 or an allelic variant thereof; or is a fragment thereof having endoglucanase activity. In another aspect, the polypeptide comprises or consists of the mature polypeptide of SEQ ID NO: 2. In another aspect, the polypeptide comprises or consists of amino acids 24 to 498 of SEQ ID NO: 2.

In another aspect, the present disclosure relates to isolated polypeptides having endoglucanase activity that are encoded by polynucleotides that hybridize under very low stringency conditions, low stringency conditions, medium stringency conditions, medium-high stringency conditions, high stringency conditions, or very high stringency conditions with the mature polypeptide coding sequence of SEQ ID NO: 1 or the full-length complement thereof (Sambrook et al., 1989, Molecular Cloning, A Laboratory Manual, 2d edition, Cold Spring Harbor, New York).

The polynucleotide of SEQ ID NO: 1 or a subsequence thereof, as well as the polypeptide of SEQ ID NO: 2 or a fragment thereof, may be used to design nucleic acid probes to identify and clone DNA encoding polypeptides having endoglucanase activity from strains of different genera or species according to methods well known in the art. In particular, such probes can be used for hybridization with the genomic DNA or cDNA of a cell of interest, following standard Southern blotting procedures, in order to identify and isolate the corresponding gene therein. Such probes can be considerably shorter than the entire sequence, but should be at least 15, e.g., at least 25, at least 35, or at least 70 nucleotides in length. Preferably, the nucleic acid probe is at least 100 nucleotides in length, e.g., at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present disclosure.

A genomic DNA or cDNA library prepared from such other strains may be screened for DNA that hybridizes with the probes described above and encodes a polypeptide having endoglucanase activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that hybridizes with SEQ ID NO: 1 or a subsequence thereof, the carrier material is used in a Southern blot.

For purposes of the present disclosure, hybridization indicates that the polynucleotide hybridizes to a labeled nucleic acid probe corresponding to (i) SEQ ID NO: 1; (ii) the mature polypeptide coding sequence of SEQ ID NO: 1; (iii) the full-length complement thereof; or (iv) a subsequence thereof; under very low to very high stringency conditions. Molecules to which the nucleic acid probe hybridizes under these conditions can be detected using, for example, X-ray film or any other detection means known in the art.

In one aspect, the nucleic acid probe is the mature polypeptide coding sequence of SEQ ID NO: 1. In another aspect, the nucleic acid probe is nucleotides 70 to 1494 of SEQ ID NO: 1. In another aspect, the nucleic acid probe is a polynucleotide that encodes the polypeptide of SEQ ID NO: 2 or the mature polypeptide thereof; or a fragment thereof. In another preferred aspect, the nucleic acid probe is SEQ ID NO: 1. In another aspect, the nucleic acid probe is the polynucleotide contained in plasmid pGEM-T-Ppin13 which is contained in *E. coli* DSM 24144, wherein the polynucleotide encodes a polypeptide having endoglucanase activity. In another aspect, the nucleic acid probe is the mature polypeptide coding region contained in plasmid pGEM-T-Ppin13 which is contained in *E. coli* DSM 24144.

In another aspect, the present disclosure relates to isolated polypeptides having endoglucanase activity encoded by polynucleotides having a sequence identity to the mature polypeptide coding sequence of SEQ ID NO: 1 of at least 90%, e.g., at least 91%, at least 92%, at least 93%, at least 94%, at least 95%, at least 96%, at least 97%, at least 98%, at least 99%, or 100%.

In another aspect, the present disclosure relates to variants of the mature polypeptide of SEQ ID NO: 2 comprising a substitution, deletion, and/or insertion at one or more (e.g., several) positions. In an aspect, the number of amino acid substitutions, deletions and/or insertions introduced into the mature polypeptide of SEQ ID NO: 2 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for endoglucanase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present disclosure. A fusion polypeptide is produced by fusing a polynucleotide encoding another polypeptide to a polynucleotide of the present disclosure. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

**Sources of Polypeptides Having Endoglucanase Activity**

A polypeptide having endoglucanase activity of the present disclosure may be obtained from microorganisms of any genus. For purposes of the present disclosure, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

The polypeptide may be a bacterial polypeptide. For example, the polypeptide may be a Gram-positive bacterial polypeptide such as a *Bacillus, Clostridium*, *Enterococcus*, *Geobacillus, Lactobacillus, Lactococcus, Oceanobacillus, Staphylococcus, Streptococcus,* or *Streptomyces* polypeptide having endoglucanase activity, or a Gram-negative bacterial polypeptide such as a *Campylobacter, E. coli*, *Flavobacterium, Fusobacterium, Helicobacter, Ilyobacter*, *Neisseria*, *Pseudomonas, Salmonella*, or *Ureaplasma* polypeptide.

In one aspect, the polypeptide is a *Bacillus alkalophilus*, *Bacillus amyloliquefaciens*, *Bacillus brevis, Bacillus circulans, Bacillus clausii, Bacillus coagulans, Bacillus firmus, Bacillus lautus*, *Bacillus lentus*, *Bacillus licheniformis, Bacillus megaterium, Bacillus pumilus, Bacillus stearothermophilus, Bacillus subtilis*, or *Bacillus thuringiensis* polypeptide.

In another aspect, the polypeptide is a *Streptococcus equisimilis*, *Streptococcus pyogenes*, *Streptococcus uberis*, or *Streptococcus equi* subsp. *Zooepidemicus* polypeptide.

In another aspect, the polypeptide is a *Streptomyces achromogenes, Streptomyces avermitilis, Streptomyces coelicolor*, *Streptomyces griseus,* or *Streptomyces lividans* polypeptide.

The polypeptide may be a fungal polypeptide. For example, the polypeptide may be a yeast polypeptide such as a *Candida*, *Kluyveromyces, Pichia, Saccharomyces, Schizosaccharomyces,* or *Yarrowia* polypeptide; or a filamentous fungal polypeptide such as an *Acremonium, Agaricus, Alternaria*, *Aspergillus, Aureobasidium, Botryospaeria, Ceriporiopsis, Chaetomidium, Chrysosporium, Claviceps*, *Cochliobolus, Coprinopsis*, *Coptotermes, Corynascus, Cryphonectria, Cryptococcus, Diplodia*, *Exidia*, *Filibasidium, Fusarium, Gibberella, Holomastigotoides, Humicola*, *Irpex*, *Lentinula, Leptospaeria, Magnaporthe*, *Melanocarpus, Meripilus, Mucor, Myceliophthora*, *Neocallimastix*, *Neurospora, Paecilomyces*, *Penicillium*, *Phanerochaete, Piromyces*, *Poitrasia*, *Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* polypeptide.

In another aspect, the polypeptide is a *Saccharomyces carlsbergensis*, *Saccharomyces cerevisiae*, *Saccharomyces diastaticus*, *Saccharomyces douglasii, Saccharomyces kluyveri*, *Saccharomyces norbensis,* or *Saccharomyces oviformis* polypeptide.

In another aspect, the polypeptide is an *Acremonium cellulolyticus, Aspergillus aculeatus, Aspergillus awamori, Aspergillus foetidus, Aspergillus fumigatus*, *Aspergillus japonicus, Aspergillus nidulans*, *Aspergillus niger*, *Aspergillus oryzae*, *Chrysosporium inops*, *Chrysosporium keratinophilum, Chrysosporium lucknowense, Chrysosporium merdarium*, *Chrysosporium pannicola*, *Chrysosporium queenslandicum, Chrysosporium tropicum*, *Chrysosporium zonatum*, *Fusarium bactridioides, Fusarium cerealis*, *Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens*, *Humicola lanuginosa, Irpex lacteus, Mucor miehei, Myceliophthora thermophila, Neurospora crassa, Penicillium funiculosum*, *Penicillium purpurogenum, Phanerochaete chrysosporium, Thielavia achromatica, Thielavia albomyces, Thielavia albopilosa*, *Thielavia australeinsis*, *Thielavia fimeti, Thielavia microspora*, *Thielavia ovispora*, *Thielavia peruviana*, *Thielavia setosa, Thielavia*

*spededonium, Thielavia subthermophila, Thielavia terrestris, Trichoderma harzianum, Trichoderma koningii*, *Trichoderma longibrachiatum, Trichoderma reesei*, or *Trichoderma viride* polypeptide.

In another aspect, the polypeptide is a *Penicillium* polypeptide. In another aspect, the polypeptide is a *Penicillium pinophilum* polypeptide. In another aspect, the polypeptide is a *Penicillium pinophilum* NN046877 polypeptide.

It will be understood that for the aforementioned species the disclosure encompasses both the perfect and imperfect states, and other taxonomic equivalents, *e.g.,* anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The polypeptide may be identified and obtained from other sources including microorganisms isolated from nature (*e.g.,* soil, composts, water, etc.) or DNA samples obtained directly from natural materials (*e.g.,* soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, *e.g.,* Sambrook *et al*., 1989, *supra).*

## Catalytic Domains

In one aspect, the present disclosure relates to catalytic domains having a sequence identity to amino acids 24 to 419 of SEQ ID NO: 2 of at least 91%, e.g., at least 92%, at least 93%, at least 94%, at least 95%, at least 96%, at least 97%, at least 98%, at least 99%, or 100%. In one aspect, the catalytic domains comprise amino acid sequences that differ by up to 10 amino acids, e.g., 1, 2, 3, 4, 5, 6, 7, 8, 9, or 10, from amino acids 24 to 419 of SEQ ID NO: 2.

The catalytic domain preferably comprises or consists of amino acids 24 to 419 of SEQ ID NO: 2 or an allelic variant thereof; or is a fragment thereof having endoglucanase activity.

In another aspect, the present disclosure relates to catalytic domains encoded by polynucleotides that hybridize under very low stringency conditions, low stringency conditions, medium stringency conditions, medium-high stringency conditions, high stringency conditions, and very high stringency conditions (as defined above) with nucleotides 70 to 1257 of SEQ ID NO: 1 or the full-length complement thereof (Sambrook *et al*., 1989, *supra*).

In another aspect, the present disclosure relates to catalytic domains encoded by polynucleotides having a sequence identity to nucleotides 70 to 1257 of SEQ ID NO: 1 of at least 91%, e.g., at least 92%, at least 93%, at least 94%, at least 95%, at least 96%, at least 97%, at least 98%, at least 99%, or 100%.

The polynucleotide encoding the catalytic domain preferably comprises or consists of nucleotides 70 to 1257 of SEQ ID NO: 1, or is the sequence contained in plasmid pGEM-T-Ppin13 which is contained in *E. coli* DSM 24144.

In another aspect, the present disclosure relates to catalytic domain variants of amino acids 24 to 419 of SEQ ID NO: 2 comprising a substitution, deletion, and/or insertion at one or more (e.g., several) positions. In one aspect, the number of amino acid substitutions, deletions and/or insertions introduced into the sequence of amino acids 24 to 419 of SEQ ID NO: 2 is up to 10, *e.g.,* 1, 2, 3, 4, 5, 6, 8, 9, or 10.

## Cellulose Binding Domains

In one aspect, the present disclosure relates to cellulose binding domains having a sequence identity to amino acids 463 to 498 of SEQ ID NO: 2 of at least 91%, e.g., at least 92%, at least 93%, at least 94%, at least 95%, at least 96%, at least 97%, at least 98%, at least 99%, or 100%. In one aspect, the cellulose binding domains comprise amino acid sequences that differ by up to 10 amino acids, *e.g.,* 1, 2, 3, 4, 5, 6, 7, 8, 9, or 10, from amino acids 463 to 498 of SEQ ID NO: 2.

The cellulose binding domain preferably comprises or consists of amino acids 463 to 498 of SEQ ID NO: 2 or an allelic variant thereof; or is a fragment thereof having cellulose binding activity.

In another aspect, the present disclosure relates to cellulose binding domains encoded by polynucleotides that hybridize under very low stringency conditions, low stringency conditions, medium stringency conditions, medium-high stringency conditions, high stringency conditions, or very high stringency conditions (as defined above) with the nucleotides 1387 to 1494 of SEQ ID NO: 1, or the full-length complement thereof (Sambrook *et al*., 1989, *supra*).

In another aspect, the present disclosure relates to cellulose binding domains encoded by polynucleotides having a sequence identity to nucleotides 1387 to 1494 of SEQ ID NO: 1 of at least 91%, e.g., at least 92%, at least 93%, at least 94%, at least 95%, at least 96%, at least 97%, at least 98%, at least 99%, or 100%.

The polynucleotide encoding the cellulose binding domain preferably comprises or consists of nucleotides 1387 to 1494 of SEQ ID NO: 1 or is the sequence contained in plasmid pGEM-T-Ppin13 which is contained in *E. coli* DSM 24144.

In another aspect, the present disclosure relates to cellulose binding domain variants of amino acids 463 to 498 of SEQ ID NO: 2 comprising a substitution, deletion, and/or insertion at one or more (e.g., several) positions. In one aspect, the number of amino acid substitutions, deletions and/or insertions introduced into the sequence of amino acids 463 to 498 of SEQ ID NO: 2 is up to 10, *e.g.,* 1, 2, 3, 4, 5, 6, 8, 9, or 10.

The cellulose binding domain can be coupled to another catalytic domain. The catalytic domain may be obtained from an oxidoreductase, transferase, hydrolase, lyase, isomerase, or ligase, *e.g.,* an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phytase, polyphenoloxidase, proteolytic enzyme, ribonuclease, transglutaminase, xylanase, or beta-xylosidase. The polynucleotide encoding the catalytic domain may be obtained from any prokaryotic, eukaryotic, or other source.

**Polynucleotides**

The present disclosure also relates to isolated polynucleotides encoding a polypeptide, a catalytic domain, or cellulose binding domain of the present disclosure, as described herein.

The techniques used to isolate or clone a polynucleotide are known in the art and include isolation from genomic DNA or cDNA, or a combination thereof. The cloning of the polynucleotides from genomic DNA can be effected, *e.g.,* by using the well known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of *Penicillium*, or a related organism and thus, for example, may be an allelic or species variant of the polypeptide encoding region of the polynucleotide.

Modification of a polynucleotide encoding a polypeptide of the present disclosure may be necessary for synthesizing polypeptides substantially similar to the polypeptide. The term "substantially similar" to the polypeptide refers to non-naturally occurring forms of the polypeptide. These polypeptides may differ in some engineered way from the polypeptide isolated from its native source, e.g., variants that differ in specific activity, thermostability, pH optimum, or the like. The variants may be constructed on the basis of the polynucleotide presented as the mature polypeptide coding sequence of SEQ ID NO: 1 by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, see, *e.g.,* Ford et al., 1991, Protein Expression and Purification 2: 95-107.

**Nucleic Acid Constructs**

The present disclosure also relates to nucleic acid constructs comprising a polynucleotide of the present disclosure operably linked to one or more (*e.g.,* several) control sequences that direct the expression of the coding sequence in a suitable host cell under conditions compatible with the control sequences.

A polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present disclosure. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present disclosure in a bacterial host cell are the promoters obtained from the *Bacillus amyloliquefaciens* alpha-amylase gene (*amy*Q), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene *(dagA),* and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.*, 1989, *supra.* Examples of tandem promoters are disclosed in WO 99/43835.

Examples of suitable promoters for directing transcription of the nucleic acid constructs of the present disclosure in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase *(glaA), Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (*aprH*), *Bacillus licheniformis* alpha-amylase (*amyL*), and *Escherichia coli* ribosomal RNA (*rrnB*).

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al*., 1992, *supra.*

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis crylIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT*, *nprS*, *nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al*., 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac*, *tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

**Expression Vectors**

The present disclosure also relates to recombinant expression vectors comprising a polynucleotide of the present disclosure, a promoter, and transcriptional and translational stop signals. The various nucleotide and control sequences may be joined together to produce a recombinant expression vector that may include one or more (*e.g.,* several) convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.,* a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.*, a vector that exists as an extrachromosomal entity, the replication of which is independent of chromosomal replication, *e.g.,* a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more (*e.g.,* several) selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosyl-aminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA*, *adeB*, *amdS*, *hph,* and *pyrG* genes.

The selectable marker may be a dual selectable marker system as described in WO 2010/039889. In one aspect, the dual selectable marker is a *hph-tk* dual selectable marker system.

The vector preferably contains an element(s) that permits integration of the vector into the host cell's genome or autonomous replication of the vector in the cell independent of the genome.

For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli*, and pUB110, pE194, pTA1060, and pAMß1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present disclosure may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present disclosure are well known to one skilled in the art (see, *e.g.,* Sambrook *et al.*, 1989, *supra*).

**Host Cells**

The present disclosure also relates to recombinant host cells, comprising a polynucleotide of the present disclosure operably linked to one or more (e.g., several) control sequences that direct the production of a polypeptide of the present disclosure. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present disclosure, *e.g.,* a prokaryote or a eukaryote.

The prokaryotic host cell may be any Gram-positive or Gram-negative bacterium. Gram-positive bacteria include, but are not limited to, *Bacillus, Clostridium, Enterococcus*, *Geobacillus, Lactobacillus, Lactococcus, Oceanobacillus, Staphylococcus, Streptococcus,* and *Streptomyces.* Gram-negative bacteria include, but are not limited to, *Campylobacter, E. coli*, *Flavobacterium, Fusobacterium, Helicobacter, Ilyobacter*, *Neisseria*, *Pseudomonas*, *Salmonella*, and *Ureaplasma.*

The bacterial host cell may be any *Bacillus* cell including, but not limited to, *Bacillus alkalophilus*, *Bacillus amyloliquefaciens*, *Bacillus brevis, Bacillus circulans, Bacillus clausii, Bacillus coagulans, Bacillus firmus, Bacillus lautus, Bacillus lentus*, *Bacillus licheniformis, Bacillus megaterium, Bacillus pumilus, Bacillus stearothermophilus, Bacillus subtilis*, and *Bacillus thuringiensis* cells.

The bacterial host cell may also be any *Streptococcus* cell including, but not limited to, *Streptococcus equisimilis*, *Streptococcus pyogenes*, *Streptococcus uberis,* and *Streptococcus equi* subsp. *Zooepidemicus* cells.

The bacterial host cell may also be any *Streptomyces* cell including, but not limited to, *Streptomyces achromogenes, Streptomyces avermitilis, Streptomyces coelicolor*, *Streptomyces griseus,* and *Streptomyces lividans* cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this disclosure, yeast shall be defined as described in *Biology and Activities of Yeast* (Skinner, Passmore, and Davenport, editors, Soc. App. Bacteriol. Symposium Series No. 9, 1980).

The yeast host cell may be a *Candida*, *Hansenula*, *Kluyveromyces, Pichia, Saccharomyces, Schizosaccharomyces*, or *Yarrowia* cell, such as a *Kluyveromyces lactis*, *Saccharomyces carlsbergensis, Saccharomyces cerevisiae*, *Saccharomyces diastaticus, Saccharomyces douglasii, Saccharomyces kluyveri*, *Saccharomyces norbensis*, *Saccharomyces oviformis*, or *Yarrowia lipolytica* cell.

The fungal host cell may be a filamentous fungal cell. "Filamentous fungi" include all filamentous forms of the subdivision Eumycota and Oomycota (as defined by Hawksworth *et al*., 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola*, *Magnaporthe, Mucor, Myceliophthora, Neocallimastix*, *Neurospora, Paecilomyces*, *Penicillium*, *Phanerochaete, Phlebia, Piromyces*, *Pleurotus, Schizophyllum, Talaromyces*, *Thermoascus, Thielavia*, *Tolypocladium, Trametes,* or *Trichoderma* cell.

For example, the filamentous fungal host cell may be an *Aspergillus awamori, Aspergillus foetidus, Aspergillus fumigatus*, *Aspergillus japonicus, Aspergillus nidulans*, *Aspergillus niger*, *Aspergillus oryzae*, *Bjerkandera adusta, Ceriporiopsis aneirina*, *Ceriporiopsis caregiea*, *Ceriporiopsis gilvescens, Ceriporiopsis pannocinta, Ceriporiopsis rivulosa*, *Ceriporiopsis subrufa, Ceriporiopsis subvermispora, Chrysosporium inops*, *Chrysosporium keratinophilum, Chrysosporium lucknowense, Chrysosporium merdarium*, *Chrysosporium pannicola*, *Chrysosporium queenslandicum, Chrysosporium tropicum, Chrysosporium zonatum*, *Coprinus cinereus, Coriolus hirsutus, Fusarium bactridioides, Fusarium cerealis*, *Fusarium crookwellense, Fusarium culmorum, Fusarium graminearum, Fusarium graminum, Fusarium heterosporum, Fusarium negundi, Fusarium oxysporum*, *Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens*, *Humicola lanuginosa, Mucor miehei, Myceliophthora thermophila*, *Neurospora crassa, Penicillium purpurogenum, Phanerochaete chrysosporium, Phlebia radiata, Pleurotus eryngii*, *Thielavia terrestris, Trametes villosa*, *Trametes versicolor, Trichoderma harzianum, Trichoderma koningii*, *Trichoderma longibrachiatum, Trichoderma reesei*, or *Trichoderma viride* cell.

Fungal cells may be transformed by a process involving protoplast formation, transformation of the protoplasts, and regeneration of the cell wall in a manner known *per* se. Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

## Methods of Production

The present disclosure also relates to methods of producing a polypeptide of the present disclosure, comprising: (a) cultivating a cell, which in its wild-type form produces the polypeptide, under conditions conducive for production of the polypeptide; and (b) recovering the polypeptide. In one aspect, the cell is of the genus *Penicillium.* In another aspect, the cell is *Penicillium pinophilum.* In another aspect, the cell is *Penicillium pinophilum* NN046877.

The present disclosure also relates to methods of producing a polypeptide of the present disclosure, comprising (a) cultivating a recombinant host cell of the present disclosure under conditions conducive for production of the polypeptide; and (b) recovering the polypeptide.

The host cells are cultivated in a nutrient medium suitable for production of the polypeptide using methods known in the art. For example, the cell may be cultivated by shake flask cultivation, or small-scale or large-scale fermentation (including continuous, batch, fed-batch, or solid state fermentations) in laboratory or industrial fermentors in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods include, but are not limited to, use of specific antibodies, formation of an enzyme product, or disappearance of an enzyme substrate. For example, an enzyme assay may be used to determine the activity of the polypeptide.

The polypeptide may be recovered using methods known in the art. For example, the polypeptide may be recovered from the nutrient medium by conventional procedures including, but not limited to, collection, centrifugation, filtration, extraction, spray-drying, evaporation, or precipitation. In one aspect, the whole fermentation broth is recovered.

The polypeptide may be purified by a variety of procedures known in the art including, but not limited to, chromatography (*e.g.,* ion exchange, affinity, hydrophobic, chromatofocusing, and size exclusion), electrophoretic procedures (*e.g.,* preparative isoelectric focusing), differential solubility (*e.g.,* ammonium sulfate precipitation), SDS-PAGE, or extraction (see, *e.g.,* Protein Purification, Janson and Ryden, editors, VCH Publishers, New York, 1989) to obtain substantially pure polypeptides.

In an alternative aspect, the polypeptide is not recovered, but rather a host cell of the present disclosure expressing the polypeptide is used as a source of the polypeptide.

## Plants

The present disclosure also relates to isolated plants, *e.g.,* a transgenic plant, plant part, or plant cell, comprising a polynucleotide of the present disclosure so as to express and produce a polypeptide or domain in recoverable quantities. The polypeptide or domain may be recovered from the plant or plant part. Alternatively, the plant or plant part containing the polypeptide or domain may be used as such for improving the quality of a food or feed, *e.g.,* improving nutritional value, palatability, and rheological properties, or to destroy an antinutritive factor.

The transgenic plant can be dicotyledonous (a dicot) or monocotyledonous (a monocot). Examples of monocot plants are grasses, such as meadow grass (blue grass, Poa), forage grass such as Festuca, Lolium, temperate grass, such as Agrostis, and cereals, *e.g.,* wheat, oats, rye, barley, rice, sorghum, and maize (corn).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.*

Examples of plant parts are stem, callus, leaves, root, fruits, seeds, and tubers as well as the individual tissues comprising these parts, *e.g.,* epidermis, mesophyll, parenchyme, vascular tissues, meristems. Specific plant cell compartments, such as chloroplasts, apoplasts, mitochondria, vacuoles, peroxisomes and cytoplasm are also considered to be a plant part. Furthermore, any plant cell, whatever the tissue origin, is considered to be a plant part. Likewise, plant parts such as specific tissues and cells isolated to facilitate the utilization of the disclosure are also considered plant parts, *e.g.,* embryos, endosperms, aleurone and seed coats.

Also included within the scope of the present disclosure are the progeny of such plants, plant parts, and plant cells.

The transgenic plant or plant cell expressing the polypeptide or domain may be constructed in accordance with methods known in the art. In short, the plant or plant cell is constructed by incorporating one or more expression constructs encoding the polypeptide or domain into the plant host genome or chloroplast genome and propagating the resulting modified plant or plant cell into a transgenic plant or plant cell.

The expression construct is conveniently a nucleic acid construct that comprises a polynucleotide encoding a polypeptide or domain operably linked with appropriate regulatory sequences required for expression of the polynucleotide in the plant or plant part of choice. Furthermore, the expression construct may comprise a selectable marker useful for identifying plant cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences, is determined, for example, on the basis of when, where, and how the polypeptide or domain is desired to be expressed. For instance, the expression of the gene encoding a polypeptide or domain may be constitutive or inducible, or may be developmental, stage or tissue specific, and the gene product may be targeted to a specific tissue or plant part such as seeds or leaves. Regulatory sequences are, for example, described by Tague et al., 1988, Plant Physiology 86: 506.

For constitutive expression, the 35S-CaMV, the maize ubiquitin 1, or the rice actin 1 promoter may be used (Franck et al., 1980, Cell 21: 285-294; Christensen et al., 1992, Plant Mol. Biol. 18: 675-689; Zhang et al., 1991, Plant Cell 3: 1155-1165). Organ-specific promoters may be, for example, a promoter from storage sink tissues such as seeds, potato tubers, and fruits (Edwards and Coruzzi, 1990, Ann. Rev. Genet. 24: 275-303), or from metabolic sink tissues such as meristems (Ito et al., 1994, Plant Mol. Biol. 24: 863-878), a seed specific promoter such as the glutelin, prolamin, globulin, or albumin promoter from rice (Wu et al., 1998, Plant Cell Physiol. 39: 885-889), a *Vicia faba* promoter from the legumin B4 and the unknown seed protein gene from *Vicia faba* (Conrad et al., 1998, J. Plant Physiol. 152: 708-711), a promoter from a seed oil body protein (Chen et al., 1998, Plant Cell Physiol. 39: 935-941), the storage protein *napA* promoter from *Brassica napus,* or any other seed specific promoter known in the art, e.g., as described in WO 91/14772. Furthermore, the promoter may be a leaf specific promoter such as the *rbcs* promoter from rice or tomato (Kyozuka et al., 1993, Plant Physiol. 102: 991-1000), the chlorella virus adenine methyltransferase gene promoter (Mitra and Higgins, 1994, Plant Mol. Biol. 26: 85-93), the *aldP* gene promoter from rice (Kagaya et al., 1995, Mol. Gen. Genet. 248: 668-674), or a wound inducible promoter such as the potato *pin2* promoter (Xu et al., 1993, Plant Mol. Biol. 22: 573-588). Likewise, the promoter may be induced by abiotic treatments such as temperature, drought, or alterations in salinity or induced by exogenously applied substances that activate the promoter, *e.g.,* ethanol, oestrogens, plant hormones such as ethylene, abscisic acid, and gibberellic acid, and heavy metals.

A promoter enhancer element may also be used to achieve higher expression of a polypeptide or domain in the plant. For instance, the promoter enhancer element may be an intron that is placed between the promoter and the polynucleotide encoding a polypeptide or domain. For instance, Xu *et al.*, 1993, *supra,* disclose the use of the first intron of the rice actin 1 gene to enhance expression.

The selectable marker gene and any other parts of the expression construct may be chosen from those available in the art.

The nucleic acid construct is incorporated into the plant genome according to conventional techniques known in the art, including *Agrobacterium-mediated* transformation, virus-mediated transformation, microinjection, particle bombardment, biolistic transformation, and electroporation (Gasser et al., 1990, Science 244: 1293; Potrykus, 1990, Bio/Technology 8: 535; Shimamoto et al., 1989, Nature 338: 274).

*Agrobacterium tumefaciens*-mediated gene transfer is a method for generating transgenic dicots (for a review, see Hooykas and Schilperoort, 1992, Plant Mol. Biol. 19: 15-38) and for transforming monocots, although other transformation methods may be used for these plants. A method for generating transgenic monocots is particle bombardment (microscopic gold or tungsten particles coated with the transforming DNA) of embryonic calli or developing embryos (Christou, 1992, Plant J. 2: 275-281; Shimamoto, 1994, Curr. Opin. Biotechnol. 5: 158-162; Vasil et al., 1992, Bio/Technology 10: 667-674). An alternative method for transformation of monocots is based on protoplast transformation as described by Omirulleh et al., 1993, Plant Mol. Biol. 21: 415-428. Additional transformation methods include those described in U.S. Patent Nos. 6,395,966 and 7,151,204.

Following transformation, the transformants having incorporated the expression construct are selected and regenerated into whole plants according to methods well known in the art. Often the transformation procedure is designed for the selective elimination of selection genes either during regeneration or in the following generations by using, for example, co-transformation with two separate T-DNA constructs or site specific excision of the selection gene by a specific recombinase.

In addition to direct transformation of a particular plant genotype with a construct of the present disclosure, transgenic plants may be made by crossing a plant having the construct to a second plant lacking the construct. For example, a construct encoding a polypeptide or domain can be introduced into a particular plant variety by crossing, without the need for ever directly transforming a plant of that given variety. Therefore, the present disclosure encompasses not only a plant directly regenerated from cells which have been transformed in accordance with the present disclosure, but also the progeny of such plants. As used herein, progeny may refer to the offspring of any generation of a parent plant prepared in accordance with the present disclosure. Such progeny may include a DNA construct prepared in accordance with the present disclosure. Crossing results in the introduction of a transgene into a plant line by cross pollinating a starting line with a donor plant line. Non-limiting examples of such steps are described in U.S. Patent No. 7,151,204.

Plants may be generated through a process of backcross conversion. For example, plants include plants referred to as a backcross converted genotype, line, inbred, or hybrid.

Genetic markers may be used to assist in the introgression of one or more transgenes of the disclosure from one genetic background into another. Marker assisted selection offers advantages relative to conventional breeding in that it can be used to avoid errors caused by phenotypic variations. Further, genetic markers may provide data regarding the relative degree of elite germplasm in the individual progeny of a particular cross. For example, when a plant with a desired trait which otherwise has a non-agronomically desirable genetic background is crossed to an elite parent, genetic markers may be used to select progeny which not only possess the trait of interest, but also have a relatively large proportion of the desired germplasm. In this way, the number of generations required to introgress one or more traits into a particular genetic background is minimized.

The present disclosure also relates to methods of producing a polypeptide or domain of the present disclosure comprising (a) cultivating a transgenic plant or a plant cell comprising a polynucleotide encoding the polypeptide or domain under conditions conducive for production of the polypeptide or domain; and (b) recovering the polypeptide or domain.

**Removal or Reduction of Endoglucanase Activity**

The present invention also relates to methods of producing a mutant of a parent cell, which comprises disrupting or deleting a polynucleotide, or a portion thereof, encoding a polypeptide of the present invention, which results in the mutant cell producing less of the polypeptide than the parent cell when cultivated under the same conditions.

The mutant cell may be constructed by reducing or eliminating expression of the polynucleotide using methods well known in the art, for example, insertions, disruptions, replacements, or deletions. In a preferred aspect, the polynucleotide is inactivated. The polynucleotide to be modified or inactivated may be, for example, the coding region or a part thereof essential for activity, or a regulatory element required for expression of the coding region. An example of such a regulatory or control sequence may be a promoter sequence or a functional part thereof, *i.e.*, a part that is sufficient for affecting expression of the polynucleotide. Other control sequences for possible modification include, but are not limited to, a leader, polyadenylation sequence, propeptide sequence, signal peptide sequence, transcription terminator, and transcriptional activator.

Modification or inactivation of the polynucleotide may be performed by subjecting the parent cell to mutagenesis and selecting for mutant cells in which expression of the polynucleotide has been reduced or eliminated. The mutagenesis, which may be specific or random, may be performed, for example, by use of a suitable physical or chemical mutagenizing agent, by use of a suitable oligonucleotide, or by subjecting the DNA sequence to PCR generated mutagenesis. Furthermore, the mutagenesis may be performed by use of any combination of these mutagenizing agents.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues.

When such agents are used, the mutagenesis is typically performed by incubating the parent cell to be mutagenized in the presence of the mutagenizing agent of choice under suitable conditions, and screening and/or selecting for mutant cells exhibiting reduced or no expression of the gene.

Modification or inactivation of the polynucleotide may be accomplished by insertion, substitution, or deletion of one or more nucleotides in the gene or a regulatory element required for transcription or translation thereof. For example, nucleotides may be inserted or removed so as to result in the introduction of a stop codon, the removal of the start codon, or a change in the open reading frame. Such modification or inactivation may be accomplished by site-directed mutagenesis or PCR generated mutagenesis in accordance with methods known in the art. Although, in principle, the modification may be performed *in vivo, i.e.*, directly on the cell expressing the polynucleotide to be modified, it is preferred that the modification be performed *in vitro* as exemplified below.

An example of a convenient way to eliminate or reduce expression of a polynucleotide is based on techniques of gene replacement, gene deletion, or gene disruption. For example, in the gene disruption method, a nucleic acid sequence corresponding to the endogenous polynucleotide is mutagenized *in vitro* to produce a defective nucleic acid sequence that is then transformed into the parent cell to produce a defective gene. By homologous recombination, the defective nucleic acid sequence replaces the endogenous polynucleotide. It may be desirable that the defective polynucleotide also encodes a marker that may be used for selection of transformants in which the polynucleotide has been modified or destroyed. In an aspect, the polynucleotide is disrupted with a selectable marker such as those described herein.

The present disclosure also relates to methods of inhibiting the expression of a polypeptide having endoglucanase activity in a cell, comprising administering to the cell or expressing in the cell a double-stranded RNA (dsRNA) molecule, wherein the dsRNA comprises a subsequence of a polynucleotide of the present disclosure. In a preferred aspect, the dsRNA is about 15, 16, 17, 18, 19, 20, 21, 22, 23, 24, 25 or more duplex nucleotides in length.

The dsRNA is preferably a small interfering RNA (siRNA) or a micro RNA (miRNA). In a preferred aspect, the dsRNA is small interfering RNA for inhibiting transcription. In another preferred aspect, the dsRNA is micro RNA for inhibiting translation.

The present disclosure also relates to such double-stranded RNA (dsRNA) molecules, comprising a portion of the mature polypeptide coding sequence of SEQ ID NO: 1 for inhibiting expression of the polypeptide in a cell. While the present disclosure is not limited by any particular mechanism of action, the dsRNA can enter a cell and cause the degradation of a single-stranded RNA (ssRNA) of similar or identical sequences, including endogenous mRNAs. When a cell is exposed to dsRNA, mRNA from the homologous gene is selectively degraded by a process called RNA interference (RNAi).

The dsRNAs of the present disclosure can be used in gene-silencing. In one aspect, the disclosure provides methods to selectively degrade RNA using a dsRNAi of the present disclosure. The process may be practiced *in vitro*, *ex vivo* or *in vivo.* In one aspect, the dsRNA molecules can be used to generate a loss-of-function mutation in a cell, an organ or an animal. Methods for making and using dsRNA molecules to selectively degrade RNA are well known in the art; see, for example, U.S. Patent Nos. 6,489,127; 6,506,559; 6,511,824; and 6,515,109.

The present disclosure further relates to a mutant cell of a parent cell that comprises a disruption or deletion of a polynucleotide encoding the polypeptide or a control sequence thereof or a silenced gene encoding the polypeptide, which results in the mutant cell producing less of the polypeptide or no polypeptide compared to the parent cell.

The polypeptide-deficient mutant cells are particularly useful as host cells for expression of native and heterologous polypeptides. Therefore, the present disclosure further relates to methods of producing a native or heterologous polypeptide, comprising: (a) cultivating the mutant cell under conditions conducive for production of the polypeptide; and (b) recovering the polypeptide. The term "heterologous polypeptides" means polypeptides that are not native to the host cell, *e.g.,* a variant of a native protein. The host cell may comprise more than one copy of a polynucleotide encoding the native or heterologous polypeptide.

The methods used for cultivation and purification of the product of interest may be performed by methods known in the art.

The methods of the present disclosure for producing an essentially endoglucanase-free product is of particular interest in the production of eukaryotic polypeptides, in particular fungal proteins such as enzymes. The endoglucanase-deficient cells may also be used to express heterologous proteins of pharmaceutical interest such as hormones, growth factors, receptors, and the like. The term "eukaryotic polypeptides" includes not only native polypeptides, but also those polypeptides, *e.g.,* enzymes, which have been modified by amino acid substitutions, deletions or additions, or other such modifications to enhance activity, thermostability, pH tolerance and the like.

In a further aspect, the present disclosure relates to a protein product essentially free from endoglucanase activity that is produced by a method of the present disclosure.

**Compositions**

The present disclosure also relates to compositions comprising a polypeptide of the present disclosure. Preferably, the compositions are enriched in such a polypeptide. The term "enriched" indicates that the endoglucanase activity of the composition has been increased, e.g., with an enrichment factor of at least 1.1.

The compositions may comprise a polypeptide of the present disclosure as the major enzymatic component, e.g., a mono-component composition. Alternatively, the compositions may comprise multiple enzymatic activities, such as one or more (*e.g.,* several) enzymes selected from the group consisting of a cellulase, a polypeptide having cellulolytic enhancing activity, a hemicellulase, an esterase, an expansin, a laccase, a ligninolytic enzyme, a pectinase, a peroxidase, a protease, and a swollenin.

The compositions may be prepared in accordance with methods known in the art and may be in the form of a liquid or a dry composition. The compositions may be stabilized in accordance with methods known in the art.

The compositions may be a fermentation broth formulation or a cell composition, as described herein. Consequently, the present disclosure also relates to fermentation broth formulations and cell compositions comprising a polypeptide having endoglucanase activity of the present disclosure. In some aspects, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.,* expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.,* filamentous fungal cells) are removed, *e.g.,* by centrifugation. In some aspects, the fermentation broth contains spent cell culture medium, extracellular enzymes, and viable and/or nonviable microbial cells.

In an aspect, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific aspect, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In one aspect, the killed cells and/or cell debris are removed from a cell-killed whole broth to provide a composition that is free of these components.

The fermentation broth formulations or cell compostions may further comprise a preservative and/or anti-microbial (*e.g.,* bacteriostatic) agent, including, but not limited to, sorbitol, sodium chloride, potassium sorbate, and others known in the art.

The cell-killed whole broth or composition may further comprise one or more enzyme activities such as acetylxylan esterase, alpha-arabinofuranosidase, alpha-galactosidase, alpha-glucuronidase, amylase, arabinanase, arabinofuranosidase, beta-galactosidase, beta-glucosidase, cellobiohydrolase, endoglucanase, endo-beta-1,3(4)-glucanase, ferrulic acid esterase, galactanase, glucoamylase, glucohydrolase, hybrid peroxidases, with combined properties of lignin peroxidases and manganese-dependent peroxidases, laccase, lignin peroxidase, manganese-dependent peroxidases, mannanase, mannan acetyl esterase, mannosidase, pectate lyase, pectin acetyl esterase, pectinase lyase, pectin methyl esterase, polygalacturonase, protease, rhamnogalacturonan lyase, rhamnogalacturonan acetyl esterase, rhamnogalacturonase, xylanase, xylogalacturonosidase, xylogalacturonase, xyloglucanase, and xylosidase.

In some aspects, the cell-killed whole broth or composition includes cellulolytic enzymes including, but not limited to, (i) endoglucanases (EG) or 1,4-D-glucan-4-glucanohydrolases (EC 3.2.1.4), (ii) exoglucanases, including 1,4-D-glucan glucanohydrolases (also known as cellodextnnases) (EC 3.2.1.74) and 1,4-D-glucan cellobiohydrolases (exo-cellobiohydrolases, CBH) (EC 3.2.1.91), and (iii) beta-glucosidase (BG) or beta-glucoside glucohydrolases (EC 3.2.1.21).

The cell-killed whole broth or composition may contain the unfractionated contents of the fermentation materials derived at the end of the fermentation. Typically, the cell-killed whole broth or composition contains the spent culture medium and cell debris present after the microbial cells (*e.g.,* filamentous fungal cells) are grown to saturation, incubated under carbon-limiting conditions to allow protein synthesis (*e.g.,* expression of cellulase and/or glucosidase enzyme(s)). In some aspects, the cell-killed whole broth or composition contains the spent cell culture medium, extracellular enzymes, and killed filamentous fungal cells. In some aspects, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some aspects, insoluble components may be removed to provide a clarified liquid composition.

The whole broth formulations and cell compositions of the present disclosure may be produced by a method described in WO 90/15861 or WO 2010/096673.

Examples are given below of preferred uses of the compositions of the present disclosure. The dosage of the composition and other conditions under which the composition is used may be determined on the basis of methods known in the art.

**Uses**

The present invention is also directed to the following methods for using the polypeptides having endoglucanase activity, or compositions thereof.

The present invention also relates to methods for degrading or converting a cellulosic material, comprising: treating the cellulosic material with an enzyme composition in the presence of a polypeptide having endoglucanase activity of the present disclosure. In one aspect, the method further comprises recovering the degraded or converted cellulosic material. Soluble products of degradation or conversion of the cellulosic material can be separated from insoluble cellulosic material using technology well known in the art such as, for example, centrifugation, filtration, and gravity settling.

The present invention also relates to methods of producing a fermentation product, comprising: (a) saccharifying a cellulosic material with an enzyme composition in the presence of a polypeptide having endoglucanase activity of the present disclosure; (b) fermenting the saccharified cellulosic material with one or more (*e.g.,* several) fermenting microorganisms to produce the fermentation product; and (c) recovering the fermentation product from the fermentation.

The present invention also relates to methods of fermenting a cellulosic material, comprising: fermenting the cellulosic material with one or more (*e.g.,* several) fermenting microorganisms, wherein the cellulosic material is saccharified with an enzyme composition in the presence of a polypeptide having endoglucanase activity of the present disclosure. In one aspect, the fermenting of the cellulosic material produces a fermentation product. In another aspect, the method further comprises recovering the fermentation product from the fermentation.

The methods of the present invention can be used to saccharify the cellulosic material to fermentable sugars and to convert the fermentable sugars to many useful fermentation products, *e.g.,* fuel, potable ethanol, and/or platform chemicals (*e.g.,* acids, alcohols, ketones, gases, and the like). The production of a desired fermentation product from the cellulosic material typically involves pretreatment, enzymatic hydrolysis (saccharification), and fermentation.

The processing of the cellulosic material according to the present invention can be accomplished using methods conventional in the art. Moreover, the methods of the present invention can be implemented using any conventional biomass processing apparatus configured to operate in accordance with the invention.

Hydrolysis (saccharification) and fermentation, separate or simultaneous, include, but are not limited to, separate hydrolysis and fermentation (SHF); simultaneous saccharification and fermentation (SSF); simultaneous saccharification and co-fermentation (SSCF); hybrid hydrolysis and fermentation (HHF); separate hydrolysis and co-fermentation (SHCF); hybrid hydrolysis and co-fermentation (HHCF); and direct microbial conversion (DMC), also sometimes called consolidated bioprocessing (CBP). SHF uses separate process steps to first enzymatically hydrolyze the cellulosic material to fermentable sugars, *e.g.,* glucose, cellobiose, and pentose monomers, and then ferment the fermentable sugars to ethanol. In SSF, the enzymatic hydrolysis of the cellulosic material and the fermentation of sugars to ethanol are combined in one step (Philippidis, G. P., 1996, Cellulose bioconversion technology, in Handbook on Bioethanol: Production and Utilization, Wyman, C. E., ed., Taylor & Francis, Washington, DC, 179-212). SSCF involves the co-fermentation of multiple sugars (Sheehan, J., and Himmel, M., 1999, Enzymes, energy and the environment: A strategic perspective on the U.S. Department of Energy's research and development activities for bioethanol, Biotechnol. Prog. 15: 817-827). HHF involves a separate hydrolysis step, and in addition a simultaneous saccharification and hydrolysis step, which can be carried out in the same reactor. The steps in an HHF process can be carried out at different temperatures, *i.e.*, high temperature enzymatic saccharification followed by SSF at a lower temperature that the fermentation strain can tolerate. DMC combines all three processes (enzyme production, hydrolysis, and fermentation) in one or more (*e.g.,* several) steps where the same organism is used to produce the enzymes for conversion of the cellulosic material to fermentable sugars and to convert the fermentable sugars into a final product (Lynd, L. R., Weimer, P. J., van Zyl, W. H., and Pretorius, I. S., 2002, Microbial cellulose utilization: Fundamentals and biotechnology, Microbiol. Mol. Biol. Reviews 66: 506-577). It is understood herein that any method known in the art comprising pretreatment, enzymatic hydrolysis (saccharification), fermentation, or a combination thereof, can be used in the practicing the methods of the present invention.

A conventional apparatus can include a fed-batch stirred reactor, a batch stirred reactor, a continuous flow stirred reactor with ultrafiltration, and/or a continuous plug-flow column reactor (Fernanda de Castilhos Corazza, Flávio Faria de Moraes, Gisella Maria Zanin and Ivo Neitzel, 2003, Optimal control in fed-batch reactor for the cellobiose hydrolysis, Acta Scientiarum. Technology 25: 33-38; Gusakov, A. V., and Sinitsyn, A. P., 1985, Kinetics of the enzymatic hydrolysis of cellulose: 1. A mathematical model for a batch reactor process, Enz. Microb. Technol. 7: 346-352), an attrition reactor (Ryu, S. K., and Lee, J. M., 1983, Bioconversion of waste cellulose by using an attrition bioreactor, Biotechnol. Bioeng. 25: 53-65), or a reactor with intensive stirring induced by an electromagnetic field (Gusakov, A. V., Sinitsyn, A. P., Davydkin, I. Y., Davydkin, V. Y., Protas, O. V., 1996, Enhancement of enzymatic cellulose hydrolysis using a novel type of bioreactor with intensive stirring induced by electromagnetic field, Appl. Biochem. Biotechnol. 56: 141-153). Additional reactor types include fluidized bed, upflow blanket, immobilized, and extruder type reactors for hydrolysis and/or fermentation.

Pretreatment. In practicing the methods of the present invention, any pretreatment process known in the art can be used to disrupt plant cell wall components of the cellulosic material (Chandra et al., 2007, Substrate pretreatment: The key to effective enzymatic hydrolysis of lignocellulosics?, Adv. Biochem. Engin./Biotechnol. 108: 67-93; Galbe and Zacchi, 2007, Pretreatment of lignocellulosic materials for efficient bioethanol production, Adv. Biochem. Engin./Biotechnol. 108: 41-65; Hendriks and Zeeman, 2009, Pretreatments to enhance the digestibility of lignocellulosic biomass, Bioresource Technol. 100: 10-18; Mosier et al., 2005, Features of promising technologies for pretreatment of lignocellulosic biomass, Bioresource Technol. 96: 673-686; Taherzadeh and Karimi, 2008, Pretreatment of lignocellulosic wastes to improve ethanol and biogas production: A review, Int. J. of Mol. Sci. 9: 1621-1651; Yang and Wyman, 2008, Pretreatment: the key to unlocking low-cost cellulosic ethanol, Biofuels Bioproducts and Biorefining-Biofpr. 2: 26-40).

The cellulosic material can also be subjected to particle size reduction, sieving, presoaking, wetting, washing, and/or conditioning prior to pretreatment using methods known in the art.

Conventional pretreatments include, but are not limited to, steam pretreatment (with or without explosion), dilute acid pretreatment, hot water pretreatment, alkaline pretreatment, lime pretreatment, wet oxidation, wet explosion, ammonia fiber explosion, organosolv pretreatment, and biological pretreatment. Additional pretreatments include ammonia percolation, ultrasound, electroporation, microwave, supercritical $CO_2$, supercritical $H_2O$, ozone, ionic liquid, and gamma irradiation pretreatments.

The cellulosic material can be pretreated before hydrolysis and/or fermentation. Pretreatment is preferably performed prior to the hydrolysis. Alternatively, the pretreatment can be carried out simultaneously with enzyme hydrolysis to release fermentable sugars, such as glucose, xylose, and/or cellobiose. In most cases the pretreatment step itself results in some conversion of biomass to fermentable sugars (even in absence of enzymes).

Steam Pretreatment. In steam pretreatment, the cellulosic material is heated to disrupt the plant cell wall components, including lignin, hemicellulose, and cellulose to make the cellulose and other fractions, e.g., hemicellulose, accessible to enzymes. The cellulosic material is passed to or through a reaction vessel where steam is injected to increase the temperature to the required temperature and pressure and is retained therein for the desired reaction time. Steam pretreatment is preferably performed at 140-250°C, e.g., 160-200°C or 170-190°C, where the optimal temperature range depends on addition of a chemical catalyst. Residence time for the steam pretreatment is preferably 1-60 minutes, *e.g.,* 1-30 minutes, 1-20 minutes, 3-12 minutes, or 4-10 minutes, where the optimal residence time depends on temperature range and addition of a chemical catalyst. Steam pretreatment allows for relatively high solids loadings, so that the cellulosic material is generally only moist during the pretreatment. The steam pretreatment is often combined with an explosive discharge of the material after the pretreatment, which is known as steam explosion, that is, rapid flashing to atmospheric pressure and turbulent flow of the material to increase the accessible surface area by fragmentation (Duff and Murray, 1996, Bioresource Technology 855: 1-33; Galbe and Zacchi, 2002, Appl. Microbiol. Biotechnol. 59: 618-628; U.S. Patent Application No. 20020164730). During steam pretreatment, hemicellulose acetyl groups are cleaved and the resulting acid autocatalyzes partial hydrolysis of the hemicellulose to monosaccharides and oligosaccharides. Lignin is removed to only a limited extent.

Chemical Pretreatment: The term "chemical treatment" refers to any chemical pretreatment that promotes the separation and/or release of cellulose, hemicellulose, and/or lignin. Such a pretreatment can convert crystalline cellulose to amorphous cellulose. Examples of suitable chemical pretreatment processes include, for example, dilute acid pretreatment, lime pretreatment, wet oxidation, ammonia fiber/freeze explosion (AFEX), ammonia percolation (APR), ionic liquid, and organosolv pretreatments.

A catalyst such as $H_2SO_4$ or $SO_2$ (typically 0.3 to 5% w/w) is often added prior to steam pretreatment, which decreases the time and temperature, increases the recovery, and improves enzymatic hydrolysis (Ballesteros et al., 2006, Appl. Biochem. Biotechnol. 129-132: 496-508; Varga et al., 2004, Appl. Biochem. Biotechnol. 113-116: 509-523; Sassner et al., 2006, Enzyme Microb. Technol. 39: 756-762). In dilute acid pretreatment, the cellulosic material is mixed with dilute acid, typically $H_2SO_4$, and water to form a slurry, heated by steam to the desired temperature, and after a residence time flashed to atmospheric pressure. The dilute acid pretreatment can be performed with a number of reactor designs, e.g., plug-flow reactors, counter-current reactors, or continuous counter-current shrinking bed reactors (Duff and Murray, 1996, *supra;* Schell et al., 2004, Bioresource Technol. 91: 179-188; Lee et al., 1999, Adv. Biochem. Eng. Biotechnol. 65: 93-115).

Several methods of pretreatment under alkaline conditions can also be used. These alkaline pretreatments include, but are not limited to, sodium hydroxide, lime, wet oxidation, ammonia percolation (APR), and ammonia fiber/freeze explosion (AFEX).

Lime pretreatment is performed with calcium oxide or calcium hydroxide at temperatures of 85-150°C and residence times from 1 hour to several days (Wyman et al., 2005, Bioresource Technol. 96: 1959-1966; Mosier et al., 2005, Bioresource Technol. 96: 673-686). WO 2006/110891, WO 2006/110899, WO 2006/110900, and WO 2006/110901 disclose pretreatment methods using ammonia.

Wet oxidation is a thermal pretreatment performed typically at 180-200°C for 5-15 minutes with addition of an oxidative agent such as hydrogen peroxide or over-pressure of oxygen (Schmidt and Thomsen, 1998, Bioresource Technol. 64: 139-151; Palonen et al., 2004, Appl. Biochem. Biotechnol. 117: 1-17; Varga et al., 2004, Biotechnol. Bioeng. 88: 567-574; Martin et al., 2006, J. Chem. Technol. Biotechnol. 81: 1669-1677). The pretreatment is performed preferably at 1-40% dry matter, e.g., 2-30% dry matter or 5-20% dry matter, and often the initial pH is increased by the addition of alkali such as sodium carbonate.

A modification of the wet oxidation pretreatment method, known as wet explosion (combination of wet oxidation and steam explosion) can handle dry matter up to 30%. In wet explosion, the oxidizing agent is introduced during pretreatment after a certain residence time. The pretreatment is then ended by flashing to atmospheric pressure (WO 2006/032282).

Ammonia fiber explosion (AFEX) involves treating the cellulosic material with liquid or gaseous ammonia at moderate temperatures such as 90-150°C and high pressure such as 17-20 bar for 5-10 minutes, where the dry matter content can be as high as 60% (Gollapalli et al., 2002, Appl. Biochem. Biotechnol. 98: 23-35; Chundawat et al., 2007, Biotechnol. Bioeng. 96: 219-231; Alizadeh et al., 2005, Appl. Biochem. Biotechnol. 121: 1133-1141; Teymouri et al., 2005, Bioresource Technol. 96: 2014-2018). During AFEX pretreatment cellulose and hemicelluloses remain relatively intact. Lignin-carbohydrate complexes are cleaved.

Organosolv pretreatment delignifies the cellulosic material by extraction using aqueous ethanol (40-60% ethanol) at 160-200°C for 30-60 minutes (Pan et al., 2005, Biotechnol. Bioeng. 90: 473-481; Pan et al., 2006, Biotechnol. Bioeng. 94: 851-861; Kurabi et al., 2005, Appl. Biochem. Biotechnol. 121: 219-230). Sulphuric acid is usually added as a catalyst. In organosolv pretreatment, the majority of hemicellulose and lignin is removed.

Other examples of suitable pretreatment methods are described by Schell et al., 2003, Appl. Biochem. and Biotechnol. Vol. 105-108, p. 69-85, and Mosier et al., 2005, Bioresource Technology 96: 673-686, and U.S. Published Application 2002/0164730.

In one aspect, the chemical pretreatment is preferably carried out as a dilute acid treatment, and more preferably as a continuous dilute acid treatment. The acid is typically sulfuric acid, but other acids can also be used, such as acetic acid, citric acid, nitric acid, phosphoric acid, tartaric acid, succinic acid, hydrogen chloride, or mixtures thereof. Mild acid treatment is conducted in the pH range of preferably 1-5, *e.g.,* 1-4 or 1-2.5. In one aspect, the acid concentration is in the range from preferably 0.01 to 10 wt % acid, *e.g.,* 0.05 to 5 wt % acid or 0.1 to 2 wt % acid. The acid is contacted with the cellulosic material and held at a temperature in the range of preferably 140-200°C, *e.g.,* 165-190°C, for periods ranging from 1 to 60 minutes.

In another aspect, pretreatment takes place in an aqueous slurry. In preferred aspects, the cellulosic material is present during pretreatment in amounts preferably between 10-80 wt %, *e.g.,* 20-70 wt % or 30-60 wt %, such as around 40 wt %. The pretreated cellulosic material can be unwashed or washed using any method known in the art, *e.g.,* washed with water.

Mechanical Pretreatment or Physical Pretreatment: The term "mechanical pretreatment" or "physical pretreatment" refers to any pretreatment that promotes size reduction of particles. For example, such pretreatment can involve various types of grinding or milling (*e.g.,* dry milling, wet milling, or vibratory ball milling).

The cellulosic material can be pretreated both physically (mechanically) and chemically. Mechanical or physical pretreatment can be coupled with steaming/steam explosion, hydrothermolysis, dilute or mild acid treatment, high temperature, high pressure treatment, irradiation (*e.g.,* microwave irradiation), or combinations thereof. In one aspect, high pressure means pressure in the range of preferably about 100 to about 400 psi, *e.g.,* about 150 to about 250 psi. In another aspect, high temperature means temperatures in the range of about 100 to about 300°C, *e.g.,* about 140 to about 200°C. In a preferred aspect, mechanical or physical pretreatment is performed in a batch-process using a steam gun hydrolyzer system that uses high pressure and high temperature as defined above, *e.g.,* a Sunds Hydrolyzer available from Sunds Defibrator AB, Sweden. The physical and chemical pretreatments can be carried out sequentially or simultaneously, as desired.

Accordingly, in a preferred aspect, the cellulosic material is subjected to physical (mechanical) or chemical pretreatment, or any combination thereof, to promote the separation and/or release of cellulose, hemicellulose, and/or lignin.

Biological Pretreatment: The term "biological pretreatment" refers to any biological pretreatment that promotes the separation and/or release of cellulose, hemicellulose, and/or lignin from the cellulosic material. Biological pretreatment techniques can involve applying lignin-solubilizing microorganisms and/or enzymes (see, for example, Hsu, T.-A., 1996, Pretreatment of biomass, in Handbook on Bioethanol: Production and Utilization, Wyman, C. E., ed., Taylor & Francis, Washington, DC, 179-212; Ghosh and Singh, 1993, Physicochemical and biological treatments for enzymatic/microbial conversion of cellulosic biomass, Adv. Appl. Microbiol. 39: 295-333; McMillan, J. D., 1994, Pretreating lignocellulosic biomass: a review, in Enzymatic Conversion of Biomass for Fuels Production, Himmel, M. E., Baker, J. O., and Overend, R. P., eds., ACS Symposium Series 566, American Chemical Society, Washington, DC, chapter 15; Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Olsson and Hahn-Hagerdal, 1996, Fermentation of lignocellulosic hydrolysates for ethanol production, Enz. Microb. Tech. 18: 312-331; and Vallander and Eriksson, 1990, Production of ethanol from lignocellulosic materials: State of the art, Adv. Biochem. Eng./Biotechnol. 42: 63-95).

Saccharification. In the hydrolysis step, also known as saccharification, the cellulosic material, *e.g.,* pretreated, is hydrolyzed to break down cellulose and/or hemicellulose to fermentable sugars, such as glucose, cellobiose, xylose, xylulose, arabinose, mannose, galactose, and/or soluble oligosaccharides. The hydrolysis is performed enzymatically by an enzyme composition in the presence of a polypeptide having endoglucanase activity of the present disclosure. The enzymes of the compositions can be added simultaneously or sequentially.

Enzymatic hydrolysis is preferably carried out in a suitable aqueous environment under conditions that can be readily determined by one skilled in the art. In one aspect, hydrolysis is performed under conditions suitable for the activity of the enzyme(s), *i.e.*, optimal for the enzyme(s). The hydrolysis can be carried out as a fed batch or continuous process where the cellulosic material is fed gradually to, for example, an enzyme containing hydrolysis solution.

The saccharification is generally performed in stirred-tank reactors or fermentors under controlled pH, temperature, and mixing conditions. Suitable process time, temperature and pH conditions can readily be determined by one skilled in the art. For example, the saccharification can last up to 200 hours, but is typically performed for preferably about 12 to about 120 hours, *e.g.,* about 16 to about 72 hours or about 24 to about 48 hours. The temperature is in the range of preferably about 25°C to about 70°C, *e.g.,* about 30°C to about 65°C, about 40°C to about 60°C, or about 50°C to about 55°C. The pH is in the range of preferably about 3 to about 8, *e.g.,* about 3.5 to about 7, about 4 to about 6, or about 5.0 to about 5.5. The dry solids content is in the range of preferably about 5 to about 50 wt %, *e.g.,* about 10 to about 40 wt % or about 20 to about 30 wt %.

The enzyme compositions can comprise any protein useful in degrading the cellulosic material.

In one aspect, the enzyme composition comprises or further comprises one or more (e.g., several) proteins selected from the group consisting of a cellulase, a GH61 polypeptide having cellulolytic enhancing activity, a hemicellulase, an esterase, an expansin, a laccase, a ligninolytic enzyme, a pectinase, a peroxidase, a protease, and a swollenin. In another aspect, the cellulase is preferably one or more (*e.g.,* several) enzymes selected from the group consisting of an endoglucanase, a cellobiohydrolase, and a beta-glucosidase. In another aspect, the hemicellulase is preferably one or more (*e.g.,* several) enzymes selected from the group consisting of an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase.

In another aspect, the enzyme composition comprises one or more (*e.g.,* several) cellulolytic enzymes. In another aspect, the enzyme composition comprises or further comprises one or more (*e.g.,* several) hemicellulolytic enzymes. In another aspect, the enzyme composition comprises one or more (*e.g.,* several) cellulolytic enzymes and one or more (*e.g.,* several) hemicellulolytic enzymes. In another aspect, the enzyme composition comprises one or more (*e.g.,* several) enzymes selected from the group of cellulolytic enzymes and hemicellulolytic enzymes. In another aspect, the enzyme composition comprises an endoglucanase. In another aspect, the enzyme composition comprises a cellobiohydrolase. In another aspect, the enzyme composition comprises a beta-glucosidase. In another aspect, the enzyme composition comprises a polypeptide having cellulolytic enhancing activity. In another aspect, the enzyme composition comprises an endoglucanase and a polypeptide having cellulolytic enhancing activity. In another aspect, the enzyme composition comprises a cellobiohydrolase and a polypeptide having cellulolytic enhancing activity. In another aspect, the enzyme composition comprises a beta-glucosidase and a polypeptide having cellulolytic enhancing activity. In another aspect, the enzyme composition comprises an endoglucanase and a cellobiohydrolase. In another aspect, the enzyme composition comprises an endoglucanase and a beta-glucosidase. In another aspect, the enzyme composition comprises a cellobiohydrolase and a beta-glucosidase. In another aspect, the enzyme composition comprises an endoglucanase, a cellobiohydrolase, and a polypeptide having cellulolytic enhancing activity. In another aspect, the enzyme composition comprises an endoglucanase, a beta-glucosidase, and a polypeptide having cellulolytic enhancing activity. In another aspect, the enzyme composition comprises a cellobiohydrolase, a beta-glucosidase, and a polypeptide having cellulolytic enhancing activity. In another aspect, the enzyme composition comprises an endoglucanase, a cellobiohydrolase, and a beta-glucosidase. In another aspect, the enzyme composition comprises an endoglucanase, a cellobiohydrolase, a beta-glucosidase, and a polypeptide having cellulolytic enhancing activity.

In another aspect, the enzyme composition comprises an acetylmannan esterase. In another aspect, the enzyme composition comprises an acetylxylan esterase. In another aspect, the enzyme composition comprises an arabinanase (*e.g.,* alpha-L-arabinanase). In another aspect, the enzyme composition comprises an arabinofuranosidase (*e.g.,* alpha-L-arabinofuranosidase). In another aspect, the enzyme composition comprises a coumaric acid esterase. In another aspect, the enzyme composition comprises a feruloyl esterase. In another aspect, the enzyme composition comprises a galactosidase (*e.g.,* alpha-galactosidase and/or beta-galactosidase). In another aspect, the enzyme composition comprises a glucuronidase (*e.g.,* alpha-D-glucuronidase). In another aspect, the enzyme composition comprises a glucuronoyl esterase. In another aspect, the enzyme composition comprises a mannanase. In another aspect, the enzyme composition comprises a mannosidase (*e.g.,* beta-mannosidase). In another aspect, the enzyme composition comprises a xylanase. In a preferred aspect, the xylanase is a Family 10 xylanase. In another aspect, the enzyme composition comprises a xylosidase (*e.g.,* beta-xylosidase).

In another aspect, the enzyme composition comprises an esterase. In another aspect, the enzyme composition comprises an expansin. In another aspect, the enzyme composition comprises a laccase. In another aspect, the enzyme composition comprises a ligninolytic enzyme. In a preferred aspect, the ligninolytic enzyme is a manganese peroxidase. In another preferred aspect, the ligninolytic enzyme is a lignin peroxidase. In another preferred aspect, the ligninolytic enzyme is a $H_2O_2$-producing enzyme. In another aspect, the enzyme composition comprises a pectinase. In another aspect, the enzyme composition comprises a peroxidase. In another aspect, the enzyme composition comprises a protease. In another aspect, the enzyme composition comprises a swollenin.

In the methods of the present invention, the enzyme(s) can be added prior to or during saccharification, saccharification and fermentation, or fermentation.

One or more (*e.g.,* several) components of the enzyme composition may be wild-type proteins, recombinant proteins, or a combination of wild-type proteins and recombinant proteins. For example, one or more (*e.g.,* several) components may be native proteins of a cell, which is used as a host cell to express recombinantly one or more (*e.g.,* several) other components of the enzyme composition. One or more (*e.g.,* several) components of the enzyme composition may be produced as monocomponents, which are then combined to form the enzyme composition. The enzyme composition may be a combination of multicomponent and monocomponent protein preparations.

The enzymes used in the methods of the present invention may be in any form suitable for use, such as, for example, a fermentation broth formulation or a cell composition, a cell lysate with or without cellular debris, a semi-purified or purified enzyme preparation, or a host cell as a source of the enzymes. The enzyme composition may be a dry powder or granulate, a non-dusting granulate, a liquid, a stabilized liquid, or a stabilized protected enzyme. Liquid enzyme preparations may, for instance, be stabilized by adding stabilizers such as a sugar, a sugar alcohol or another polyol, and/or lactic acid or another organic acid according to established processes.

The optimum amounts of the enzymes and polypeptides having endoglucanase activity depend on several factors including, but not limited to, the mixture of component cellulolytic enzymes and/or hemicellulolytic enzymes, the cellulosic material, the concentration of cellulosic material, the pretreatment(s) of the cellulosic material, temperature, time, pH, and inclusion of fermenting organism (*e.g.,* yeast for Simultaneous Saccharification and Fermentation).

In one aspect, an effective amount of cellulolytic or hemicellulolytic enzyme to the cellulosic material is about 0.5 to about 50 mg, e.g., about 0.5 to about 40 mg, about 0.5 to about 25 mg, about 0.75 to about 20 mg, about 0.75 to about 15 mg, about 0.5 to about 10 mg, or about 2.5 to about 10 mg per g of the cellulosic material.

In another aspect, an effective amount of a polypeptide having endoglucanase activity to the cellulosic material is about 0.01 to about 50.0 mg, *e.g.,* about 0.01 to about 40 mg, about 0.01 to about 30 mg, about 0.01 to about 20 mg, about 0.01 to about 10 mg, about 0.01 to about 5 mg, about 0.025 to about 1.5 mg, about 0.05 to about 1.25 mg, about 0.075 to about 1.25 mg, about 0.1 to about 1.25 mg, about 0.15 to about 1.25 mg, or about 0.25 to about 1.0 mg per g of the cellulosic material.

In another aspect, an effective amount of a polypeptide having endoglucanase activity to cellulolytic or hemicellulolytic enzyme is about 0.005 to about 1.0 g, e.g., about 0.01 to about 1.0 g, about 0.15 to about 0.75 g, about 0.15 to about 0.5 g, about 0.1 to about 0.5 g, about 0.1 to about 0.25 g, or about 0.05 to about 0.2 g per g of cellulolytic or hemicellulolytic enzyme.

The polypeptides having cellulolytic enzyme activity or hemicellulolytic enzyme activity as well as other proteins/polypeptides useful in the degradation of the cellulosic material, *e.g.,* GH61 polypeptides having cellulolytic enhancing activity (collectively hereinafter "polypeptides having enzyme activity") can be derived or obtained from any suitable origin, including, bacterial, fungal, yeast, plant, or mammalian origin. The term "obtained" also means herein that the enzyme may have been produced recombinantly in a host organism employing methods described herein, wherein the recombinantly produced enzyme is either native or foreign to the host organism or has a modified amino acid sequence, *e.g.,* having one or more (*e.g.,* several) amino acids that are deleted, inserted and/or substituted, *i.e.*, a recombinantly produced enzyme that is a mutant and/or a fragment of a native amino acid sequence or an enzyme produced by nucleic acid shuffling processes known in the art. Encompassed within the meaning of a native enzyme are natural variants and within the meaning of a foreign enzyme are variants obtained recombinantly, such as by site-directed mutagenesis or shuffling.

A polypeptide having enzyme activity may be a bacterial polypeptide. For example, the polypeptide may be a Gram-positive bacterial polypeptide such as a *Bacillus, Streptococcus, Streptomyces, Staphylococcus, Enterococcus, Lactobacillus, Lactococcus, Clostridium*, *Geobacillus, Caldicellulosiruptor*, *Acidothermus, Thermobifidia*, or *Oceanobacillus* polypeptide having enzyme activity, or a Gram-negative bacterial polypeptide such as an *E. coli*, *Pseudomonas, Salmonella*, *Campylobacter, Helicobacter, Flavobacterium, Fusobacterium, Ilyobacter*, *Neisseria*, or *Ureaplasma* polypeptide having enzyme activity.

In one aspect, the polypeptide is a *Bacillus alkalophilus*, *Bacillus amyloliquefaciens*, *Bacillus brevis, Bacillus circulans, Bacillus clausii, Bacillus coagulans, Bacillus firmus, Bacillus lautus, Bacillus lentus, Bacillus licheniformis,*

*Bacillus megaterium, Bacillus pumilus, Bacillus stearothermophilus, Bacillus subtilis*, or *Bacillus thuringiensis* polypeptide having enzyme activity.

In another aspect, the polypeptide is a *Streptococcus equisimilis*, *Streptococcus pyogenes*, *Streptococcus uberis*, or *Streptococcus equi* subsp. *Zooepidemicus* polypeptide having enzyme activity.

In another aspect, the polypeptide is a *Streptomyces achromogenes, Streptomyces avermitilis, Streptomyces coelicolor*, *Streptomyces griseus,* or *Streptomyces lividans* polypeptide having enzyme activity.

The polypeptide having enzyme activity may also be a fungal polypeptide, and more preferably a yeast polypeptide such as a *Candida*, *Kluyveromyces, Pichia, Saccharomyces, Schizosaccharomyces,* or *Yarrowia* polypeptide having enzyme activity; or more preferably a filamentous fungal polypeptide such as an *Acremonium, Agaricus, Alternaria*, *Aspergillus, Aureobasidium, Botryospaeria, Ceriporiopsis, Chaetomidium, Chrysosporium, Claviceps*, *Cochliobolus, Coprinopsis*, *Coptotermes*, *Corynascus, Cryphonectria, Cryptococcus, Diplodia, Exidia, Filibasidium, Fusarium, Gibberella, Holomastigotoides, Humicola*, *Irpex*, *Lentinula, Leptospaeria, Magnaporthe, Melanocarpus, Meripilus, Mucor, Myceliophthora, Neocallimastix*, *Neurospora, Paecilomyces*, *Penicillium*, *Phanerochaete, Piromyces*, *Poitrasia*, *Pseudoplectania*, *Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* polypeptide having enzyme activity.

In one aspect, the polypeptide is a *Saccharomyces carlsbergensis, Saccharomyces cerevisiae*, *Saccharomyces diastaticus, Saccharomyces douglasii, Saccharomyces kluyveri*, *Saccharomyces norbensis,* or *Saccharomyces oviformis* polypeptide having enzyme activity.

In another aspect, the polypeptide is an *Acremonium cellulolyticus, Aspergillus aculeatus, Aspergillus awamori, Aspergillus fumigatus, Aspergillus foetidus*, *Aspergillus japonicus, Aspergillus nidulans*, *Aspergillus niger*, *Aspergillus oryzae*, *Chrysosporium keratinophilum*, *Chrysosporium lucknowense, Chrysosporium tropicum*, *Chrysosporium merdarium, Chrysosporium inops*, *Chrysosporium pannicola*, *Chrysosporium queenslandicum, Chrysosporium zonatum*, *Fusarium bactridioides, Fusarium cerealis*, *Fusarium crookwellense, Fusarium culmorum, Fusarium graminearum, Fusarium graminum, Fusarium heterosporum, Fusarium negundi, Fusarium oxysporum, Fusarium reticulatum*, *Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens*, *Humicola lanuginosa, Irpex lacteus, Mucor miehei, Myceliophthora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis*, *Thielavia fimeti*, *Thielavia microspora*, *Thielavia ovispora*, *Thielavia peruviana*, *Thielavia spededonium, Thielavia setosa, Thielavia subthermophila, Thielavia terrestris, Trichoderma harzianum, Trichoderma koningii*, *Trichoderma longibrachiatum, Trichoderma reesei*, *Trichoderma viride,* or *Trichophaea saccata* polypeptide having enzyme activity.

Chemically modified or protein engineered mutants of polypeptides having enzyme activity may also be used.

One or more (e.g., several) components of the enzyme composition may be a recombinant component, *i.e.*, produced by cloning of a DNA sequence encoding the single component and subsequent cell transformed with the DNA sequence and expressed in a host (see, for example, WO 91/17243 and WO 91/17244). The host is preferably a heterologous host (enzyme is foreign to host), but the host may under certain conditions also be a homologous host (enzyme is native to host). Monocomponent cellulolytic proteins may also be prepared by purifying such a protein from a fermentation broth.

In one aspect, the one or more (e.g., several) cellulolytic enzymes comprise a commercial cellulolytic enzyme preparation. Examples of commercial cellulolytic enzyme preparations suitable for use in the present invention include, for example, CELLIC® CTec (Novozymes A/S), CELLIC® CTec2 (Novozymes A/S), CELLUCLAST™ (Novozymes A/S), NOVOZYM™ 188 (Novozymes A/S), CELLUZYME™ (Novozymes A/S), CEREFLO™ (Novozymes A/S), and ULTRAFLO™ (Novozymes A/S), ACCELERASE™ (Genencor Int.), LAMINEX™ (Genencor Int.), SPEZYME™ CP (Genencor Int.), FILTRASE® NL (DSM); METHAPLUS® S/L 100 (DSM), ROHAMENT™ 7069 W (Röhm GmbH), FIBREZYME® LDI (Dyadic International, Inc.), FIBREZYME® LBR (Dyadic International, Inc.), or VISCOSTAR® 150L (Dyadic International, Inc.). The cellulase enzymes are added in amounts effective from about 0.001 to about 5.0 wt % of solids, e.g., about 0.025 to about 4.0 wt % of solids or about 0.005 to about 2.0 wt % of solids.

Examples of bacterial endoglucanases that can be used in the methods of the present invention, include, but are not limited to, an *Acidothermus cellulolyticus* endoglucanase (WO 91/05039; WO 93/15186; U.S. Patent No. 5,275,944; WO 96/02551; U.S. Patent No. 5,536,655, WO 00/70031, WO 05/093050); *Thermobifida fusca* endoglucanase III (WO 05/093050); and *Thermobifida fusca* endoglucanase V (WO 05/093050).

Examples of fungal endoglucanases that can be used in the present invention, include, but are not limited to, a *Trichoderma reesei* endoglucanase I (Penttila et al., 1986, Gene 45: 253-263, *Trichoderma reesei* Cel7B endoglucanase I (GENBANK™ accession no. M15665), *Trichoderma reesei* endoglucanase II (Saloheimo, et al., 1988, Gene 63:11-22), *Trichoderma reesei* Cel5A endoglucanase II (GENBANK™ accession no. M19373), *Trichoderma reesei* endoglucanase III (Okada et al., 1988, Appl. Environ. Microbiol. 64: 555-563, GENBANK™ accession no. AB003694), *Trichoderma reesei* endoglucanase V (Saloheimo et al., 1994, Molecular Microbiology 13: 219-228, GENBANK™ accession no.

Z33381), *Aspergillus aculeatus* endoglucanase (Ooi et al., 1990, Nucleic Acids Research 18: 5884), *Aspergillus kawachii* endoglucanase (Sakamoto et al., 1995, Current Genetics 27: 435-439), *Erwinia carotovara* endoglucanase (Saarilahti et al., 1990, Gene 90: 9-14), *Fusarium oxysporum* endoglucanase (GENBANK™ accession no. L29381), *Humicola grisea* var. *thermoidea* endoglucanase (GENBANK™ accession no. AB003107), *Melanocarpus albomyces* endoglucanase (GENBANK™ accession no. MAL515703), *Neurospora crassa* endoglucanase (GENBANK™ accession no. XM_324477), *Humicola insolens* endoglucanase V, *Myceliophthora thermophila* CBS 117.65 endoglucanase, basidiomycete CBS 495.95 endoglucanase, basidiomycete CBS 494.95 endoglucanase, *Thielavia terrestris* NRRL 8126 CEL6B endoglucanase, *Thielavia terrestris* NRRL 8126 CEL6C endoglucanase, *Thielavia terrestris* NRRL 8126 CEL7C endoglucanase, *Thielavia terrestris* NRRL 8126 CEL7E endoglucanase, *Thielavia terrestris* NRRL 8126 CEL7F endoglucanase, *Cladorrhinum foecundissimum* ATCC 62373 CEL7A endoglucanase, and *Trichoderma reesei* strain No. VTT-D-80133 endoglucanase (GENBANK™ accession no. M15665).

Examples of cellobiohydrolases useful in the present invention include, but are not limited to, *Aspergillus aculeatus* cellobiohydrolase II (WO 2011/059740), *Chaetomium thermophilum* cellobiohydrolase I, *Chaetomium thermophilum* cellobiohydrolase II, *Humicola insolens* cellobiohydrolase I, *Myceliophthora thermophila* cellobiohydrolase II (WO 2009/042871), *Thielavia hyrcanie* cellobiohydrolase II (WO 2010/141325), *Thielavia terrestris* cellobiohydrolase II (CEL6A, WO 2006/074435), *Trichoderma reesei* cellobiohydrolase I, *Trichoderma reesei* cellobiohydrolase II, and *Trichophaea saccata* cellobiohydrolase II (WO 2010/057086).

Examples of beta-glucosidases useful in the present invention include, but are not limited to, beta-glucosidases from *Aspergillus aculeatus* (Kawaguchi et al., 1996, Gene 173: 287-288), *Aspergillus fumigatus* (WO 2005/047499), *Aspergillus niger* (Dan et al., 2000, J. Biol. Chem. 275: 4973-4980), *Aspergillus oryzae* (WO 2002/095014), *Penicillium brasilianum* IBT 20888 (WO 2007/019442 and WO 2010/088387), *Thielavia terrestris* (WO 2011/035029), and *Trichophaea saccata* (WO 2007/019442).

The beta-glucosidase may be a fusion protein. In one aspect, the beta-glucosidase is an *Aspergillus oryzae* beta-glucosidase variant BG fusion protein (WO 2008/057637) or an *Aspergillus oryzae* beta-glucosidase fusion protein (WO 2008/057637).

Other useful endoglucanases, cellobiohydrolases, and beta-glucosidases are disclosed in numerous Glycosyl Hydrolase families using the classification according to Henrissat B., 1991, A classification of glycosyl hydrolases based on amino-acid sequence similarities, Biochem. J. 280: 309-316, and Henrissat B., and Bairoch A., 1996, Updating the sequence-based classification of glycosyl hydrolases, Biochem. J. 316: 695-696.

Other cellulolytic enzymes that may be used in the present invention are described in WO 98/13465, WO 98/015619, WO 98/015633, WO 99/06574, WO 99/10481, WO 99/025847, WO 99/031255, WO 2002/101078, WO 2003/027306, WO 2003/052054, WO 2003/052055, WO 2003/052056, WO 2003/052057, WO 2003/052118, WO 2004/016760, WO 2004/043980, WO 2004/048592, WO 2005/001065, WO 2005/028636, WO 2005/093050, WO 2005/093073, WO 2006/074005, WO 2006/117432, WO 2007/071818, WO 2007/071820, WO 2008/008070, WO 2008/008793, U.S. Patent No. 5,457,046, U.S. Patent No. 5,648,263, and U.S. Patent No. 5,686,593.

In the methods of the present invention, any GH61 polypeptide having cellulolytic enhancing activity can be used.

In a first aspect, the GH61 polypeptide having cellulolytic enhancing activity comprises the following motifs:

[ILMV]-P-X(4,5)-G-X-Y-[ILMV]-X-R-X-[EQ]-X(4)-[HNQ] (SEQ ID NO: 19 or SEQ ID NO: 20) and [FW]-[TF]-K-[AIV],

wherein X is any amino acid, X(4,5) is any amino acid at 4 or 5 contiguous positions, and X(4) is any amino acid at 4 contiguous positions.

The isolated polypeptide comprising the above-noted motifs may further comprise:

H-X(1,2)-G-P-X(3)-[YW]-[AILMV] (SEQ ID NO: 21 or SEQ ID NO: 22),
[EQ]-X-Y-X(2)-C-X-[EHQN]-[FILV]-X-[ILV] (SEQ ID NO: 23), or
H-X(1,2)-G-P-X(3)-[YW]-[AILMV] (SEQ ID NO: 24 or SEQ ID NO: 25) and [EQ]-X-Y-X(2)-C-X-[EHQN]-[FILV]-X-[ILV] (SEQ ID NO: 26),

wherein X is any amino acid, X(1,2) is any amino acid at 1 position or 2 contiguous positions, X(3) is any amino acid at 3 contiguous positions, and X(2) is any amino acid at 2 contiguous positions. In the above motifs, the accepted IUPAC single letter amino acid abbreviation is employed.

In a preferred aspect, the isolated GH61 polypeptide having cellulolytic enhancing activity further comprises H-X(1,2)-G-P-X(3)-[YW]-[AILMV] (SEQ ID NO: 27 or SEQ ID NO: 28). In another preferred aspect, the isolated GH61 polypeptide having cellulolytic enhancing activity further comprises [EQ]-X-Y-X(2)-C-X-[EHQN]-[FILV]-X-[ILV] (SEQ ID NO: 29). In another preferred aspect, the isolated GH61 polypeptide having cellulolytic enhancing activity further comprises H-X(1,2)-G-P-X(3)-[YW]-[AILMV] (SEQ ID NO: 30 or SEQ ID NO: 31) and [EQ]-X-Y-X(2)-C-X-[EHQN]-[FILV]-X-[ILV] (SEQ ID NO: 32).

In a second aspect, isolated polypeptides having cellulolytic enhancing activity, comprise the following motif:

[ILMV]-P-X(4,5)-G-X-Y-[ILMV]-X-R-X-[EQ]-X(3)-A-[HNQ] (SEQ ID NO: 33 or SEQ ID NO: 34),

wherein X is any amino acid, X(4,5) is any amino acid at 4 or 5 contiguous positions, and X(3) is any amino acid at 3 contiguous positions. In the above motif, the accepted IUPAC single letter amino acid abbreviation is employed.

Examples of GH61 polypeptides having cellulolytic enhancing activity useful in the methods of the present invention include, but are not limited to, GH61 polypeptides from *Thielavia terrestris* (WO 2005/074647, WO 2008/148131, and WO 2011/035027), *Thermoascus aurantiacus* (WO 2005/074656 and WO 2010/065830), *Trichoderma reesei* (WO 2007/089290), *Myceliophthora thermophila* (WO 2009/085935, WO 2009/085859, WO 2009/085864, WO 2009/085868), *Aspergillus fumigatus* (WO 2010/138754), GH61 polypeptides from *Penicillium pinophilum* (WO 2011/005867), *Thermoascus* sp. (WO 2011/039319), *Penicillium* sp. (WO 2011/041397), and *Thermoascus crustaceous* (WO 2011/041504).

In one aspect, the GH61 polypeptide having cellulolytic enhancing activity is used in the presence of a soluble activating divalent metal cation according to WO 2008/151043, e.g., manganese sulfate.

In another aspect, the GH61 polypeptide having cellulolytic enhancing activity is used in the presence of a dioxy compound, a bicylic compound, a heterocyclic compound, a nitrogen-containing compound, a quinone compound, a sulfur-containing compound, or a liquor obtained from a pretreated cellulosic material such as pretreated corn stover (PCS).

The dioxy compound may include any suitable compound containing two or more oxygen atoms. In some aspects, the dioxy compounds contain a substituted aryl moiety as described herein. The dioxy compounds may comprise one or more (e.g., several) hydroxyl and/or hydroxyl derivatives, but also include substituted aryl moieties lacking hydroxyl and hydroxyl derivatives. Non-limiting examples of the dioxy compounds include pyrocatechol or catechol; caffeic acid; 3,4-dihydroxybenzoic acid; 4-tert-butyl-5-methoxy-1,2-benzenediol; pyrogallol; gallic acid; methyl-3,4,5-trihydroxybenzoate; 2,3,4-trihydroxybenzophenone; 2,6-dimethoxyphenol; sinapinic acid; 3,5-dihydroxybenzoic acid; 4-chloro-1,2-benzenediol; 4-nitro-1,2-benzenediol; tannic acid; ethyl gallate; methyl glycolate; dihydroxyfumaric acid; 2-butyne-1,4-diol; (croconic acid; 1,3-propanediol; tartaric acid; 2,4-pentanediol; 3-ethyoxy-1,2-propanediol; 2,4,4'-trihydroxybenzophenone; cis-2-butene-1,4-diol; 3,4-dihydroxy-3-cyclobutene-1,2-dione; dihydroxyacetone; acrolein acetal; methyl-4-hydroxybenzoate; 4-hydroxybenzoic acid; and methyl-3,5-dimethoxy-4-hydroxybenzoate; or a salt or solvate thereof.

The bicyclic compound may include any suitable substituted fused ring system as described herein. The compounds may comprise one or more (e.g., several) additional rings, and are not limited to a specific number of rings unless otherwise stated. In one aspect, the bicyclic compound is a flavonoid. In another aspect, the bicyclic compound is an optionally subsituted isoflavonoid. In another aspect, the bicyclic compound is an optionally substituted flavylium ion, such as an optionally substituted anthocyanidin or optionally substituted anthocyanin, or derivative thereof. Non-limiting examples of the bicyclic compounds include epicatechin; quercetin; myricetin; taxifolin; kaempferol; morin; acacetin; naringenin; isorhamnetin; apigenin; cyanidin; cyanin; kuromanin; keracyanin; or a salt or solvate thereof.

The heterocyclic compound may be any suitable compound, such as an optionally substituted aromatic or non-aromatic ring comprising a heteroatom, as described herein. In one aspect, the heterocyclic is a compound comprising an optionally substituted heterocycloalkyl moiety or an optionally substituted heteroaryl moiety. In another aspect, the optionally substituted heterocycloalkyl moiety or optionally substituted heteroaryl moiety is an optionally substituted 5-membered heterocycloalkyl or an optionally substituted 5-membered heteroaryl moiety. In another aspect, the optionally substituted heterocycloalkyl or optionally substituted heteroaryl moiety is an optionally substituted moiety selected from pyrazolyl, furanyl, imidazolyl, isoxazolyl, oxadiazolyl, oxazolyl, pyrrolyl, pyridyl, pyrimidyl, pyridazinyl, thiazolyl, triazolyl, thienyl, dihydrothieno-pyrazolyl, thianaphthenyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, isoindolyl, acridinyl, benzoisazolyl, dimethylhydantoin, pyrazinyl, tetrahydrofuranyl, pyrrolinyl, pyrrolidinyl, morpholinyl, indolyl, diazepinyl, azepinyl, thiepinyl, piperidinyl, and oxepinyl. In another aspect, the optionally substituted heterocycloalkyl moiety or optionally substituted heteroaryl moiety is an optionally substituted furanyl. Non-limiting examples of the heterocyclic compounds include (1,2-dihydroxyethyl)-3,4-dihydroxyfuran-2(5H)-one; 4-hydroxy-5-methyl-3-furanone; 5-hydroxy-2(5H)-furanone; [1,2-dihydroxyethyl]furan-2,3,4(5H)-trione; $\alpha$-hydroxy-$\gamma$-butyrolactone; ribonic $\gamma$-lactone; aldohexuronicaldohexuronic acid $\gamma$-lactone; gluconic acid $\delta$-lactone; 4-hydroxycoumarin; dihydrobenzofuran; 5-(hydroxymethyl)furfural; furoin; 2(5H)-furanone; 5,6-dihydro-2H-pyran-2-one; and 5,6-dihydro-4-hydroxy-6-methyl-2H-pyran-2-one; or a salt or solvate thereof.

The nitrogen-containing compound may be any suitable compound with one or more nitrogen atoms. In one aspect, the nitrogen-containing compound comprises an amine, imine, hydroxylamine, or nitroxide moiety. Non-limiting examples of thenitrogen-containing compounds include acetone oxime; violuric acid; pyridine-2-aldoxime; 2-aminophenol; 1,2-benzenediamine; 2,2,6,6-tetramethyl-1-piperidinyloxy; 5,6,7,8-tetrahydrobiopterin; 6,7-dimethyl-5,6,7,8-tetrahydropterine; and maleamic acid; or a salt or solvate thereof.

The quinone compound may be any suitable compound comprising a quinone moiety as described herein. Non-limiting examples of the quinone compounds include 1,4-benzoquinone; 1,4-naphthoquinone; 2-hydroxy-1,4-naphthoquinone; 2,3-dimethoxy-5-methyl-1,4-benzoquinone or coenzyme $Q_0$; 2,3,5,6-tetramethyl-1,4-benzoquinone or duroquinone; 1,4-dihydroxyanthraquinone; 3-hydroxy-1-methyl-5,6-indolinedione or adrenochrome; 4-tert-butyl-5-methoxy-1,2-benzoquinone; pyrroloquinoline quinone; or a salt or solvate thereof.

The sulfur-containing compound may be any suitable compound comprising one or more sulfur atoms. In one aspect, the sulfur-containing comprises a moiety selected from thionyl, thioether, sulfinyl, sulfonyl, sulfamide, sulfonamide, sulfonic acid, and sulfonic ester. Non-limiting examples of the sulfur-containing compounds include ethanethiol; 2-propanethiol; 2-propene-1-thiol; 2-mercaptoethanesulfonic acid; benzenethiol; benzene-1,2-dithiol; cysteine; methionine; glutathione; cystine; or a salt or solvate thereof.

In one aspect, an effective amount of such a compound described above to cellulosic material as a molar ratio to glucosyl units of cellulose is about $10^{-6}$ to about 10, e.g., about $10^{-6}$ to about 7.5, about $10^{-6}$ to about 5, about $10^{-6}$ to about 2.5, about $10^{-6}$ to about 1, about $10^{-5}$ to about 1, about $10^{-5}$ to about $10^{-1}$, about $10^{-4}$ to about $10^{-1}$, about $10^{-3}$ to about $10^{-1}$, or about $10^{-3}$ to about $10^{-2}$. In another aspect, an effective amount of such a compound described above is about 0.1 μM to about 1 M, e.g., about 0.5 μM to about 0.75 M, about 0.75 μM to about 0.5 M, about 1 μM to about 0.25 M, about 1 μM to about 0.1 M, about 5 μM to about 50 mM, about 10 μM to about 25 mM, about 50 μM to about 25 mM, about 10 μM to about 10 mM, about 5 μM to about 5 mM, or about 0.1 mM to about 1 mM.

The term "liquor" means the solution phase, either aqueous, organic, or a combination thereof, arising from treatment of a lignocellulose and/or hemicellulose material in a slurry, or monosaccharides thereof, *e.g.,* xylose, arabinose, mannose, *etc.,* under conditions as described herein, and the soluble contents thereof. A liquor for cellulolytic enhancement of a GH61 polypeptide can be produced by treating a lignocellulose or hemicellulose material (or feedstock) by applying heat and/or pressure, optionally in the presence of a catalyst, *e.g.*, acid, optionally in the presence of an organic solvent, and optionally in combination with physical disruption of the material, and then separating the solution from the residual solids. Such conditions determine the degree of cellulolytic enhancement obtainable through the combination of liquor and a GH61 polypeptide during hydrolysis of a cellulosic substrate by a cellulase preparation. The liquor can be separated from the treated material using a method standard in the art, such as filtration, sedimentation, or centrifugation.

In one aspect, an effective amount of the liquor to cellulose is about $10^{-6}$ to about 10 g per g of cellulose, *e.g.*, about $10^{-6}$ to about 7.5 g, about $10^{-6}$ to about 5, about $10^{-6}$ to about 2.5 g, about $10^{-6}$ to about 1 g, about $10^{-5}$ to about 1 g, about $10^{-5}$ to about $10^{-1}$ g, about $10^{-4}$ to about $10^{-1}$ g, about $10^{-3}$ to about $10^{-1}$ g, or about $10^{-3}$ to about $10^{-2}$ g per g of cellulose.

In one aspect, the one or more (e.g., several) hemicellulolytic enzymes comprise a commercial hemicellulolytic enzyme preparation. Examples of commercial hemicellulolytic enzyme preparations suitable for use in the present invention include, for example, SHEARZYME™ (Novozymes A/S), CELLIC® HTec (Novozymes A/S), CELLIC® HTec2 (Novozymes A/S), VISCOZYME® (Novozymes A/S), ULTRAFLO® (Novozymes A/S), PULPZYME® HC (Novozymes A/S), MULTIFECT® Xylanase (Genencor), ACCELLERASE® XY (Genencor), ACCELLERASE® XC (Genencor), ECOPULP® TX-200A (AB Enzymes), HSP 6000 Xylanase (DSM), DEPOL™ 333P (Biocatalysts Limit, Wales, UK), DEPOL™ 740L. (Biocatalysts Limit, Wales, UK), and DEPOL™ 762P (Biocatalysts Limit, Wales, UK).

Examples of xylanases useful in the methods of the present invention include, but are not limited to, xylanases from *Aspergillus aculeatus* (GeneSeqP:AAR63790; WO 94/21785), *Aspergillus fumigatus* (WO 2006/078256), *Penicillium pinophilum* (WO 2011/041405), *Penicillium* sp. (WO 2010/126772), *Thielavia terrestris* NRRL 8126 (WO 2009/079210), and *Trichophaea saccata* GH10 (WO 2011/057083).

Examples of beta-xylosidases useful in the methods of the present invention include, but are not limited to, beta-xylosidases from *Neurospora crassa* (SwissProt accession number Q7SOW4), *Trichoderma reesei* (UniProtKB/TrEMBL accession number Q92458), and *Talaromyces emersonii* (SwissProt accession number Q8X212).

Examples of acetylxylan esterases useful in the methods of the present invention include, but are not limited to, acetylxylan esterases from *Aspergillus aculeatus* (WO 2010/108918), *Chaetomium globosum* (Uniprot accession number Q2GWX4), *Chaetomium gracile* (GeneSeqP accession number AAB82124), *Humicola insolens* DSM 1800 (WO 2009/073709), *Hypocrea jecorina* (WO 2005/001036), *Myceliophtera thermophila* (WO 2010/014880), *Neurospora crassa* (UniProt accession number q7s259), *Phaeosphaeria nodorum* (Uniprot accession number Q0UHJ1), and *Thielavia terrestris* NRRL 8126 (WO 2009/042846).

Examples of feruloyl esterases (ferulic acid esterases) useful in the methods of the present invention include, but are not limited to, feruloyl esterases form *Humicola insolens* DSM 1800 (WO 2009/076122), *Neosartorya fischeri* (UniProt Accession number A1D9T4), *Neurospora crassa* (UniProt accession number Q9HGR3), *Penicillium aurantiogriseum* (WO 2009/127729), and *Thielavia terrestris* (WO 2010/053838 and WO 2010/065448).

Examples of arabinofuranosidases useful in the methods of the present invention include, but are not limited to, arabinofuranosidases from *Aspergillus niger* (GeneSeqP accession number AAR94170), *Humicola insolens* DSM 1800 (WO 2006/114094 and WO 2009/073383), and *M. giganteus* (WO 2006/114094).

Examples of alpha-glucuronidases useful in the methods of the present invention include, but are not limited to, alpha-glucuronidases from *Aspergillus clavatus* (UniProt accession number alcc12), *Aspergillus fumigatus* (SwissProt accession number Q4WW45), *Aspergillus niger* (Uniprot accession number Q96WX9), *Aspergillus terreus* (SwissProt accession number Q0CJP9), *Humicola insolens* (WO 2010/014706), *Penicillium aurantiogriseum* (WO 2009/068565), *Talaromyces emersonii* (UniProt accession number Q8X211), and *Trichoderma reesei* (Uniprot accession number Q99024).

The polypeptides having enzyme activity used in the methods of the present invention may be produced by fermentation of the above-noted microbial strains on a nutrient medium containing suitable carbon and nitrogen sources and inorganic salts, using procedures known in the art (see, *e.g.*, Bennett, J.W. and LaSure, L. (eds.), More Gene Manipulations in Fungi, Academic Press, CA, 1991). Suitable media are available from commercial suppliers or may be prepared according to published compositions (*e.g.*, in catalogues of the American Type Culture Collection). Temperature ranges and other conditions suitable for growth and enzyme production are known in the art (see, *e.g.*, Bailey, J.E., and Ollis, D.F., Biochemical Engineering Fundamentals, McGraw-Hill Book Company, NY, 1986).

The fermentation can be any method of cultivation of a cell resulting in the expression or isolation of an enzyme or protein. Fermentation may, therefore, be understood as comprising shake flask cultivation, or small- or large-scale fermentation (including continuous, batch, fed-batch, or solid state fermentations) in laboratory or industrial fermentors performed in a suitable medium and under conditions allowing the enzyme to be expressed or isolated. The resulting enzymes produced by the methods described above may be recovered from the fermentation medium and purified by conventional procedures.

Fermentation. The fermentable sugars obtained from the hydrolyzed cellulosic material can be fermented by one or more (e.g., several) fermenting microorganisms capable of fermenting the sugars directly or indirectly into a desired fermentation product. "Fermentation" or "fermentation process" refers to any fermentation process or any process comprising a fermentation step. Fermentation processes also include fermentation processes used in the consumable alcohol industry (*e.g.*, beer and wine), dairy industry (*e.g.*, fermented dairy products), leather industry, and tobacco industry. The fermentation conditions depend on the desired fermentation product and fermenting organism and can easily be determined by one skilled in the art.

In the fermentation step, sugars, released from the cellulosic material as a result of the pretreatment and enzymatic hydrolysis steps, are fermented to a product, *e.g.*, ethanol, by a fermenting organism, such as yeast. Hydrolysis (saccharification) and fermentation can be separate or simultaneous, as described herein.

Any suitable hydrolyzed cellulosic material can be used in the fermentation step in practicing the present invention. The material is generally selected based on the desired fermentation product, *i.e.*, the substance to be obtained from the fermentation, and the process employed, as is well known in the art.

The term "fermentation medium" is understood herein to refer to a medium before the fermenting microorganism(s) is(are) added, such as, a medium resulting from a saccharification process, as well as a medium used in a simultaneous saccharification and fermentation process (SSF).

"Fermenting microorganism" refers to any microorganism, including bacterial and fungal organisms, suitable for use in a desired fermentation process to produce a fermentation product. The fermenting organism can be hexose and/or pentose fermenting organisms, or a combination thereof. Both hexose and pentose fermenting organisms are well known in the art. Suitable fermenting microorganisms are able to ferment, *i.e.*, convert, sugars, such as glucose, xylose, xylulose, arabinose, maltose, mannose, galactose, and/or oligosaccharides, directly or indirectly into the desired fermentation product. Examples of bacterial and fungal fermenting organisms producing ethanol are described by Lin et al., 2006, Appl. Microbiol. Biotechnol. 69: 627-642.

Examples of fermenting microorganisms that can ferment hexose sugars include bacterial and fungal organisms, such as yeast. Preferred yeast includes strains of *Candida*, *Kluyveromyces,* and *Saccharomyces, e.g., Candida sonorensis*, *Kluyveromyces marxianus*, and *Saccharomyces cerevisiae.*

Examples of fermenting organisms that can ferment pentose sugars in their native state include bacterial and fungal organisms, such as some yeast. Preferred xylose fermenting yeast include strains of *Candida,* preferably *C. sheatae* or *C. sonorensis*; and strains of *Pichia,* preferably *P. stipitis,* such as *P. stipitis* CBS 5773. Preferred pentose fermenting yeast include strains of *Pachysolen,* preferably *P. tannophilus.* Organisms not capable of fermenting pentose sugars, such as xylose and arabinose, may be genetically modified to do so by methods known in the art.

Examples of bacteria that can efficiently ferment hexose and pentose to ethanol include, for example, *Bacillus coagulans, Clostridium acetobutylicum, Clostridium thermocellum*, *Clostridium phytofermentans*, *Geobacillus* sp., *Thermoanaerobacter saccharolyticum,* and *Zymomonas mobilis* (Philippidis, 1996, *supra*).

Other fermenting organisms include strains of *Bacillus,* such as *Bacillus coagulans*; *Candida*, such as *C. sonorensis*, *C. methanosorbosa, C. diddensiae*, *C. parapsilosis*, *C. naedodendra*, *C. blankii, C. entomophilia, C. brassicae, C. pseudotropicalis*, *C. boidinii, C. utilis*, and *C. scehatae; Clostridium*, such as *C. acetobutylicum, C. thermocellum*, and *C. phytofermentans; E. coli*, especially *E. coli* strains that have been genetically modified to improve the yield of ethanol; *Geobacillus* sp.; *Hansenula,* such as *Hansenula anomala*; *Klebsiella,* such as *K. oxytoca; Kluyveromyces,* such as *K. marxianus, K. lactis*, *K. thermotolerans*, and *K. fragilis*; *Schizosaccharomyces,* such as *S. pombe*; *Thermoanaerobacter,* such as *Thermoanaerobacter saccharolyticum*; and *Zymomonas,* such as *Zymomonas mobilis.*

In a preferred aspect, the yeast is a *Bretannomyces.* In a more preferred aspect, the yeast is *Bretannomyces clausenii.* In another preferred aspect, the yeast is a *Candida.* In another more preferred aspect, the yeast is *Candida sonorensis.* In another more preferred aspect, the yeast is *Candida boidinii.* In another more preferred aspect, the yeast is *Candida blankii.* In another more preferred aspect, the yeast is *Candida brassicae.* In another more preferred aspect, the yeast is *Candida diddensii.* In another more preferred aspect, the yeast is *Candida entomophiliia.* In another more preferred aspect, the yeast is *Candida pseudotropicalis.* In another more preferred aspect, the yeast is *Candida scehatae.* In another more preferred aspect, the yeast is *Candida utilis*. In another preferred aspect, the yeast is a *Clavispora*. In another more preferred aspect, the yeast is *Clavispora lusitaniae*. In another more preferred aspect, the yeast is *Clavispora opuntiae*. In another preferred aspect, the yeast is a *Kluyveromyces.* In another more preferred aspect, the yeast is *Kluyveromyces fragilis*. In another more preferred aspect, the yeast is *Kluyveromyces marxianus.* In another more preferred aspect, the yeast is *Kluyveromyces thermotolerans*. In another preferred aspect, the yeast is a *Pachysolen.* In another more preferred aspect, the yeast is *Pachysolen tannophilus.* In another preferred aspect, the yeast is a *Pichia.* In another more preferred aspect, the yeast is a *Pichia stipitis*. In another preferred aspect, the yeast is a *Saccharomyces* spp. In another more preferred aspect, the yeast is *Saccharomyces cerevisiae*. In another more preferred aspect, the yeast is *Saccharomyces distaticus.* In another more preferred aspect, the yeast is *Saccharomyces uvarum*.

In a preferred aspect, the bacterium is a *Bacillus.* In a more preferred aspect, the bacterium is *Bacillus coagulans.* In another preferred aspect, the bacterium is a *Clostridium.* In another more preferred aspect, the bacterium is *Clostridium acetobutylicum.* In another more preferred aspect, the bacterium is *Clostridium phytofermentans.* In another more preferred aspect, the bacterium is *Clostridium thermocellum.* In another more preferred aspect, the bacterium is *Geobacilus* sp. In another more preferred aspect, the bacterium is a *Thermoanaerobacter.* In another more preferred aspect, the bacterium is *Thermoanaerobacter saccharolyticum.* In another preferred aspect, the bacterium is a *Zymomonas.* In another more preferred aspect, the bacterium is *Zymomonas mobilis.*

Commercially available yeast suitable for ethanol production include, *e.g.*, BIOFERM™ AFT and XR (NABC - North American Bioproducts Corporation, GA, USA), ETHANOL RED™ yeast (Fermentis/Lesaffre, USA), FALI™ (Fleischmann's Yeast, USA), FERMIOL™ (DSM Specialties), GERT STRAND™ (Gert Strand AB, Sweden), and SUPERSTART™ and THERMOSACC™ fresh yeast (Ethanol Technology, WI, USA).

In a preferred aspect, the fermenting microorganism has been genetically modified to provide the ability to ferment pentose sugars, such as xylose utilizing, arabinose utilizing, and xylose and arabinose co-utilizing microorganisms.

The cloning of heterologous genes into various fermenting microorganisms has led to the construction of organisms capable of converting hexoses and pentoses to ethanol (co-fermentation) (Chen and Ho, 1993, Cloning and improving the expression of Pichia stipitis xylose reductase gene in Saccharomyces cerevisiae, Appl. Biochem. Biotechnol. 39-40: 135-147; Ho et al., 1998, Genetically engineered Saccharomyces yeast capable of effectively cofermenting glucose and xylose, Appl. Environ. Microbiol. 64: 1852-1859; Kotter and Ciriacy, 1993, Xylose fermentation by Saccharomyces cerevisiae, Appl. Microbiol. Biotechnol. 38: 776-783; Walfridsson et al., 1995, Xylose-metabolizing Saccharomyces cerevisiae strains overexpressing the TKL1 and TAL1 genes encoding the pentose phosphate pathway enzymes transketolase and transaldolase, Appl. Environ. Microbiol. 61: 4184-4190; Kuyper et al., 2004, Minimal metabolic engineering of Saccharomyces cerevisiae for efficient anaerobic xylose fermentation: a proof of principle, FEMS Yeast Research 4: 655-664; Beall et al., 1991, Parametric studies of ethanol production from xylose and other sugars by recombinant Escherichia coli, Biotech. Bioeng. 38: 296-303; Ingram et al., 1998, Metabolic engineering of bacteria for ethanol production, Biotechnol. Bioeng. 58: 204-214; Zhang et al., 1995, Metabolic engineering of a pentose metabolism pathway in ethanologenic Zymomonas mobilis, Science 267: 240-243; Deanda et al., 1996, Development of an arabinose-fermenting Zymomonas mobilis strain by metabolic pathway engineering, Appl. Environ. Microbiol. 62: 4465-4470; WO 2003/062430, xylose isomerase).

In a preferred aspect, the genetically modified fermenting microorganism is *Candida sonorensis.* In another preferred aspect, the genetically modified fermenting microorganism is *Escherichia coli.* In another preferred aspect, the genetically modified fermenting microorganism is *Klebsiella oxytoca.* In another preferred aspect, the genetically modified fermenting microorganism is *Kluyveromyces marxianus.* In another preferred aspect, the genetically modified fermenting microorganism is *Saccharomyces cerevisiae.* In another preferred aspect, the genetically modified fermenting microorganism is *Zymomonas mobilis.*

It is well known in the art that the organisms described above can also be used to produce other substances, as described herein.

The fermenting microorganism is typically added to the degraded cellulosic material or hydrolysate and the fermentation is performed for about 8 to about 96 hours, e.g., about 24 to about 60 hours. The temperature is typically between about 26°C to about 60°C, e.g., about 32°C or 50°C, and about pH 3 to about pH 8, e.g., pH 4-5, 6, or 7.

In one aspect, the yeast and/or another microorganism are applied to the degraded cellulosic material and the fermentation is performed for about 12 to about 96 hours, such as typically 24-60 hours. In another aspect, the temperature is preferably between about 20°C to about 60°C, e.g., about 25°C to about 50°C, about 32°C to about 50°C, or about 32°C to about 50°C, and the pH is generally from about pH 3 to about pH 7, e.g., about pH 4 to about pH 7. However, some fermenting organisms, e.g., bacteria, have higher fermentation temperature optima. Yeast or another microorganism is preferably applied in amounts of approximately $10^5$ to $10^{12}$, preferably from approximately $10^7$ to $10^{10}$, especially approximately 2 x $10^8$ viable cell count per ml of fermentation broth. Further guidance in respect of using yeast for fermentation can be found in, e.g., "The Alcohol Textbook" (Editors K. Jacques, T.P. Lyons and D.R. Kelsall, Nottingham University Press, United Kingdom 1999).

A fermentation stimulator can be used in combination with any of the processes described herein to further improve the fermentation process, and in particular, the performance of the fermenting microorganism, such as, rate enhancement and ethanol yield. A "fermentation stimulator" refers to stimulators for growth of the fermenting microorganisms, in particular, yeast. Preferred fermentation stimulators for growth include vitamins and minerals. Examples of vitamins include multivitamins, biotin, pantothenate, nicotinic acid, meso-inositol, thiamine, pyridoxine, para-aminobenzoic acid, folic acid, riboflavin, and Vitamins A, B, C, D, and E. See, for example, Alfenore et al., Improving ethanol production and viability of Saccharomyces cerevisiae by a vitamin feeding strategy during fed-batch process, Springer-Verlag (2002). Examples of minerals include minerals and mineral salts that can supply nutrients comprising P, K, Mg, S, Ca, Fe, Zn, Mn, and Cu.

Fermentation products: A fermentation product can be any substance derived from the fermentation. The fermentation product can be, without limitation, an alcohol (*e.g.*, arabinitol, n-butanol, isobutanol, ethanol, glycerol, methanol, ethylene glycol, 1,3-propanediol [propylene glycol], butanediol, glycerin, sorbitol, and xylitol); an alkane (*e.g.*, pentane, hexane, heptane, octane, nonane, decane, undecane, and dodecane), a cycloalkane (*e.g.*, cyclopentane, cyclohexane, cycloheptane, and cyclooctane), an alkene (*e.g.* pentene, hexene, heptene, and octene); an amino acid (*e.g.*, aspartic acid, glutamic acid, glycine, lysine, serine, and threonine); a gas (*e.g.*, methane, hydrogen ($H_2$), carbon dioxide ($CO_2$), and carbon monoxide (CO)); isoprene; a ketone (*e.g.*, acetone); an organic acid (*e.g.*, acetic acid, acetonic acid, adipic acid, ascorbic acid, citric acid, 2,5-diketo-D-gluconic acid, formic acid, fumaric acid, glucaric acid, gluconic acid, glucuronic acid, glutaric acid, 3-hydroxypropionic acid, itaconic acid, lactic acid, malic acid, malonic acid, oxalic acid, oxaloacetic acid, propionic acid, succinic acid, and xylonic acid); and polyketide. The fermentation product can also be protein as a high value product.

In a preferred aspect, the fermentation product is an alcohol. It will be understood that the term "alcohol" encompasses a substance that contains one or more hydroxyl moieties. In a more preferred aspect, the alcohol is n-butanol. In another more preferred aspect, the alcohol is isobutanol. In another more preferred aspect, the alcohol is ethanol. In another more preferred aspect, the alcohol is methanol. In another more preferred aspect, the alcohol is arabinitol. In another more preferred aspect, the alcohol is butanediol. In another more preferred aspect, the alcohol is ethylene glycol. In another more preferred aspect, the alcohol is glycerin. In another more preferred aspect, the alcohol is glycerol. In another more preferred aspect, the alcohol is 1,3-propanediol. In another more preferred aspect, the alcohol is sorbitol. In another more preferred aspect, the alcohol is xylitol. See, for example, Gong, C. S., Cao, N. J., Du, J., and Tsao, G. T., 1999, Ethanol production from renewable resources, in Advances in Biochemical Engineering/Biotechnology, Scheper, T., ed., Springer-Verlag Berlin Heidelberg, Germany, 65: 207-241; Silveira, M. M., and Jonas, R., 2002, The biotechnological production of sorbitol, Appl. Microbiol. Biotechnol. 59: 400-408; Nigam, P., and Singh, D., 1995, Processes for fermentative production of xylitol - a sugar substitute, Process Biochemistry 30 (2): 117-124; Ezeji, T. C., Qureshi, N. and Blaschek, H. P., 2003, Production of acetone, butanol and ethanol by Clostridium beijerinckii BA101 and in situ recovery by gas stripping, World Journal of Microbiology and Biotechnology 19 (6): 595-603.

In another preferred aspect, the fermentation product is an alkane. The alkane can be an unbranched or a branched alkane. In another more preferred aspect, the alkane is pentane. In another more preferred aspect, the alkane is hexane. In another more preferred aspect, the alkane is heptane. In another more preferred aspect, the alkane is octane. In another more preferred aspect, the alkane is nonane. In another more preferred aspect, the alkane is decane. In another more preferred aspect, the alkane is undecane. In another more preferred aspect, the alkane is dodecane.

In another preferred aspect, the fermentation product is a cycloalkane. In another more preferred aspect, the cycloalkane is cyclopentane. In another more preferred aspect, the cycloalkane is cyclohexane. In another more preferred aspect, the cycloalkane is cycloheptane. In another more preferred aspect, the cycloalkane is cyclooctane.

In another preferred aspect, the fermentation product is an alkene. The alkene can be an unbranched or a branched alkene. In another more preferred aspect, the alkene is pentene. In another more preferred aspect, the alkene is hexene. In another more preferred aspect, the alkene is heptene. In another more preferred aspect, the alkene is octene.

In another preferred aspect, the fermentation product is an amino acid. In another more preferred aspect, the organic acid is aspartic acid. In another more preferred aspect, the amino acid is glutamic acid. In another more preferred aspect, the amino acid is glycine. In another more preferred aspect, the amino acid is lysine. In another more preferred aspect, the amino acid is serine. In another more preferred aspect, the amino acid is threonine. See, for example, Richard, A., and Margaritis, A., 2004, Empirical modeling of batch fermentation kinetics for poly(glutamic acid) production and other microbial biopolymers, Biotechnology and Bioengineering 87 (4): 501-515.

In another preferred aspect, the fermentation product is a gas. In another more preferred aspect, the gas is methane. In another more preferred aspect, the gas is $H_2$. In another more preferred aspect, the gas is $CO_2$. In another more preferred aspect, the gas is CO. See, for example, Kataoka, N., A. Miya, and K. Kiriyama, 1997, Studies on hydrogen production by continuous culture system of hydrogen-producing anaerobic bacteria, Water Science and Technology 36 (6-7): 41-47; and Gunaseelan V.N. in Biomass and Bioenergy, Vol. 13 (1-2), pp. 83-114, 1997, Anaerobic digestion of biomass for methane production: A review.

In another preferred aspect, the fermentation product is isoprene.

In another preferred aspect, the fermentation product is a ketone. It will be understood that the term "ketone" encompasses a substance that contains one or more ketone moieties. In another more preferred aspect, the ketone is acetone. See, for example, Qureshi and Blaschek, 2003, *supra.*

In another preferred aspect, the fermentation product is an organic acid. In another more preferred aspect, the organic acid is acetic acid. In another more preferred aspect, the organic acid is acetonic acid. In another more preferred aspect, the organic acid is adipic acid. In another more preferred aspect, the organic acid is ascorbic acid. In another more preferred aspect, the organic acid is citric acid. In another more preferred aspect, the organic acid is 2,5-diketo-D-gluconic acid. In another more preferred aspect, the organic acid is formic acid. In another more preferred aspect, the organic acid is fumaric acid. In another more preferred aspect, the organic acid is glucaric acid. In another more preferred aspect, the organic acid is gluconic acid. In another more preferred aspect, the organic acid is glucuronic acid. In another more preferred aspect, the organic acid is glutaric acid. In another preferred aspect, the organic acid is 3-hydroxypropionic acid. In another more preferred aspect, the organic acid is itaconic acid. In another more preferred aspect, the organic acid is lactic acid. In another more preferred aspect, the organic acid is malic acid. In another more preferred aspect, the organic acid is malonic acid. In another more preferred aspect, the organic acid is oxalic acid. In another more preferred aspect, the organic acid is propionic acid. In another more preferred aspect, the organic acid is succinic acid. In another more preferred aspect, the organic acid is xylonic acid. See, for example, Chen, R., and Lee, Y. Y., 1997, Membrane-mediated extractive fermentation for lactic acid production from cellulosic biomass, Appl. Biochem. Biotechnol. 63-65: 435-448.

In another preferred aspect, the fermentation product is polyketide.

Recovery. The fermentation product(s) can be optionally recovered from the fermentation medium using any method known in the art including, but not limited to, chromatography, electrophoretic procedures, differential solubility, distillation, or extraction. For example, alcohol is separated from the fermented cellulosic material and purified by conventional methods of distillation. Ethanol with a purity of up to about 96 vol.% can be obtained, which can be used as, for example, fuel ethanol, drinking ethanol, *i.e.*, potable neutral spirits, or industrial ethanol.

**Signal Peptide**

The present disclosure also relates to an isolated polynucleotide encoding a signal peptide comprising or consisting of amino acids 1 to 23 of SEQ ID NO: 2. In one aspect, the polynucleotide is nucleotides 1 to 69 of SEQ ID NO: 1. The polynucleotides may further comprise a gene encoding a protein, which is operably linked to the signal peptide.

The present disclosure also relates to nucleic acid constructs, expression vectors and recombinant host cells comprising such polynucleotides.

The present disclosure also relates to methods of producing a protein, comprising: (a) cultivating a recombinant host cell comprising such a polynucleotide operably linked to a gene encoding the protein; and (b) recovering the protein.

The protein may be native or heterologous to a host cell. The term "protein" is not meant herein to refer to a specific length of the encoded product and, therefore, encompasses peptides, oligopeptides, and polypeptides. The term "protein" also encompasses two or more polypeptides combined to form the encoded product. The proteins also include hybrid polypeptides and fused polypeptides.

Preferably, the protein is a hormone, enzyme, receptor or portion thereof, antibody or portion thereof, or reporter. For example, the protein may be a hydrolase, isomerase, ligase, lyase, oxidoreductase, or transferase, e.g., an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

The gene may be obtained from any prokaryotic, eukaryotic, or other source.

The present disclosure is further described by the following examples.

## Examples

### Strain

*Penicillium pinophilum* NN046877 was isolated from a soil from Hunan, China by directly plating the soil sample onto a PDA plate followed by incubation at 37°C for 5 days. The strain was then purified by transferring the mycelia onto a YG agar plate. The strain NN046877 was identified as *Penicillium pinophilum* based on both morphological and molecular (ITS sequencing) characterization.

### Media

PDA plates were composed of 39 grams of potato dextrose agar and deionized water to 1 liter.

YG agar plates were composed of 5.0 g of yeast extract, 10.0 g of glucose, 20.0 g of agar, and deionized water to 1 liter.

NNCYP-PCS medium was composed of 5.0 g of $NaNO_3$, 3.0 g of $NH_4Cl$, 2.0 g of MES, 2.5 g of citric acid, 0.2 g of $CaCl_2$ $2H_2O$, 1.0 g of Bacto Peptone, 5.0 g of yeast extract, 0.2 g of $MgSO_4$ $7H_2O$, 4.0 g of $K_2HPO_4$, 1.0 ml of COVE trace elements solution, 2.5 g of glucose, 25.0 g of pretreated corn stover (PCS), and deionized water to 1 liter.

COVE trace elements solution was composed of 0.04 g of $Na_2B_4O_7{\cdot}10H_2O$, 0.4 g of $CuSO_4{\cdot}5H_2O$, 1.2 g of $FeSO_4{\cdot}7H_2O$, 0.7 g of $MnSO_4{\cdot}H_2O$, 0.8 g of $Na_2MoO_2{\cdot}2H_2O$, 10 g of $ZnSO_4{\cdot}7H_2O$, and deionized water to 1 liter.

LB plates were composed of 10 g of tryptone, 5 g of yeast extract, 10 g of sodium chloride, 15 g of agar, and deionized water to 1 liter.

SOC medium was composed of 2% tryptone, 0.5% yeast extract, 10 mM NaCl, 2.5 mM KCl, 10 mM $MgCl_2$, and 10 mM $MgSO_4$ in deionized water; sterilized by autoclaving and then filter-sterilized glucose was added to 20 mM.

Minimal medium plates were composed of 6 g of $NaNO_3$, 0.52 g of KCl, 1.52 g of $KH_2PO_4$, 1 ml of COVE trace elements solution, 20 g of Noble agar, 20 ml of 50% glucose, 2.5 ml of $MgSO_4{\cdot}7H_2O$, 20 ml of a 0.02% biotin solution, and deionized water to 1 liter.

YPM medium was composed of 1% yeast extract, 2% of peptone, and 2% of maltose in deionized water.

### Example 1: Preparation of *Penicillium pinophilum* strain NN046877 mycelia for cDNA library production

*Penicillium pinophilum* strain NN046877 was inoculated onto a PDA plate and incubated for 4 days at 37°C in the darkness. Several mycelia-PDA plugs were inoculated into 500 ml shake flasks containing 100 ml of NNCYP-PCS medium. The flasks were incubated for 5 days at 37°C with shaking at 160 rpm. The mycelia were collected at 4 and 5 days. Then the mycelia from each day were frozen in liquid nitrogen and stored in a -80°C freezer until use.

### Example 2: *Penicillium pinophilum* strain NN046877 RNA isolation

The frozen *P. pinophilum* mycelia were transferred into a liquid nitrogen prechilled mortar and pestle and ground to a fine powder. Total RNA was prepared from the powdered mycelia of each day by extraction with TRIZOL® reagent (Invitrogen, Carlsbad, CA, USA). The polyA enriched RNA was isolated by using a mTRAP™ Total Kit (Active Motif, Carlsbad, CA, USA).

### Example 3: Construction of a *Penicillium pinophilum* strain NN046877 cDNA library

Double stranded cDNA from day 4 and day 5 (Example 2) was synthesized with a SMART™ cDNA Library Construct Kit (Takara Biotechnology Co., Ltd., Dalian, China). The cDNA was cleaved with *Sfi* I (New England Biolabs Ltd., Beijing, China) according to the manufacturer's instructions and the cDNA was size fractionated by 0.8% agarose gel electrophoresis using 44 mM Tris base, 44 mM boric acid, 0.5 mM EDTA buffer. The fraction of cDNA of 500 bp and larger was excised from the gel and purified using a GFX® PCR DNA and Gel Band Purification Kit according to the manufacturer's instructions (GE Healthcare, Buckinghamshire, UK). Then equal amounts of cDNA from day 4 and day 5 were pooled for library construction.

The prepared cDNA was then directionally cloned by ligation into Sfi I cleaved pMHas7 (WO 2009/037253) using T4 ligase (New England Biolabs Ltd., Beijing, China) according to the manufacturer's instructions. The ligation mixture was electroporated into *E. coli* ELECTROMAX™ DH10B™ cells (Invitrogen, Carlsbad, CA, USA) using a GENE PULSER® and Pulse Controller (Bio-Rad Laboratories, Inc., Hercules, CA, USA) at 25 μF, 25 mAmp, 1.8 kV with a 1 mm gap cuvette according to the manufacturer's procedure.

The electroporated cells were plated onto LB plates supplemented with 50 mg of kanamycin per liter. A cDNA plasmid pool was prepared from 60,000 total transformants of the original pMHas7 vector ligation. Plasmid DNA was prepared directly from the pool of colonies using a QIAGEN® Plasmid Midi Kit (QIAGEN Inc., Valencia, CA, USA).

**Example 4: Construction of a SigA4 transposon containing the β-lactamase reporter gene**

A transposon containing plasmid designated pSigA4 was constructed from the pSigA2 transposon containing plasmid described in WO 01/77315 in order to create an improved version of the signal trapping transposon of pSigA2 with decreased selection background. The pSigA2 transposon contains a signal-less beta-lactamase construct encoded on the transposon itself. PCR was used to create a deletion of the intact beta-lactamase gene found on the plasmid backbone using a proofreading PROOFSTART™ DNA polymerase (QIAGEN GmbH Corporation, Hilden, Germany) and the following 5' phosphorylated primers (TAG Copenhagen, Denmark):

SigA2NotU-P:

5'-TCGCGATCCGTTTTCGCATTTATCGTGAAACGCT-3' (SEQ ID NO: 3)

SigA2NotD-P:

5'-CCGCAAACGCTGGTGAAAGTAAAAGATGCTGAA-3' (SEQ ID NO: 4)

The amplification reaction was composed of 1 μl of pSigA2 (10 ng/ μl), 5 μl of 10X PROOFSTART™ Buffer (QIAGEN GmbH Corporation, Hilden, Germany), 2.5 μl of dNTP mix (20 mM), 0.5 μl of SigA2NotU-P (10 mM), 0.5 μl of SigA2NotD-P (10 mM), 10 μl of Q solution (QIAGEN GmbH Corporation, Hilden, Germany), and 31.25 μl of deionized water. A DNA ENGINE™ Thermal Cycler (MJ Research Inc., Waltham, MA, USA) was used for amplification programmed for one cycle at 95°C for 5 minutes; and 20 cycles each at 94°C for 30 seconds, 62°C for 30 seconds, and 72°C for 4 minutes.

A 3.9 kb PCR reaction product was isolated on a 0.8% agarose gel using 40 mM Tris base-20 mM sodium acetate-1 mM disodium EDTA (TAE) buffer and 0.1 μg of ethidium bromide per ml. The DNA band was visualized with the aid of an EAGLE EYE® Imaging System (Stratagene, La Jolla, CA, USA) at 360 nm. The 3.9 kb DNA band was excised from the gel and purified by using a GFX® PCR DNA and Gel Band Purification Kit according to the manufacturer's instructions.

The 3.9 kb fragment was self-ligated at 16°C overnight with 10 units of T4 DNA ligase (New England Biolabs, Inc., Beverly, MA, USA), 9 μl of the 3.9 kb PCR fragment, and 1 μl of 10X ligation buffer (New England Biolabs, Inc., Beverly, MA, USA). The ligation was heat inactivated for 10 minutes at 65°C and then digested with *Dpn* I at 37°C for 2 hours. After incubation, the digestion was purified using a GFX® PCR DNA and Gel Band Purification Kit.

The purified material was then transformed into *E. coli* TOP10 competent cells (Invitrogen Corp., Carlsbad, CA, USA) according to the manufacturer's instructions. The transformation mixture was plated onto LB plates supplemented with 25 μg of chloramphenicol per ml. Plasmid minipreps were prepared from several transformants and digested with *Bgl* II. One plasmid with the correct construction was chosen. The plasmid was designated pSigA4. Plasmid pSigA4 contains the *Bgl* II flanked transposon SigA2 identical to that disclosed in WO 01/77315.

A 60 μl sample of plasmid pSigA4 DNA (0.3 μg/μl) was digested with *Bgl* II and separated on a 0.8% agarose gel using 90 mM Tris-borate-2 mM EDTA pH 8.0 buffer. A SigA2 transposon DNA band of 2 kb was eluted with 200 μl of EB buffer (QIAGEN GmbH Corporation, Hilden, Germany) and purified using a GFX® PCR DNA and Gel Band Purification Kit according to the manufacturer's instructions and eluted in 200 μl of EB buffer. SigA2 was used for transposon assisted signal trapping.

**Example 5: Transposon Assisted Signal Trapping of *Penicillium pinophilum* strain**

A complete description of transposon assisted signal trapping can be found in WO 2001/77315. The plasmid pool (Example 3) was treated with transposon SigA2 and HYPERMU™ transposase (Epicenter Biotechnologies, Madison, WI, USA) according to the manufacturer's instructions.

For *in vitro* transposon tagging of the *P. pinophilum* cDNA library, 2 μl of SigA2 transposon containing approximately 100 ng of DNA were mixed with 1 μl of the plasmid DNA pool of the *P. pinophilum* cDNA library containing 1 μg of DNA, 1 μl of HyperMu™ transposase, and 2 μl of 10X buffer (Epicenter Biotechnologies, Madison, WI, USA) in a total volume of 20 μl and incubated at 30°C for 3 hours followed by addition of 2 μl of stop solution (Epicenter Biotechnologies, Madison, WI, USA) and heat inactivation at 75°C for 10 minutes. The DNA was precipitated by addition of 2 μl of 3 M sodium acetate pH 5 and 55 μl of 96% ethanol and centrifuged for 30 minutes at 10,000 x g, 4°C. The pellet was washed in 70% ethanol, air dried at room temperature, and resuspended in 10 μl of deionized water.

A 2 μl volume of the transposon tagged plasmid pool was electroporated into 50 μl of *E. coli* ELECTROMAX™ DH10B™ cells according to the manufacturer's instructions using a GENE PULSER® and Pulse Controller at 25 μF, 25 mAmp, 1.8 kV with a 1 mm gap cuvette according to the manufacturer's procedure.

The electroporated cells were incubated in SOC medium with shaking at 225 rpm for 1 hour at 37°C before being plated on the following selective media: LB plates supplemented with 50 μg of kanamycin per ml; LB plates supplemented with 50 μg of kanamycin per ml and 15 μg of chloramphencol per ml; and LB plates supplemented with 50 μg of kanamycin per ml, 15 μg of chloramphencol per ml, and 30 μg of ampicillin per ml.

From plating of the electroporation onto LB plates supplemented with kanamycin, chloramphencol and ampicillin, approximately 200 colonies per 50 μl were observed after 3 days at 30°C. All colonies were replica plated onto LB plates supplemented with 50 μg of kanamycin per ml, 15 μg of chloramphencol per ml, and 100 μg of ampicillin per ml. Five hundred colonies were recovered under this selection condition. The colonies were sequenced with the transposon forward and reverse primers (primers A and B), shown below, according to the procedure disclosed in WO 01/77315 (page 28).

Primer A:

5'-agcgtttgcggccgcgatcc-3' (SEQ ID NO: 5)

Primer B:

5'-ttattcggtcgaaaaggatc c-3' (SEQ ID NO: 6)

**Example 6: Sequence assembly and annotation**

DNA sequences were obtained from SinoGenoMax Co. Ltd. (Beijing, China). Primer A and primer B sequence reads for each plasmid were trimmed to remove vector and transposon sequence. The assembled sequences were grouped into contigs by using the program PhredPhrap (Ewing et al., 1998, Genome Research 8: 175-185; Ewing and Green, 1998, Genome Research 8: 186-194). All contigs were subsequently compared to sequences available in standard public DNA and protein sequences databases (TrEMBL, SWALL, PDB, EnsemblPep, GeneSeqP) by using the program BLASTX 2.0a19MP-WashU [14-Jul-1998] [Build linux-x86 18:51:44 30-Jul-1998] (Gish et al., 1993, Nat. Genet. 3: 266-72). A Family 7 endoglucanase was identified directly by analysis of the BlastX results.

**Example 7: Cloning of the *Penicillium pinophilum* GH7 endoglucanase gene from genomic DNA**

*P. pinophilum* was grown on PDA plate at 37°C for 3 days. Mycelia were collected directly from the agar plate into a sterilized mortar and frozen under liquid nitrogen. Frozen mycelia were ground, by mortar and pestle, to a fine powder, and genomic DNA was isolated using a DNEASY® Plant Mini Kit (QIAGEN Inc., Valencia, CA, USA).

Based on the *P. pinophilum* GH7 endoglucanase gene sequence obtained in Example 6, oligonucleotide primers, shown below, were designed to amplify the gene from genomic DNA of *P. pinophilum.* An IN-FUSION™ CF Dry-down PCR Cloning Kit (Clontech Laboratories, Inc., Mountain View, CA, USA) was used to clone the fragment directly into the expression vector pPFJO355, without the need for restriction digestion and ligation.

Sense primer:

5'-ACACAACTGGGGATCCACC**ATGTCTAACATGGCGACTAGACCATTG**-3' (SEQ ID NO: 7)

Antisense primer:

5'-GTCACCCTCTAGATCT**TCAAAGGCACTGTGAATAGTACGGATTCTGTA**-3' (SEQ ID NO: 8)

Bold letters represent the coding sequence for the sense primer or the reverse compliment sequence including the stop codon for the antisense primer. The remaining sequence is homologous to the insertion sites of pPFJO355.

The expression vector pPFJO355 contains the TAKA amylase promoter derived from *Aspergillus oryzae*, the *Aspergillus niger* glucoamylase terminator elements, pUC18 derived sequences for selection and propagation in *E. coli*, and an *Aspergillus nidulans pyrG* gene, which encodes an orotidine decarboxylase for selection of a transformant of a *pyrG* mutant *Aspergillus* strain.

Twenty picomoles of each of the primers above were used in a PCR reaction composed of *P. pinophilum* genomic DNA, 10 μl of 5X GC Buffer (Takara Biotechnology Co., Ltd., Dalian, China), 1.5 μl of DMSO, 2 μl of 2.5 mM each of dATP, dTTP, dGTP, and dCTP, and 1 unit of PHUSION™ High-Fidelity DNA Polymerase (Finnzymes Oy, Espoo, Finland) in a final volume of 50 µl. The amplification was performed using a Peltier Thermal Cycler (MJ Research, Inc., Waltham, MA, USA) programmed for denaturing at 98°C for 1 minutes; 5 cycles of denaturing at 98°C for 15 seconds, annealing at 70°C for 30 seconds, with a 1°C decrease per cycle and elongation at 72°C for 30 seconds; 25 cycles each at 98°C for 15 seconds and 72°C for 90 seconds; and a final extension at 72°C for 10 minutes. The heat block then went to a 4°C soak cycle.

The reaction products were isolated by 1.0% agarose gel electrophoresis using 90 mM Tris-borate and 1 mM EDTA (TBE) buffer where an approximate 1.5 kb product band was excised from the gel, and purified using an illustra GFX® PCR DNA and Gel Band Purification Kit (GE Healthcare, Buckinghamshire, UK) according to the manufacturer's instructions.

Plasmid pPFJO355 was digested with *Bam* I and *Bgl* II, isolated by 1.0% agarose gel electrophoresis using TBE buffer, and purified using an illustra GFX® PCR DNA and Gel Band Purification Kit according to the manufacturer's instructions.

The gene fragment and the digested vector were ligated together using an IN-FUSION™ CF Dry-down PCR Cloning Kit resulting in pPpin13 (Figure 2) in which transcription of the *P. pinophilum* GH7 endoglucanse gene was under the control of the *Aspergillus oryzae* TAKA alpha-amylase promoter. In brief, 30 ng of pPFJO355 digested with *Bam* I and *Bgl* II, and 50 ng of the *P. pinophilum* GH7 endoglucanase gene purified PCR product were added to a reaction vial and the reaction powder was resuspended in a final volume of 10 µl with addition of deionized water. The reaction was incubated at 37°C for 15 minutes and then 50°C for 15 minutes. Three µl of the reaction were used to transform *E. coli* TOP10 competent cells (TIANGEN Biotech (Beijing) Co. Ltd., Beijing, China). An *E. coli* transformant containing pPpin13 was detected by colony PCR and plasmid DNA was prepared using a QIAprep Spin Miniprep Kit (QIAGEN Inc., Valencia, CA, USA). The P. *pinophilum* GH7 endoglucanase coding sequence inserted in pPpin13 was confirmed by DNA sequencing using a 3730XL DNA Analyzer (Applied Biosystems Inc, Foster City, CA, USA).

The same gene fragment was then incubated in 10X *Taq* DNA polymerase mix (TIANGEN Biotech (Beijing) Co. Ltd., Beijing, China) at 72°C for 20 minutes to add adenine to the 3' end of each nucleotide strand. Then the gene fragment was ligated to plasmid pGEM-T using a pGEM-T Vector System (Promega Corporation, Madison, WI, USA), to generate pGEM-T-Ppin13. The *Penicillium pinophilum* endoglucanase gene inserted in pGEM-T-Ppin13 was confirmed by DNA sequencing using a 3730XL DNA Analyzer. *E. coli* strain T-Ppin13, containing pGEM-T-Ppin13, was deposited on October 28, 2010 with the Deutsche Sammlung von Mikroorganismen und Zellkulturen GmbH (DSMZ), Mascheroder Weg 1 B, D-38124 Braunschweig, Germany, and assigned the accession number DSM 24144.

### Example 8: Characterization of the *Penicillium pinophilum* genomic DNA sequence encoding a Family GH7 endoglucanase

Nucleotide sequence data were scrutinized for quality and all sequences were compared to each other with assistance of PHRED/PHRAP software (University of Washington, Seattle, WA, USA).

The nucleotide sequence (SEQ ID NO: 1) and deduced amino acid sequence (SEQ ID NO: 2) of the *Penicillium pinophilum* endoglucanase genomic DNA sequence are shown in Figure 1. The genomic fragment of 1497 bp including the stop codon encodes a polypeptide of 498 amino acids, with no predicted introns. The % G+C content of the full-length coding sequence and the mature coding sequence are 49.93% and 49.6%, respectively. Using the SignalP software program (Nielsen et al., 1997, Protein Engineering 10: 1-6), a signal peptide of 23 residues was predicted. The predicted mature protein contains 475 amino acids with a predicted molecular mass of 51.25 kDa and an isoelectric point of 4.55. Amino acids 24 to 419 are indicative of a Family 7 glycosyl hydrolase according to Coutinho and Henrissat, 1999, *supra.* Amino acids 463 to 498 are indicative of a cellulose binding domain.

A comparative pairwise global alignment of amino acid sequences was determined using the Needleman-Wunsch algorithm (Needleman and Wunsch, 1970, J. Mol. Biol. 48: 443-453) as implemented in the Needle program of EMBOSS with gap open penalty of 10, gap extension penalty of 0.5, and the EBLOSUM62 matrix. The alignment showed that the deduced amino acid sequence of the mature polypeptide of the *Penicillium pinophilum* Family GH7 endoglucanase genomic DNA sequence shares 87.76% identity (excluding gaps) to the deduced amino acid sequence of a *Penicillium marneffei* endoglucanase gene (UNIPROT:B6QWD1).

### Example 9: Expression of *Penicillium pinophilum* GH7 EG gene in *Aspergillus oryzae*

*Aspergillus oryzae* HowB101 (WO 95/35385) protoplasts were prepared according to the method of Christensen et al., 1988, Bio/Technology 6: 1419-1422 and transformed with 3 µg of pPpin13. The transformation yielded about 50 transformants. Four transformants were isolated to individual Minimal medium plates.

Four transformants were inoculated separately into 3 ml of YPM medium in a 24-well plate and incubated at 30°C with mixing at 150 rpm. After 3 days incubation, 20 µl of supernatant from each culture were analyzed by SDS-PAGE using a NUPAGE® Novex 4-12% Bis-Tris Gel with 50 mM 2-(N-morpholino)ethanesulfonic acid (MES) (Invitrogen Corporation, Carlsbad, CA, USA) according to the manufacturer's instructions. The resulting gel was stained with InstantBlue (Expedeon Ltd., Babraham Cambridge, UK). SDS-PAGE profiles of the cultures showed that the majority of the transformants had a band of approximately 80 kDa. One transformant was chosen as the expression strain and designated *Aspergillus oryzae* EXP02777.

A slant of *Aspergillus oryzae* EXP02777 was washed with 10 ml of YPM and inoculated into five 2-liter flasks, each containing 400 ml of YPM medium, to generate broth for characterization of the enzyme. The culture was harvested on day 3 by filtering the culture against MIRACLOTH® (CALBIOCHEM, Inc. La Jolla, CA, USA). The filtered culture broth was then again filtered using a 0.45 μm DURAPORE® Membrane (Millipore, Bedford, MA, USA).

**Example 10: Purification of the *Penicillium pinophilum* endoglucanase**

A 1750 ml volume of the *Aspergillus oryzae* EXP02777 filtered supernatant prepared as described in Example 9 was precipitated with ammonium sulfate (80% saturation), redissolved in 100 ml of 25 mM Bis-Tris pH 5.5, dialyzed against the same buffer, and filtered through a 0.45 μm filter. The final volume was 200 ml. The solution was applied to a 40 ml Q SEPHAROSE® Fast Flow column (GE Healthcare, Buckinghamshire, UK) equilibrated in 25 mM Bis-Tris pH 5.5, and the proteins were eluted with a linear 0-0.3 M NaCl gradient. Fractions from the column were analyzed by SDS-PAGE using a NuPAGE® Novex 4-12% Bis-Tris Gel. Fractions with a molecular weight of 80 kDa were pooled and then concentrated by ultrafiltration.

The concentrate was assayed for endoglucanase activity by a microtiter plate assay as described below. A solution of 0.2% AZCL-HE-Cellulose (Megazyme International Ireland, Ltd., Bray, Co. Wicklow, Ireland) was made in a 0.1 M sodium acetate pH 5.5 buffer with stirring. The solution was distributed under stirring to a microtiter plate (200 μl to each well). Then 20 μl of enzyme sample was added and incubated in an EPPENDORF® THERMOMIXER® (Eppendorf AG, Hamburg, Germany) for 20 minutes at 50°C and 650 rpm. A denatured enzyme sample (100°C boiling for 20 minutes) was used as blank. After incubation the colored solution was separated from the solid substrate by centrifugation at 3000 rpm for 5 minutes at 4°C. A 150 μl sample of supernatant was transferred to a microtiter plate; and the absorbance was measured at 595 nm using a SPECTRAMAX® M2 Multi-Mode Microplate Reader (Molecular Devices, Beijing, China). The assay results demonstrated that the presence of endoglucanase activity.

**Example 11: Characterization of *Penicillium pinophilum* GH7 endoglucanase**

Specific activity. The specific activity of the *Penicillium pinophilum* GH7 endoglucanase was determined using carboxymethyl cellulose (CMC; Hercules Inc., Wilmington, DE, USA) as substrate. Ten g of CMC per liter was prepared in 50 mM sodium acetate pH 5.0 with 0.01 % TWEEN® 20. To 190 μl of the CMC solution was added 10 μl of the *Penicillium pinophilum* GH7 endoglucanase at different loadings. A substrate control and enzyme control were included. The reaction was incubated at 50°C for 30 minutes, before 50 μl of 0.5 M NaOH were added to stop the reaction. The reducing sugars produced were determined using para-hydroxybenzoic acid hydrazide (PHBAH, Sigma Chemical Co., Inc., St. Louis, MO, USA) in an assay adapted to a 96 well microplate format as described below. Briefly, a 100 μl aliquot of an appropriately diluted sample was placed in a 96-well conical bottomed microplate. Reactions were initiated by adding 50 μl of 1.5% (w/v) PHBAH in 2% NaOH to each well. The plates were heated uncovered at 95°C for 10 minutes and then allowed to cool to room temperature (RT). Fifty μl of distilled water were then added to each well. A 100 μl aliquot from each well was transferred to a flat bottomed 96 well plate and the absorbance at 410 nm was measured using a SPECTRAMAX® Microplate Reader (Molecular Devices, Sunnyvale, CA, USA). Glucose standards (0.1-0.0125 mg/ml diluted with 0.4% sodium hydroxide) were used to prepare a standard curve to convert the obtained $A_{410nm}$ values into glucose equivalents. The enzyme loading versus the reducing sugars produced was plotted and the linear range was used to calculate the specific activity of *Penicillium pinophilum* GH7 endoglucanase, as expressed as μmole of glucose equivalent produced per minute per mg enzyme, or IU/mg. The results are shown in Table 1.

Table 1. Specific activity on CMC

| Enzyme | Specific activity on CMC, IU/mg |
|---|---|
| *P. pinophilum* GH7 EG | 144.2 |
| *T. reesei* 7 B EG | 43.2 |

Thermostability. The *P. pinophilum* GH7 endoglucanase was diluted in 50 mM sodium acetate pH 5 containing 0.01% TWEEN® 20, and then incubated at 60°C for 3 hours and 24 hours. The same sample was stored at 4°C to serve as control. After incubation, the activity of the samples on CMC was measured following the assay protocol described above for determining specific activity, but using one enzyme loading that yielded less than 5% conversion in the specific activity assay. The activity of the sample at 4°C was normalized to 100%, and the activities of the other samples at other incubation conditions were compared to the 4°C activity. The thermostability of the *P. pinophilum* GH7 endoglucanase is shown in Table 2.

Table 2. Thermostability

| Incubation condition | Residual activity on CMC |
|---|---|
| 4°C | 100% |
| 50°C, 3 days | 100% |
| 60°C, 3 hours | 2.6% |
| 60°C, 24 hours | 0% |

pH profile. The pH activity profile of the *P. pinophilum* GH7 endoglucanase was determined relative to the *T. reesei* GH7B endoglucanase using the same protocol described above for determining the specific activity, except the endoglucanase was incubated at five different pHs (4, 5, 6, 7, and 8) using one enzyme loading that yielded less than 5% conversion in the specific activity assay. Britton Robinson buffer was used as the buffer system. A 100 mM stock solution of the Britton Robinson buffer was prepared by dissolving 0.1 mole of boric acid, 0.1 mole of acetic acid, and 0.1 mole of phosphoric acid in 1 liter of deionized water. The 100 mM stock solution was then titrated to pHs 4, 5, 6, 7, and 8 using 5 M NaOH and then diluted to 40 mM with deionized water. CMC was dissolved in the buffer, and the activity was measured at 50°C. The highest activity was normalized to be 100%, and activities at other pH values were compared to the highest activity and expressed in % activity. The pH profile of the *P. pinophilum* GH7 endoglucanase and *T. reesei* GH7B endoglucanase are shown in Table 3.

Table 3. pH profile

| | Activity on CMC | |
|---|---|---|
| pH value | *P. pinophilum* GH7 EG | *T. reesei* GH7B EG |
| 4.0 | 71.5% | 51.3% |
| 5.0 | 100% | 100% |
| 6.0 | 94.2% | 75.2% |
| 7.0 | 52.2% | 31.3% |
| 8.0 | 0% | 1.6% |

**Example 12: Pretreated corn stover hydrolysis assay**

Corn stover was pretreated at the U.S. Department of Energy National Renewable Energy Laboratory (NREL) using 1.4 wt % sulfuric acid at 165°C and 107 psi for 8 minutes. The water-insoluble solids in the pretreated corn stover (PCS) contained 56.5% cellulose, 4.6% hemicelluloses, and 28.4% lignin. Cellulose and hemicellulose were determined by a two-stage sulfuric acid hydrolysis with subsequent analysis of sugars by high performance liquid chromatography using NREL Standard Analytical Procedure #002. Lignin was determined gravimetrically after hydrolyzing the cellulose and hemicellulose fractions with sulfuric acid using NREL Standard Analytical Procedure #003.

Milled unwashed PCS (dry weight 32.35%) was prepared by milling whole slurry PCS in a Cosmos ICMG 40 wet multi-utility grinder (EssEmm Corporation, Tamil Nadu, India).

The hydrolysis of PCS was conducted using 2.2 ml deep-well plates (Axygen, Union City, CA, USA) in a total reaction volume of 1.0 ml. The hydrolysis was performed with 50 mg of insoluble PCS solids per ml of 50 mM sodium acetate pH 5.0 buffer containing 1 mM manganese sulfate and various protein loadings of various enzyme compositions (expressed as mg protein per gram of cellulose). Enzyme compositions were prepared and then added simultaneously to all wells in a volume ranging from 50 μl to 200 μl, for a final volume of 1 ml in each reaction. The plates were then sealed using an ALPS-300™ plate heat sealer (Abgene, Epsom, United Kingdom), mixed thoroughly, and incubated at a specific temperature for 72 hours. All experiments reported were performed in triplicate.

Following hydrolysis, samples were filtered using a 0.45 μm MULTISCREEN® 96-well filter plate (Millipore, Bedford, MA, USA) and filtrates analyzed for sugar content as described below. When not used immediately, filtered aliquots were frozen at -20°C. The sugar concentrations of samples diluted in 0.005 M $H_2SO_4$ were measured using a 4.6 x 250 mm AMINEX® HPX-87H column (Bio-Rad Laboratories, Inc., Hercules, CA, USA) by elution with 0.05% w/w benzoic acid-0.005 M $H_2SO_4$ at 65°C at a flow rate of 0.6 ml per minute, and quantitation by integration of the glucose, cellobiose, and xylose signals from refractive index detection (CHEMSTATION®, AGILENT® 1100 HPLC, Agilent Technologies, Santa Clara, CA, USA) calibrated by pure sugar samples. The resultant glucose and cellobiose equivalents were used to calculate the percentage of cellulose conversion for each reaction.

Glucose, cellobiose, and xylose were measured individually. Measured sugar concentrations were adjusted for the appropriate dilution factor. In case of unwashed PCS, the net concentrations of enzymatically-produced sugars were determined by adjusting the measured sugar concentrations for corresponding background sugar concentrations in unwashed PCS at zero time points. All HPLC data processing was performed using MICROSOFT EXCEL™ software (Microsoft, Seattle, WA, USA).

The degree of cellulose conversion to glucose was calculated using the following equation: % conversion = (glucose concentration / glucose concentration in a limit digest) x 100. In order to calculate % conversion, a 100% conversion point was set based on a cellulase control (100 mg of *Trichoderma reesei* cellulase per gram cellulose), and all values were divided by this number and then multiplied by 100. Triplicate data points were averaged and standard deviation was calculated.

**Example 13: Preparation of a high temperature enzyme composition**

*Aspergillus fumigatus* Cel7A NN055679 cellobiohydrolase I (CBHI) (SEQ ID NO: 9 [DNA sequence] and SEQ ID NO: 10 [deduced amino acid sequence]) was prepared recombinantly in *Aspergillus oryzae* JAL250 as described in WO 2011/057140. The filtered broth of *Aspergillus fumigatus* GH7A cellobiohydrolase I was concentrated and buffer exchanged using a tangential flow concentrator (Pall Filtron, Northborough, MA, USA) equipped with a 10 kDa polyethersulfone membrane (Pall Filtron, Northborough, MA, USA) with 20 mM Tris-HCl pH 8.0. The desalted broth of *Aspergillus fumigatus* GH7A cellobiohydrolase I was purified over a Q SEPHAROSE® ion exchange chromatography column (GE Healthcare, Piscataway, NJ, USA)) in 20 mM Tris-HCl pH 8, over a linear 0 to 1 M NaCl gradient. Fractions were collected and fractions containing the cellobiohydrolase I were pooled based on 8-16% CRITERION® Stain-free SDS-PAGE (Bio-Rad Laboratories, Inc., Hercules, CA, USA).

*Aspergillus fumigatus* GH6A cellobiohydrolase II (SEQ ID NO: 11 [DNA sequence] and SEQ ID NO: 12 [deduced amino acid sequence]) was prepared recombinantly in *Aspergillus oryzae* as described in WO 2011/057140. The filtered broth of *Aspergillus fumigatus* GH6A cellobiohydrolase II was purified according to WO 2011/057140.

*Thermoascus aurantiacus* GH61A polypeptide (SEQ ID NO: 13 [DNA sequence] and SEQ ID NO: 14 [deduced amino acid sequence]) was recombinantly prepared according to WO 2005/074656 using *Aspergillus oryzae* JaL250 as a host. The filtered broth of *Thermoascus aurantiacus* GH61A polypeptide was first concentrated by ultrafiltration using a 10 kDa membrane, buffer exchanged into 20 mM Tris-HCl pH 8.0, and then purified using a 320 ml SUPERDEX® 75 SEC column (GE Healthcare, Piscataway, NJ, USA) with isocratic elution of 150 mM NaCl-20 mM Tris-HCl pH 8.0. Fractions were collected and pooled based on 8-16% CRITERION® Stain-free SDS-PAGE (Bio-Rad Laboratories, Inc., Hercules, CA, USA).

*Aspergillus fumigatus* Cel3A beta-glucosidase (SEQ ID NO: 15 [DNA sequence] and SEQ ID NO: 16 [deduced amino acid sequence]) was recombinantly prepared according to WO 2005/047499 using *Aspergillus oryzae* as a host. The filtered broth of *Aspergillus fumigatus* Cel3A beta-glucosidase was concentrated and buffer exchanged using a tangential flow concentrator equipped with a 10 kDa polyethersulfone membrane with 20 mM Tris-HCl pH 8.5. The sample was loaded onto a Q SEPHAROSE® High Performance column (GE Healthcare, Piscataway, NJ, USA) equilibrated in 20 mM Tris pH 8.5, and bound proteins were eluted with a linear gradient from 0-600 mM sodium chloride. The fractions were concentrated and loaded onto a SUPERDEX® 75 HR 26/60 column equilibrated with 20 mM Tris-150 mM sodium chloride pH 8.5.

*Aspergillus fumigatus* NN055679 GH10 xylanase (xyn3) (SEQ ID NO: 17 [DNA sequence] and SEQ ID NO: 18 [deduced amino acid sequence]) was prepared recombinantly according to WO 2006/078256 using *Aspergillus oryzae* BECh2 (WO 2000/39322) as a host. The filtered broth of *Aspergillus fumigatus* NN055679 GH10 xylanase (xyn3) was desalted and buffer-exchanged into 50 mM sodium acetate pH 5.0 using a HIPREP® 26/10 Desalting Column (GE Healthcare, Piscataway, NJ, USA) according to the manufacturer's instructions.

The protein concentration for each of the monocomponents described above was determined using a Microplate BCA™ Protein Assay Kit (Thermo Fisher Scientific, Waltham, MA, USA) in which bovine serum albumin was used as a protein standard. Each of the components were combined together to produce a high temperature enzyme composition composed of 40% *Aspergillus fumigatus* Cel7A cellobiohydrolase I, 25% *Aspergillus fumigatus* Cel6A cellobiohydrolase II, 15% *Thermoascus aurantiacus* GH61A polypeptide having cellulolytic enhancing activity, 5% *Aspergillus fumigatus* beta-glucosidase, and 5% *Aspergillus fumigatus* GH10 xylanase (xyn3).

**Example 14: Effect of the *Penicillium pinophilum* GH7 endoglucanase on hydrolysis of milled unwashed PCS by a high-temperature enzyme composition**

The *Penicillium pinophilum* GH7 endoglucanase expressed by *Aspergillus oryzae* EXP02777 was evaluated in a high-temperature enzyme composition without endoglucanase at 50°C, 55°C, 60°C, and 65°C using milled unwashed PCS as a substrate. The high-temperature enzyme composition without endoglucanase (Example 13) was added to PCS hydrolysis reactions at 2.7 mg total protein per g cellulose and the hydrolysis results were compared with the results for a similar high-temperature enzyme composition with added GH7 endoglucanase (3.0 mg protein per g cellulose).

The assay was performed as described in Example 12. The 1 ml reactions with milled unwashed PCS (5% insoluble solids) were conducted for 72 hours in 50 mM sodium acetate pH 5.0 buffer containing 1 mM manganese sulfate. All reactions were performed in triplicate and involved single mixing at the beginning of hydrolysis.

The results shown in Figure 3 demonstrated that at 50°C, 55°C, 60°C, and 65°C the high-temperature enzyme composition that included *Penicillium pinophilum* GH7 endoglucanase significantly outperformed the enzyme composition containing no endoglucanase. The degree of cellulose conversion to glucose for *Penicillium pinophilum* GH7 endoglucanase was significantly higher than that of the high-temperature enzyme composition without endoglucanase.

**Deposit of Biological Material**

The following biological material has been deposited under the terms of the Budapest Treaty with the Deutsche Sammlung von Mikroorganismen und Zellkulturen GmbH (DSMZ), Mascheroder Weg 1 B, D-38124 Braunschweig, Germany, and assigned the following accession number:

| Deposit | Accession Number | Date of Deposit |
|---|---|---|
| *E. coli* T-Ppin13 | DSM 24144 | October 28, 2010 |

The strain has been deposited under conditions that assure that access to the culture will be available during the pendency of this patent application to one determined by foreign patent laws to be entitled thereto. The deposit represents a substantially pure culture of the deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

The present disclosure is further described by the following numbered paragraphs:

[1] An isolated polypeptide having endoglucanase activity, selected from the group consisting of: (a) a polypeptide having at least 90%, e.g., at least 91%, at least 92%, at least 93%, at least 94%, at least 95%, at least 96%, at least 97%, at least 98%, at least 99%, or 100% sequence identity to the mature polypeptide of SEQ ID NO: 2; (b) a polypeptide encoded by a polynucleotide having at least 90%, e.g., at least 91%, at least 92%, at least 93%, at least 94%, at least 95%, at least 96%, at least 97%, at least 98%, at least 99%, or 100% sequence identity to the mature polypeptide coding sequence of SEQ ID NO: 1; (c) a variant of the mature polypeptide of SEQ ID NO: 2 comprising a substitution, deletion, and/or insertion at one or more (e.g., several) positions, wherein the variant has endoglucanase activity; and (d) a fragment of a polypeptide of (a), (b), or (c) that has endoglucanase activity.

[2] The polypeptide of paragraph 1, having at least 90%, at least 91%, at least 92%, at least 93%, at least 94%, at least 95%, at least 96%, at least 97%, at least 98%, at least 99%, or 100% sequence identity to the mature polypeptide of SEQ ID NO: 2.

[3] The polypeptide of paragraph 1, comprising or consisting of SEQ ID NO: 2.

[4] The polypeptide of paragraph 1, comprising or consisting of the mature polypeptide of SEQ ID NO: 2.

[5] The polypeptide of paragraph 4, wherein the mature polypeptide is amino acids 24 to 498 of SEQ ID NO: 2.

[6] The polypeptide of paragraph 1, which is encoded by a polynucleotide having at least 90%, at least 91%, at least 92%, at least 93%, at least 94%, at least 95%, at least 96%, at least 97%, at least 98%, at least 99%, or 100% sequence identity to the mature polypeptide coding sequence of SEQ ID NO: 1.

[7] The polypeptide of paragraph 1, which is a variant of the mature polypeptide of SEQ ID NO: 2 comprising a substitution, deletion, and/or insertion at one or more (e.g., several) positions, wherein the variant has endoglucanase activity.

[8] The polypeptide of paragraph 1, which is a fragment of SEQ ID NO: 2, wherein the fragment has endoglucanase activity.

[9] The polypeptide of any of paragraphs 1-8, which is encoded by the polynucleotide contained in plasmid pGEM-T-Ppin13 which is contained in *E. coli* DSM 24144.

[10] An isolated polypeptide comprising a catalytic domain selected from the group consisting of: (a) a catalytic domain having at least 91%, e.g., at least 92%, at least 93%, at least 94%, at least 95%, at least 96%, at least 97%, at least 98%, at least 99%, or 100% sequence identity to amino acids 24 to 419 of SEQ ID NO: 2; (b) a catalytic domain encoded by a polynucleotide having at least 91%, e.g., at least 92%, at least 93%, at least 94%, at least 95%, at least 96%, at least 97%, at least 98%, at least 99%, or 100% sequence identity to nucleotides 70 to 1257 of SEQ ID NO: 1; (c) a variant of amino acids 24 to 419 of SEQ ID NO: 2 comprising a substitution, deletion, and/or insertion of one or more (e.g., several) positions; and (d) a fragment of a catalytic domain of (a), (b), or (c), which has endoglucanase activity.

[11] The polypeptide of paragraph 10, having at least at least 91%, at least 92%, at least 93%, at least 94%, at least 95%, at least 96%, at least 97%, at least 98%, at least 99%, or 100% sequence identity to the catalytic domain of SEQ ID NO: 2.

[12] The polypeptide of paragraph 10, comprising or consisting of the catalytic domain of SEQ ID NO: 2.

[13] The polypeptide of paragraph 12, wherein the catalytic domain is amino acids 24 to 419 of SEQ ID NO: 2.

[14] The polypeptide of paragraph 10, which is encoded by a polynucleotide having at least 91%, at least 92%, at least 93%, at least 94%, at least 95%, at least 96%, at least 97%, at least 98%, at least 99%, or 100% sequence identity to nucleotides 70 to 1257 of SEQ ID NO: 1.

[15] The polypeptide of paragraph 10, which is a variant of amino acids 24 to 419 of SEQ ID NO: 2 comprising a substitution, deletion, and/or insertion at one or more (e.g., several) positions, wherein the variant has endoglucanase activity.

[16] The polypeptide of paragraph 10, which is a fragment of amino acids 24 to 419 of SEQ ID NO: 2, wherein the fragment has endoglucanase activity.

[17] The polypeptide of any of paragraphs 10-16, which is encoded by the polynucleotide contained in plasmid pGEM-T-Ppin13 which is contained in *E. coli* DSM 24144.

[18] The polypeptide of any of paragraphs 10-17, further comprising a cellulose binding domain.

[19] An isolated polypeptide comprising a cellulose binding domain selected from the group consisting of: (a) a cellulose binding domain having at least 91%, e.g., at least 92%, at least 93%, at least 94%, at least 95%, at least 96%, at least 97%, at least 98%, at least 99%, or 100% sequence identity to amino acids 463 to 498 of SEQ ID NO: 2; (b) a cellulose binding domain encoded by a polynucleotide having at least 91%, e.g., at least 92%, at least 93%, at least 94%, at least 95%, at least 96%, at least 97%, at least 98%, at least 99%, or 100% sequence identity to nucleotides 1387 to 1494 of SEQ ID NO: 1; (c) a variant of amino acids 463 to 498 of SEQ ID NO: 2 comprising a substitution, deletion, and/or insertion at one or more (e.g., several) positions; and (d) a fragment of the cellulose binding domain of (a), (b), or (c), which has cellulose binding activity.

[20] The polypeptide of paragraph 16, having at least 91%, at least 92%, at least 93%, at least 94%, at least 95%, at least 96%, at least 97%, at least 98%, at least 99%, or 100% sequence identity to amino acids 463 to 498 of SEQ ID NO: 2.

[21] The polypeptide of paragraph 19, comprising or consisting of the cellulose binding domain of SEQ ID NO: 2.

[22] The polypeptide of paragraph 21, wherein the cellulose binding domain is amino acids 463 to 498 of SEQ ID NO: 2.

[23] The polypeptide of paragraph 16, which is encoded by a polynucleotide having at least 91%, at least 92%, at least 93%, at least 94%, at least 95%, at least 96%, at least 97%, at least 98%, at least 99%, or 100% sequence identity to nucleotides 1387 to 1494 of SEQ ID NO: 1.

[24] The polypeptide of paragraph 19, which is a variant of amino acids 463 to 498 of SEQ ID NO: 2 comprising a substitution, deletion, and/or insertion at one or more (e.g., several) positions, wherein the variant has cellulose binding activity.

[25] The polypeptide of paragraph 19, which is a fragment of the cellulose binding domain of SEQ ID NO: 2, wherein the fragment has cellulose binding activity.

[26] The polypeptide of any of paragraphs 19-25, which is encoded by the polynucleotide contained in plasmid pGEM-T-Ppin13 which is contained in *E. coli* DSM 24144.

[27] The polypeptide of any of paragraphs 19-26, further comprising a catalytic domain.

[28] A composition comprising the polypeptide of any of paragraphs 1-27.

[29] An isolated polynucleotide encoding the polypeptide of any of paragraphs 1-27.

[30] A nucleic acid construct or expression vector comprising the polynucleotide of paragraph 29 operably linked to one or more (e.g., several) control sequences that direct the production of the polypeptide in an expression host.

[31] A recombinant host cell comprising the polynucleotide of paragraph 29 operably linked to one or more (e.g., several) control sequences that direct the production of the polypeptide having endoglucanase activity.

[32] A method of producing the polypeptide of any of paragraphs 1-27, comprising: (a) cultivating a cell, which in its wild-type form produces the polypeptide, under conditions conducive for production of the polypeptide; and (b) recovering the polypeptide.

[33] A method of producing a polypeptide having endoglucanase activity, comprising: (a) cultivating the recombinant host cell of paragraph 31 under conditions conducive for production of the polypeptide; and (b) recovering the polypeptide.

[34] A transgenic plant, plant part or plant cell transformed with a polynucleotide encoding the polypeptide of any of paragraphs 1-27.

[35] A method of producing a polypeptide having endoglucanase activity, comprising: (a) cultivating the transgenic plant or plant cell of paragraph 34 under conditions conducive for production of the polypeptide; and (b) recovering the polypeptide.

[36] A method of producing a mutant of a parent cell, comprising inactivating a polynucleotide encoding the polypeptide of any of paragraphs 1-27, which results in the mutant producing less of the polypeptide than the parent cell.

[37] A mutant cell produced by the method of paragraph 36.

[38] The mutant cell of paragraph 37, further comprising a gene encoding a native or heterologous protein.

[39] A method of producing a protein, comprising: (a) cultivating the mutant cell of paragraph 37 or 38 under conditions conducive for production of the protein; and (b) recovering the protein.

[40] A double-stranded inhibitory RNA (dsRNA) molecule comprising a subsequence of the polynucleotide of paragraph 29, wherein optionally the dsRNA is an siRNA or an miRNA molecule.

[41] The double-stranded inhibitory RNA (dsRNA) molecule of paragraph 40, which is about 15, 16, 17, 18, 19, 20, 21, 22, 23, 24, 25 or more duplex nucleotides in length.

[42] A method of inhibiting the expression of a polypeptide having endoglucanase activity in a cell, comprising administering to the cell or expressing in the cell the double-stranded inhibitory RNA (dsRNA) molecule of paragraph 40 or 41.

[43] A cell produced by the method of paragraph 42.

[44] The cell of paragraph 43, further comprising a gene encoding a native or heterologous protein.

[45] A method of producing a protein, comprising: (a) cultivating the cell of paragraph 43 or 44 under conditions conducive for production of the protein; and (b) recovering the protein.

[46] An isolated polynucleotide encoding a signal peptide comprising or consisting of amino acids 1 to 23 of SEQ ID NO: 2.

[47] A nucleic acid construct or expression vector comprising a gene encoding a protein operably linked to the polynucleotide of paragraph 46, wherein the gene is foreign to the polynucleotide encoding the signal peptide.

[48] A recombinant host cell comprising a gene encoding a protein operably linked to the polynucleotide of paragraph 46, wherein the gene is foreign to the polynucleotide encoding the signal peptide.

[49] A method of producing a protein, comprising: (a) cultivating a recombinant host cell comprising a gene encoding a protein operably linked to the polynucleotide of paragraph 46, wherein the gene is foreign to the polynucleotide encoding the signal peptide, under conditions conducive for production of the protein; and (b) recovering the protein.

[50] A method for degrading or converting a cellulosic material, comprising: treating the cellulosic material with an enzyme composition in the presence of the polypeptide having endoglucanase activity of any of paragraphs 1-27.

[51] The method of paragraph 50, wherein the cellulosic material is pretreated.

[52] The method of paragraph 50 or 51, further comprising recovering the degraded cellulosic material.

[53] The method of any of paragraphs 50-52, wherein the enzyme composition comprises one or more (e.g., several) enzymes selected from the group consisting of a cellulase, a GH61 polypeptide having cellulolytic enhancing activity, a hemicellulase, an esterase, an expansin, a laccase, a ligninolytic enzyme, a pectinase, a peroxidase, a protease, and a swollenin.

[54] The method of paragraph 53, wherein the cellulase is one or more (e.g., several) enzymes selected from the group consisting of an endoglucanase, a endoglucanase, and a beta-glucosidase.

[55] The method of paragraph 53, wherein the hemicellulase is one or more (e.g., several) enzymes selected from the group consisting of a xylanase, an acetyxylan esterase, a feruloyl esterase, an arabinofuranosidase, a xylosidase, and a glucuronidase.

[56] The method of any of paragraphs 50-55, wherein the degraded cellulosic material is a sugar.

[57] The method of paragraph 56, wherein the sugar is selected from the group consisting of glucose, xylose, mannose, galactose, and arabinose.

[58] A method for producing a fermentation product, comprising: (a) saccharifying a cellulosic material with an enzyme composition in the presence of the polypeptide having endoglucanase activity of any of paragraphs 1-27; (b) fermenting the saccharified cellulosic material with one or more (e.g., several) fermenting microorganisms to produce the fermentation product; and (c) recovering the fermentation product from the fermentation.

[59] The method of paragraph 58, wherein the cellulosic material is pretreated.

[60] The method of paragraph 58 or 59, wherein the enzyme composition comprises one or more (e.g., several) enzymes selected from the group consisting of a cellulase, a GH61 polypeptide having cellulolytic enhancing activity, a hemicellulase, an esterase, an expansin, a laccase, a ligninolytic enzyme, a pectinase, a peroxidase, a protease, and a swollenin.

[61] The method of paragraph 60, wherein the cellulase is one or more (e.g., several) enzymes selected from the group consisting of an endoglucanase, a endoglucanase, and a beta-glucosidase.
[62] The method of paragraph 60, wherein the hemicellulase is one or more (e.g., several) enzymes selected from the group consisting of a xylanase, an acetyxylan esterase, a feruloyl esterase, an arabinofuranosidase, a xylosidase, and a glucuronidase.
[63] The method of any of paragraphs 58-62, wherein steps (a) and (b) are performed simultaneously in a simultaneous saccharification and fermentation.
[64] The method of any of paragraphs 58-63, wherein the fermentation product is an alcohol, an alkane, a cycloalkane, an alkene, an amino acid, a gas, isoprene, a ketone, an organic acid, or polyketide.
[65] A method of fermenting a cellulosic material, comprising: fermenting the cellulosic material with one or more (e.g., several) fermenting microorganisms, wherein the cellulosic material is saccharified with an enzyme composition in the presence of the polypeptide having endoglucanase activity of any of paragraphs 1-27.
[66] The method of paragraph 65, wherein the cellulosic material is pretreated before saccharification.
[67] The method of paragraph 65 or 66, wherein the enzyme composition comprises one or more (e.g., several) enzymes selected from the group consisting of a cellulase, a GH61 polypeptide having cellulolytic enhancing activity, a hemicellulase, an esterase, an expansin, a laccase, a ligninolytic enzyme, a pectinase, a peroxidase, a protease, and a swollenin.
[68] The method of paragraph 67, wherein the cellulase is one or more (e.g., several) enzymes selected from the group consisting of an endoglucanase, a endoglucanase, and a beta-glucosidase.
[69] The method of paragraph 67, wherein the hemicellulase is one or more (e.g., several) enzymes selected from the group consisting of a xylanase, an acetyxylan esterase, a feruloyl esterase, an arabinofuranosidase, a xylosidase, and a glucuronidase.
[70] The method of any of paragraphs 65-69, wherein the fermenting of the cellulosic material produces a fermentation product.
[71] The method of paragraph 70, further comprising recovering the fermentation product from the fermentation.
[72] The method of paragraph 70 or 71, wherein the fermentation product is an alcohol, an alkane, a cycloalkane, an alkene, an amino acid, a gas, isoprene, a ketone, an organic acid, or polyketide.
[73] A whole broth formulation or cell culture composition comprising a polypeptide of any of paragraphs 1-27.

## Claims

1. Method for degrading or converting a cellulosic material, comprising treating the cellulosic material at a temperature in the range from about 50°C to about 65°C, with an enzyme composition in the presence of an isolated polypeptide having endoglucanase activity, selected from the group consisting of:

   (a) a polypeptide having at least 90%, e.g., at least 91%, at least 92%, at least 93%, at least 94%, at least 95%, at least 96%, at least 97%, at least 98%, at least 99%, or 100% sequence identity to the mature polypeptide of SEQ ID NO: 2;
   (b) a polypeptide encoded by a polynucleotide having a sequence identity to the mature polypeptide coding sequence of SEQ ID NO: 1 of at least 90%, e.g., at least 91%, at least 92%, at least 93%, at least 94%, at least 95%, at least 96%, at least 97%, at least 98%, at least 99%, or 100%;
   (c) a variant of the mature polypeptide of SEQ ID NO: 2 comprising a substitution, deletion, and/or insertion at one or more positions, wherein the variant has endoglucanase activity and wherein the number of amino acid substitutions, deletions and/or insertions introduced into the mature polypeptide of SEQ ID NO: 2 is up to 10, e.g., 1, 2, 3, 4, 5, 6, 7, 8, 9, or 10; and
   (d) a fragment of a polypeptide of (a), (b), or (c) that has endoglucanase activity,

   wherein at 50°C, 55°C, 60°C, and 65°C the degree of cellulose conversion to glucose is higher than that of the enzyme composition without endoglucanase.

2. Method of claim 1, wherein the isolated polypeptide comprises or consists of SEQ ID NO: 2 or the mature polypeptide thereof.

3. Method of claim 1, wherein the isolated polypeptide comprises amino acids 24 to 498 of SEQ ID NO: 2.

4. Method of claim 1, wherein the isolated polypeptide consists of amino acids 24 to 498 of SEQ ID NO: 2.

5. Method of claim 1, wherein the isolated polypeptide is encoded by the polynucleotide contained in plasmid pGEM-T-Ppin13 which is contained in *E. coli* DSM 24144.

6. The method of any of claims 1-5, further comprising recovering the degraded cellulosic material.

7. A method for producing a fermentation product, comprising:

   (a) saccharifying a cellulosic material at a temperature in the range from about 50°C to about 65°C with an enzyme composition in the presence of the polypeptide having endoglucanase activity as defined in any of claims 1-5;
   (b) fermenting the saccharified cellulosic material with one or more fermenting microorganisms to produce the fermentation product; and
   (c) recovering the fermentation product from the fermentation.

8. A method of fermenting a cellulosic material, comprising: fermenting the cellulosic material with one or more fermenting microorganisms, wherein the cellulosic material is saccharified at a temperature in the range from about 50°C to about 65°C with an enzyme composition in the presence of the polypeptide having endoglucanase activity as defined in any of claims 1-5.

9. The method of claim 8, wherein the fermenting of the cellulosic material produces a fermentation product.

10. The method of claim 9, further comprising recovering the fermentation product from the fermentation.

## Patentansprüche

1. Verfahren zum Abbauen oder Umwandeln eines cellulosischen Materials, umfassend Behandeln des cellulosischen Materials bei einer Temperatur im Bereich von etwa 50°C bis etwa 65°C mit einer Enzymzusammensetzung in der Gegenwart eines isolierten Polypeptids mit Endoglucanaseaktivität, ausgewählt aus der Gruppe bestehend aus:

(a) einem Polypeptid mit mindestens 90%, z.B. mindestens 91%, mindestens 92%, mindestens 93%, mindestens 94%, mindestens 95%, mindestens 96%, mindestens 97%, mindestens 98%, mindestens 99% oder 100% Sequenzidentität zum reifen Polypeptid von SEQ ID NO: 2;
(b) einem Polypeptid, das durch ein Polynukleotid mit einer Sequenzidentität zur das reife Polypeptid kodierenden Sequenz von SEQ ID NO: 1 von mindestens 90%, z.B. mindestens 91%, mindestens 92%, mindestens 93%, mindestens 94%, mindestens 95%, mindestens 96%, mindestens 97%, mindestens 98%, mindestens 99% oder 100% kodiert ist;
(c) einer Variante des reifen Polypeptids von SEQ ID NO: 2, die eine Substitution, Deletion und/oder Insertion an einer oder mehreren Positionen umfasst, wobei die Variante Endoglucanaseaktivität aufweist, und wobei die Anzahl an Aminosäuresubstitutionen, Deletionen und/oder Insertionen, die in das reife Polypeptid von SEQ ID NO: 2 eingeführt sind, bis zu 10 beträgt, z.B. 1, 2, 3, 4, 5, 6, 7, 8, 9 oder 10; und
(d) einem Fragment eines Polypeptids von (a), (b) oder (c), das Endoglucanaseaktivität aufweist,

wobei bei 50°C, 55°C, 60°C und 65°C der Grad an Celluloseumwandlung zu Glucose höher ist als der der Enzymzusammensetzung ohne Endoglucanase.

**2.** Verfahren nach Anspruch 1, wobei das isolierte Polypeptid SEQ ID NO: 2 oder das reife Polypeptid davon umfasst oder daraus besteht.

**3.** Verfahren nach Anspruch 1, wobei das isolierte Polypeptid Aminosäuren 24 bis 498 von SEQ ID NO: 2 umfasst.

**4.** Verfahren nach Anspruch 1, wobei das isolierte Polypeptid aus Aminosäuren 24 bis 498 von SEQ ID NO: 2 besteht.

**5.** Verfahren nach Anspruch 1, wobei das isolierte Polypeptid durch das Polynukleotid kodiert ist, das in Plasmid pGEM-T-Ppin13 enthalten ist, das in *E. coli* DSM 24144 enthalten ist.

**6.** Verfahren nach einem beliebigen der Ansprüche 1-5, weiterhin umfassend Gewinnen des abgebauten cellulosischen Materials.

**7.** Verfahren zum Herstellen eines Fermentationsprodukts, umfassend:

(a) Verzuckern eines cellulosischen Materials bei einer Temperatur im Bereich von etwa 50°C bis etwa 65°C mit einer Enzymzusammensetzung in der Gegenwart des Polypeptids mit Endoglucanaseaktivität wie in einem beliebigen der Ansprüche 1-5 definiert;
(b) Fermentieren des verzuckerten cellulosischen Materials mit einem oder mehreren fermentierenden Mikroorganismen, um das Fermentationsprodukt herzustellen; und
(c) Gewinnen des Fermentationsprodukts aus der Fermentation.

**8.** Verfahren zum Fermentieren eines cellulosischen Materials, umfassend: Fermentieren des cellulosischen Materials mit einem oder mehreren fermentierenden Mikroorganismen, wobei das cellulosische Material bei einer Temperatur im Bereich von etwa 50°C bis etwa 65°C mit einer Enzymzusammensetzung in der Gegenwart des Polypeptids mit Endoglucanaseaktivität wie in einem beliebigen der Ansprüche 1-5 definiert verzuckert wird.

**9.** Verfahren nach Anspruch 8, wobei das Fermentieren des cellulosischen Materials ein Fermentationsprodukt herstellt.

**10.** Verfahren nach Anspruch 9, weiterhin umfassend Gewinnen des Fermentationsprodukts aus der Fermentation.

## Revendications

**1.** Méthode de dégradation ou de conversion d'un matériau cellulosique, comprenant un traitement du matériau cellulosique à une température comprise entre environ 50°C et environ 65°C, avec une composition enzymatique en présence d'un polypeptide isolé ayant une activité endoglucanase choisi dans le groupe consistant en :

(a) un polypeptide présentant une identité de séquence d'au moins 90 %, par exemple au moins 91 %, au moins 92 %, au moins 93 %, au moins 94 %, au moins 95 %, au moins 96 %, au moins 97 %, au moins 98 %, au moins 99 %, ou 100 % avec le polypeptide mature de SEQ ID NO : 2 ;

(b) un polypeptide codé par un polynucléotide présentant une identité de séquence avec la séquence codante du polypeptide mature de SEQ ID NO : 1 d'au moins 90 %, par exemple, au moins 91 %, au moins 92 %, au moins 93 %, au moins 94 %, au moins 95 %, au moins 96 %, au moins 97 %, au moins 98 %, au moins 99 %, ou 100 % ;
(c) un variant du polypeptide mature de SEQ ID NO : 2 comprenant une substitution, une délétion, et/ou une insertion à une ou plusieurs positions, dans lequel le variant a une activité endoglucanase et dans lequel le nombre de substitutions, de délétions, et/ou d'insertions d'acides aminé introduites dans le polypeptide mature de SEQ ID NO : 2 est jusqu'à 10, par exemple 1, 2, 3, 4, 5, 6, 7, 8, 9, ou 10 ; et
(d) un fragment de polypeptide de (a), (b), ou (c) qui a une activité endoglucanase, dans lequel à 50, 55, 60 et 65 °C, le degré de conversion de la cellulose en glucose est supérieur à celui de la composition enzymatique sans endoglucanase.

**2.** Méthode selon la revendication 1, dans laquelle le polypeptide isolé comprend ou consiste en SEQ ID NO : 2 ou le polypeptide mature de celui-ci.

**3.** Méthode selon la revendication 1, dans laquelle le polypeptide isolé comprend les acides aminés 24 à 498 de SEQ ID NO : 2.

**4.** Méthode selon la revendication 1, dans laquelle le polypeptide isolé consiste en les acides aminés 24 à 498 de SEQ ID NO : 2.

**5.** Méthode selon la revendication 1, dans laquelle le polypeptide isolé est codé par le polynucléotide contenu dans le plasmide pGEM-T-Ppin13 qui est contenu dans *E. coli* DSM 24144.

**6.** Méthode selon l'une quelconque des revendications 1 à 5, comprenant en outre la récupération du matériau cellulosique dégradé.

**7.** Méthode de production d'un produit de fermentation, comprenant :

(a) une saccharification d'un matériau cellulosique à une température comprise entre environ 50°C et environ 65°C, avec une composition enzymatique en présence du polypeptide ayant une activité endoglucanase comme défini dans l'une quelconque des revendications 1 à 5 ;
(b) une fermentation du matériau cellulosique saccharifié avec un ou plusieurs micro-organismes de fermentation pour produire le produit de fermentation ; et
(c) une récupération du produit de fermentation à partir de la fermentation.

**8.** Méthode de fermentation d'un matériau cellulosique, comprenant une fermentation du matériau cellulosique avec un ou plusieurs micro-organismes de fermentation, dans laquelle le matériau cellulosique est saccharifié à une température comprise entre environ 50°C et environ 65°C, avec une composition enzymatique en présence du polypeptide ayant une activité endoglucanase comme défini dans l'une quelconque des revendications 1 à 5.

**9.** Méthode selon la revendication 8, dans laquelle la fermentation du matériau cellulosique produit un produit de fermentation.

**10.** Méthode selon la revendication 9, comprenant en outre la récupération du produit de fermentation à partir de la fermentation.

M S N M A T R P L A F A A I A A L F H H
1 ATGTCTAACA TGGCGACTAG ACCATTGGCT TTTGCAGCTA TTGCTGCTCT TTTCCACCAT
A A S Q Q A P T P D N L A S L P T W K C
61 GCCGCCTCAC AGCAGGCCCC TACCCCAGAT AATTTAGCTT CTCTACCGAC CTGGAAATGT
T T S G G C V Q Q S T S I V V D W V Y H
121 ACAACTTCCG GCGGCTGTGT TCAACAGTCG ACCTCTATTG TCGTGGATTG GGTGTATCAC
W I H T V N G S T S C T T S S G L D P T
181 TGGATCCACA CAGTCAATGG GAGCACATCG TGCACCACAT CTAGCGGATT GGACCCAACT
L C G T E E E C Y T N C E I S P A T Y D
241 TTATGTGGAA CGGAAGAGGA ATGCTATACA AACTGTGAAA TCTCACCTGC AACCTACGAT
G L G I K T S G N A L T L N Q Y V T S N
301 GGCCTCGGTA TAAAAACTTC TGGAAACGCT TTAACCCTCA ATCAATACGT CACAAGCAAC
G T T S N A S P R V Y L L D P A G K N Y
361 GGAACGACAA GTAACGCCTC TCCGCGTGTA TATCTTTTGG ATCCCGCCGG CAAGAATTAT
E M L Q L L G Q E I S F D V D A S N L P
421 GAGATGCTGC AGCTCCTCGG TCAAGAGATT AGCTTTGACG TAGATGCCTC CAATTTACCA
C G E N G A L Y L S E M D A T G G R S Q
481 TGTGGCGAAA ACGGGGCTCT TTATCTCTCT GAGATGGATG CGACTGGAGG TCGAAGCCAG
Y N P A G A S Y G S G Y C D A Q C G S S
541 TACAACCCTG CCGGAGCTTC ATACGGTTCC GGTTACTGTG ATGCTCAGTG TGGAAGTAGC
S W F N G S I N S A G L G S C C N E M D
601 AGCTGGTTTA ATGGCTCGAT TAATAGCGCT GGCCTTGGTT CTTGCTGTAA CGAAATGGAT
L W E A N G E A T A L T P H P C S V D G
661 CTCTGGGAAG CAAATGGCGA GGCAACTGCT TTGACACCTC ATCCATGCAG TGTCGATGGT
P Y G C S G S A C G S T G V C D K N G C
721 CCTTATGGCT GCTCTGGTAG CGCCTGTGGT TCGACTGGAG TGTGTGACAA GAATGGTTGC
G F N P Y A L G N H S Y Y G P G L T V D
781 GGATTCAATC CATATGCCCT TGGAAATCAC AGCTACTACG GCCCAGGTCT TACAGTGGAC
T S K P F T V T T Q F V T N D G T K T G
841 ACAAGCAAGC CCTTTACAGT TACGACACAG TTTGTGACCA ACGATGGCAC CAAGACCGGC
T L T E I R R S Y T Q N G K V I A N A V
901 ACCCTGACCG AAATTCGTCG ATCTTACACT CAGAATGGCA AGGTTATTGC GAATGCCGTT
A S S S S G F S G Q S S I T E S F C T A
961 GCATCCTCTT CGTCGGGGTT TTCAGGTCAA AGTTCTATCA CAGAGTCCTT CTGTACTGCG
M D S E A G T L G G L T T M G E A L G R
1021 ATGGACTCCG AAGCCGGGAC ACTGGGTGGT CTGACTACAA TGGGTGAGGC CCTTGGCCGT
G M V L I F S I W N D A G G Y M N W L D
1081 GGCATGGTTC TTATCTTCAG CATTTGGAAT GATGCAGGTG GATACATGAA CTGGCTGGAT
S G S S G P C S S T A G I P S T I Q A N
1141 AGTGGTAGCT CGGGCCCTTG CAGTAGTACT GCAGGAATTC CGTCCACCAT TCAGGCGAAT
D P G T S V T F S N I K W G D I G S T G
1201 GACCCCGGTA CTTCGGTTAC TTTCTCAAAC ATCAAGTGGG GTGATATTGG ATCTACAGGG
S G T G G S S S S S S S T S T S P K T T
1261 TCTGGCACTG GAGGAAGCAG TTCATCATCG TCGTCAACTT CGACCTCACC AAAAACTACC
S T T T T S A T T K T S A T T T T T S T
1321 AGCACCACAA CAACATCAGC AACGACCAAA ACATCAGCAA CGACAACTAC AACCAGCACA
G V T Q T H Y G Q C G G M Y Y T G P T V
1381 GGGGTAACTC AGACTCACTA TGGTCAATGT GGAGGCATGT ATTATACTGG TCCTACTGTT
C A S P Y T C Q V Q N P Y Y S Q C L *
1441 TGTGCCTCTC CGTACACCTG TCAAGTACAG AATCCGTACT ATTCACAGTG CCTTTAG

Fig. 1

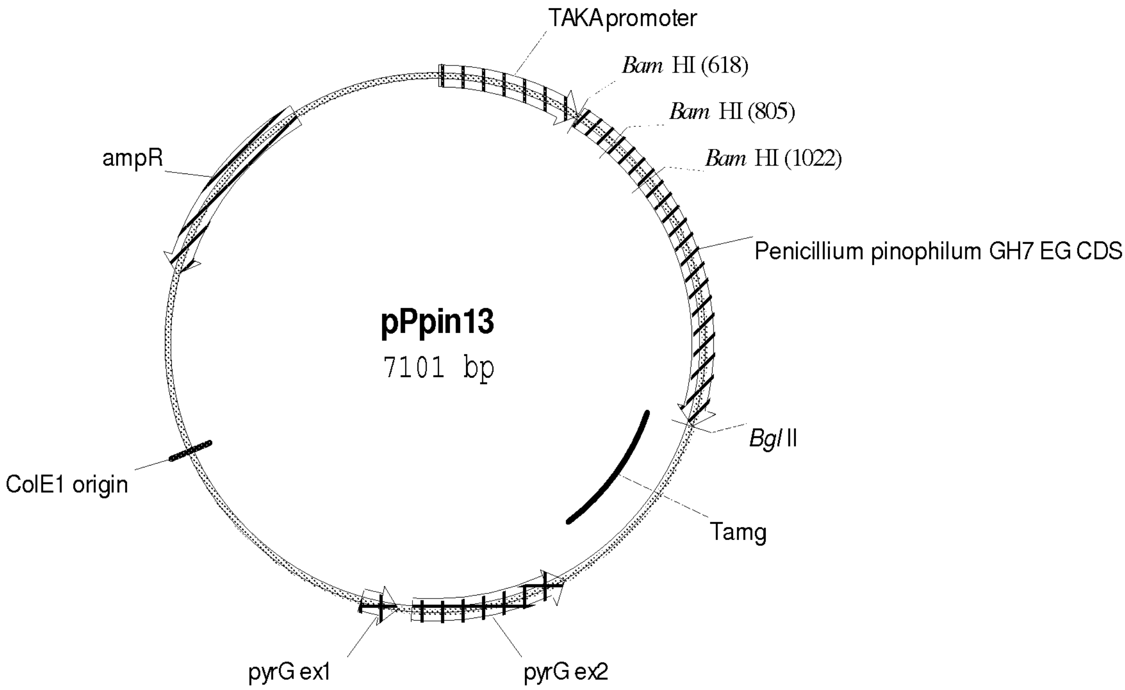
TAKA promoter
Bam HI (618)
Bam HI (805)
Bam HI (1022)
Penicillium pinophilum GH7 EG CDS
Bgl II
Tamg
pyrG ex2
pyrG ex1
ColE1 origin
ampR
pPpin13
7101 bp

# Fig. 2

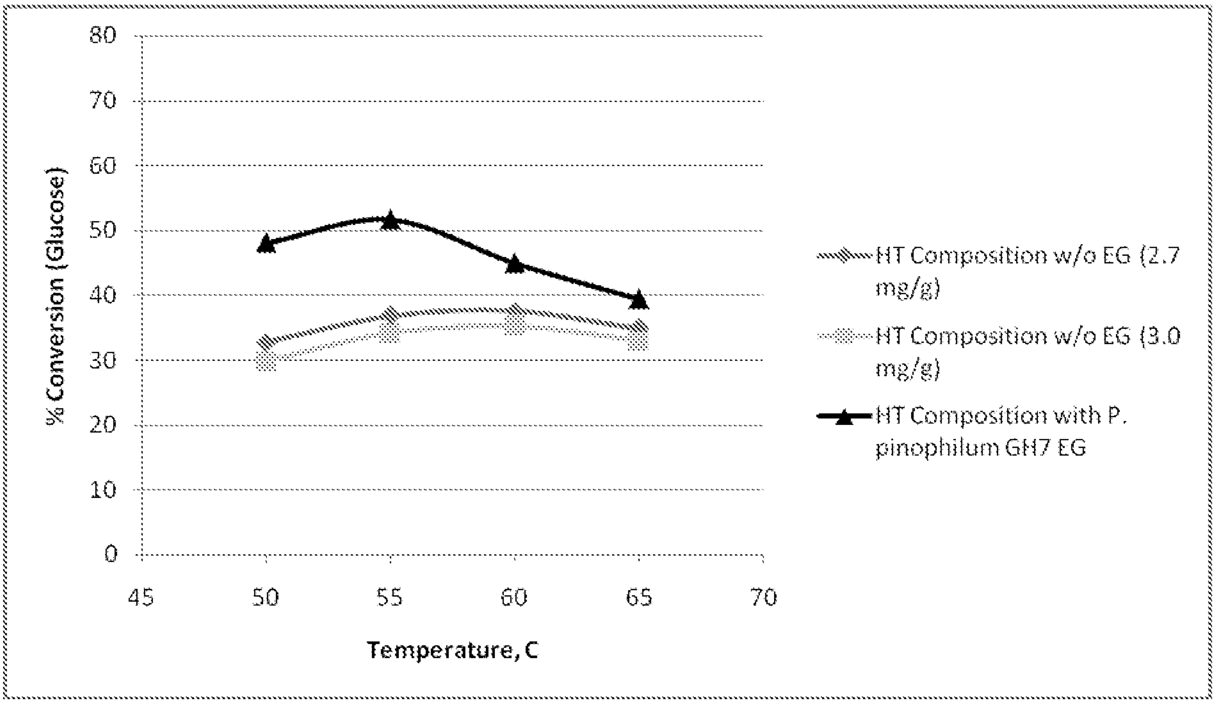
80
70
60
50
40
30
20
10
0
% Conversion (Glucose)
45
50
55
60
65
70
Temperature, C
HT Composition w/o EG (2.7 mg/g)
HT Composition w/o EG (3.0 mg/g)
HT Composition with P. pinophilum GH7 EG

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02095014 A
- WO 2002095014 A
- WO 9517413 A
- WO 9522625 A
- US 5223409 A
- WO 9206204 A
- WO 9943835 A
- WO 9600787 A
- WO 0056900 A
- US 6011147 A
- WO 9425612 A
- WO 9533836 A
- WO 2010039889 A
- WO 0024883 A
- EP 238023 A
- WO 9114772 A
- US 6395966 B
- US 7151204 B
- US 6489127 B
- US 6506559 B
- US 6511824 B
- US 6515109 B
- WO 9015861 A
- WO 2010096673 A
- US 20020164730 A
- WO 2006110891 A
- WO 2006110899 A
- WO 2006110900 A
- WO 2006110901 A
- WO 2006032282 A
- WO 9117243 A
- WO 9117244 A
- WO 9105039 A
- WO 9315186 A
- US 5275944 A
- WO 9602551 A
- US 5536655 A
- WO 0070031 A
- WO 05093050 A
- WO 2011059740 A
- WO 2009042871 A
- WO 2010141325 A
- WO 2006074435 A
- WO 2010057086 A
- WO 2005047499 A
- WO 2007019442 A
- WO 2010088387 A
- WO 2011035029 A
- WO 2008057637 A
- WO 9813465 A
- WO 98015619 A
- WO 98015633 A
- WO 9906574 A
- WO 9910481 A
- WO 99025847 A
- WO 99031255 A
- WO 2002101078 A
- WO 2003027306 A
- WO 2003052054 A
- WO 2003052055 A
- WO 2003052056 A
- WO 2003052057 A
- WO 2003052118 A
- WO 2004016760 A
- WO 2004043980 A
- WO 2004048592 A
- WO 2005001065 A
- WO 2005028636 A
- WO 2005093050 A
- WO 2005093073 A
- WO 2006074005 A
- WO 2006117432 A
- WO 2007071818 A
- WO 2007071820 A
- WO 2008008070 A
- WO 2008008793 A
- US 5457046 A
- US 5648263 A
- US 5686593 A
- WO 2005074647 A
- WO 2008148131 A
- WO 2011035027 A
- WO 2005074656 A
- WO 2010065830 A
- WO 2007089290 A
- WO 2009085935 A
- WO 2009085859 A
- WO 2009085864 A
- WO 2009085868 A
- WO 2010138754 A
- WO 2011005867 A
- WO 2011039319 A
- WO 2011041397 A
- WO 2011041504 A
- WO 2008151043 A
- WO 9421785 A
- WO 2006078256 A
- WO 2011041405 A

- WO 2010126772 A
- WO 2009079210 A
- WO 2011057083 A
- WO 2010108918 A
- WO 2009073709 A
- WO 2005001036 A
- WO 2010014880 A
- WO 2009042846 A
- WO 2009076122 A
- WO 2009127729 A
- WO 2010053838 A
- WO 2010065448 A
- WO 2006114094 A
- WO 2009073383 A
- WO 2010014706 A
- WO 2009068565 A
- WO 2003062430 A
- WO 2009037253 A
- WO 0177315 A
- WO 200177315 A
- WO 9535385 A
- WO 2011057140 A
- HR 2660
- WO 200039322 A
- CN 2010078675 W
- US 61434159 B

**Non-patent literature cited in the description**


- **DE VRIES.** *J. Bacteriol,* 1998, vol. 180, 243-249
- **VENTURI et al.** Extracellular beta-D-glucosidase from Chaetomium thermophilum var. coprophilum: production, purification and some biochemical properties. *J. Basic Microbiol,* 2002, vol. 42, 55-66
- **TEERI.** Crystalline cellulose degradation: New insight into the function of cellobiohydrolases. *Trends in Biotechnology,* 1997, vol. 15, 160-167
- **TEERI et al.** Trichoderma reesei cellobiohydrolases: why so efficient on crystalline cellulose?. *Biochem. Soc. Trans.,* 1998, vol. 26, 173-178
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **TOMME et al.** *Eur. J. Biochem.,* 1988, vol. 170, 575-581
- **ZHANG et al.** Outlook for cellulase improvement: Screening and selection strategies. *Biotechnology Advances,* 2006, vol. 24, 452-481
- **GHOSE.** Measurement of cellulase activities. *Pure Appl. Chem.,* 1987, vol. 59, 257-68
- **WISELOGEL et al.** Handbook on Bioethanol. 1995, 105-118
- **WASHINGTON D.C. ; WYMAN.** Bioresource Technology. Taylor & Francis, 1994, vol. 50, 3-16
- **LYND.** *Applied Biochemistry and Biotechnology,* 1990, vol. 24/25, 695-719
- Recent Progress in Bioconversion of Lignocellulosics. **MOSIER et al.** Advances in Biochemical Engineering/Biotechnology. Springer-Verlag, 1999, vol. 65, 23-40
- **ZHANG et al.** *Biotechnology Advances,* 2006, vol. 24, 452-481
- **GHOSE.** *Pure and Appl. Chem.,* 1987, vol. 59, 257-268
- **HENRISSAT B.** A classification of glycosyl hydrolases based on amino-acid sequence similarities. *Biochem. J.,* 1991, vol. 280, 309-316
- **HENRISSAT B. ; BAIROCH A.** Updating the sequence-based classification of glycosyl hydrolases. *Biochem. J.,* 1996, vol. 316, 695-696
- **SHALLOM, D. ; SHOHAM, Y.** Microbial hemicellulases. *Current Opinion In Microbiology,* 2003, vol. 6 (3), 219-228
- **GHOSE ; BISARIA.** *Pure & Appl. Chem.,* 1987, vol. 59, 1739-1752
- **NIELSEN et al.** *Protein Engineering,* 1997, vol. 10, 1-6
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet,* 2000, vol. 16, 276-277
- **RICE et al.** *EMBOSS: The European Molecular Biology Open Software Suite,* 2000
- **BIELY ; PUCHARD.** Recent progress in the assays of xylanolytic enzymes. *Journal of the Science of Food and Agriculture,* 2006, vol. 86 (11), 1636-1647
- **SPANIKOVA ; BIELY.** Glucuronoyl esterase - Novel carbohydrate esterase produced by Schizophyllum commune. *FEBS Letters,* 2006, vol. 580 (19), 4597-4601
- **HERRMANN ; VRSANSKA ; JURICKOVA ; HIRSCH ; BIELY ; KUBICEK.** The beta-D-xylosidase of Trichoderma reesei is a multifunctional beta-D-xylan xylohydrolase. *Biochemical Journal,* 1997, vol. 321, 375-381
- **BAILEY ; BIELY ; POUTANEN.** Interlaboratory testing of methods for assay of xylanase activity. *Journal of Biotechnology,* 1992, vol. 23 (3), 257-270
- **LEVER.** A new reaction for colorimetric determination of carbohydrates. *Anal. Biochem,* 1972, vol. 47, 273-279
- **SAMBROOK et al.** Molecular Cloning, A Laboratory Manual. Cold Spring Harbor, 1989

- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **DE VOS et al.** *Science,* 1992, vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS et al.** *Nature Biotechnology,* 1999, vol. 17, 893-896
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **INNIS et al.** PCR: A Guide to Methods and Application. Academic Press, 1990
- **FORD et al.** *Protein Expression and Purification,* 1991, vol. 2, 95-107
- **AGAISSE ; LERECLUS.** *Molecular Microbiology,* 1994, vol. 13, 97-107
- **EGON et al.** *Gene,* 1988, vol. 69, 301-315
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **GILBERT et al.** *Scientific American,* 1980, vol. 242, 74-94
- **ROMANOS et al.** *Yeast,* 1992, vol. 8, 423-488
- **HUE et al.** *Journal of Bacteriology,* 1995, vol. 177, 3465-3471
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS et al.** *Gene,* 1991, vol. 98, 61-67
- **CULLEN et al.** *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER et al.** *Nucleic Acids Res.,* 1988, vol. 16, 6127-6145
- **GONG et al.** *Folia Microbiol. (Praha),* 2004, vol. 49, 399-405
- **MAZODIER et al.** *J. Bacteriol.,* 1989, vol. 171, 3583-3585
- **BURKE et al.** *Proc. Natl. Acad. Sci. USA,* 2001, vol. 98, 6289-6294
- **CHOI et al.** *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY et al.** *Appl. Environ. Microbiol.,* 1999, vol. 65, 3800-3804
- **CLEWELL.** *Microbiol. Rev.,* 1981, vol. 45, 409-436
- **HAWKSWORTH et al.** Ainsworth and Bisby's Dictionary of The Fungi. CAB International, University Press, 1995
- App. Bacteriol. Symposium. 1980
- **YELTON et al.** *Proc. Natl. Acad. Sci. USA,* 1984, vol. 81, 1470-1474
- **CHRISTENSEN et al.** *Bio/Technology,* 1988, vol. 6, 1419-1422
- **MALARDIER et al.** *Gene,* 1989, vol. 78, 147-156
- Guide to Yeast Genetics and Molecular Biology, Methods in Enzymology. Academic Press, Inc, vol. 194, 182-187
- **ITO et al.** *J. Bacteriol.,* 1983, vol. 153, 163
- **HINNEN et al.** *Proc. Natl. Acad. Sci. USA,* 1978, vol. 75, 1920
- Protein Purification. VCH Publishers, 1989

- **TAGUE et al.** *Plant Physiology,* 1988, vol. 86, 506
- **FRANCK et al.** *Cell,* 1980, vol. 21, 285-294
- **CHRISTENSEN et al.** *Plant Mol. Biol.,* 1992, vol. 18, 675-689
- **ZHANG et al.** *Plant Cell,* 1991, vol. 3, 1155-1165
- **EDWARDS ; CORUZZI.** *Ann. Rev. Genet.,* 1990, vol. 24, 275-303
- **ITO et al.** *Plant Mol. Biol.,* 1994, vol. 24, 863-878
- **WU et al.** *Plant Cell Physiol.,* 1998, vol. 39, 885-889
- **CONRAD et al.** *J. Plant Physiol.,* 1998, vol. 152, 708-711
- **CHEN et al.** *Plant Cell Physiol.,* 1998, vol. 39, 935-941
- **KYOZUKA et al.** *Plant Physiol.,* 1993, vol. 102, 991-1000
- **MITRA ; HIGGINS.** *Plant Mol. Biol.,* 1994, vol. 26, 85-93
- **KAGAYA et al.** *Mol. Gen. Genet.,* 1995, vol. 248, 668-674
- **XU et al.** *Plant Mol. Biol.,* 1993, vol. 22, 573-588
- **GASSER et al.** *Science,* 1990, vol. 244, 1293
- **POTRYKUS.** *Bio/Technology,* 1990, vol. 8, 535
- **SHIMAMOTO et al.** *Nature,* 1989, vol. 338, 274
- **HOOYKAS ; SCHILPEROORT.** *Plant Mol. Biol.,* 1992, vol. 19, 15-38
- **CHRISTOU.** *Plant J.,* 1992, vol. 2, 275-281
- **SHIMAMOTO.** *Curr. Opin. Biotechnol.,* 1994, vol. 5, 158-162
- **VASIL et al.** *Bio/Technology,* 1992, vol. 10, 667-674
- **OMIRULLEH et al.** *Plant Mol. Biol.,* 1993, vol. 21, 415-428
- Cellulose bioconversion technology. **PHILIPPIDIS, G. P.** Handbook on Bioethanol: Production and Utilization,. Taylor & Francis, 1996, 179-212
- **SHEEHAN, J. ; HIMMEL, M.** Enzymes, energy and the environment: A strategic perspective on the U.S. Department of Energy's research and development activities for bioethanol. *Biotechnol. Prog.,* 1999, vol. 15, 817-827
- **WEIMER, P. J. ; VAN ZYL, W. H. ; PRETORIUS, I. S.** Microbial cellulose utilization: Fundamentals and biotechnology. *Microbiol. Mol. Biol. Reviews,* 2002, vol. 66, 506-577
- **FERNANDA DE CASTILHOS CORAZZA ; FLÁVIO FARIA DE MORAES ; GISELLA MARIA ZANIN ; IVO NEITZEL.** Optimal control in fed-batch reactor for the cellobiose hydrolysis. *Acta Scientiarum. Technology,* 2003, vol. 25, 33-38
- **GUSAKOV, A. V. ; SINITSYN, A. P.** Kinetics of the enzymatic hydrolysis of cellulose: 1. A mathematical model for a batch reactor process. *Enz. Microb. Technol.,* 1985, vol. 7, 346-352
- **RYU, S. K. ; LEE, J. M.** Bioconversion of waste cellulose by using an attrition bioreactor. *Biotechnol. Bioeng.,* 1983, vol. 25, 53-65
- **GUSAKOV, A. V. ; SINITSYN, A. P. ; DAVYDKIN, I. Y. ; DAVYDKIN, V. Y. ; PROTAS, O. V.** Enhancement of enzymatic cellulose hydrolysis using a novel type of bioreactor with intensive stirring induced by electromagnetic field. *Appl. Biochem. Biotechnol.,* 1996, vol. 56, 141-153
- **CHANDRA et al.** Substrate pretreatment: The key to effective enzymatic hydrolysis of lignocellulosics?. *Adv. Biochem. Engin./Biotechnol.,* 2007, vol. 108, 67-93
- **GALBE ; ZACCHI.** Pretreatment of lignocellulosic materials for efficient bioethanol production. *Adv. Biochem. Engin./Biotechnol.,* 2007, vol. 108, 41-65
- **HENDRIKS ; ZEEMAN.** Pretreatments to enhance the digestibility of lignocellulosic biomass. *Bioresource Technol.,* 2009, vol. 100, 10-18
- **MOSIER et al.** Features of promising technologies for pretreatment of lignocellulosic biomass. *Bioresource Technol.,* 2005, vol. 96, 673-686
- **TAHERZADEH ; KARIMI.** Pretreatment of lignocellulosic wastes to improve ethanol and biogas production: A review. *Int. J. of Mol. Sci.,* 2008, vol. 9, 1621-1651
- **YANG ; WYMAN.** Pretreatment: the key to unlocking low-cost cellulosic ethanol. *Biofuels Bioproducts and Biorefining-Biofpr.,* 2008, vol. 2, 26-40
- **DUFF ; MURRAY.** *Bioresource Technology,* 1996, vol. 855, 1-33
- **GALBE ; ZACCHI.** *Appl. Microbiol. Biotechnol.,* 2002, vol. 59, 618-628
- **BALLESTEROS et al.** *Appl. Biochem. Biotechnol.,* 2006, vol. 129-132, 496-508
- **VARGA et al.** *Appl. Biochem. Biotechnol.,* 2004, vol. 113-116, 509-523
- **SASSNER et al.** *Enzyme Microb. Technol.,* 2006, vol. 39, 756-762
- **SCHELL et al.** *Bioresource Technol.,* 2004, vol. 91, 179-188
- **LEE et al.** *Adv. Biochem. Eng. Biotechnol.,* 1999, vol. 65, 93-115
- **WYMAN et al.** *Bioresource Technol.,* 2005, vol. 96, 1959-1966
- **MOSIER et al.** *Bioresource Technol.,* 2005, vol. 96, 673-686
- **SCHMIDT ; THOMSEN.** *Bioresource Technol.,* 1998, vol. 64, 139-151
- **PALONEN et al.** *Appl. Biochem. Biotechnol.,* 2004, vol. 117, 1-17
- **VARGA et al.** *Biotechnol. Bioeng.,* 2004, vol. 88, 567-574

- **MARTIN et al.** *J. Chem. Technol. Biotechnol.,* 2006, vol. 81, 1669-1677
- **GOLLAPALLI et al.** *Appl. Biochem. Biotechnol.,* 2002, vol. 98, 23-35
- **CHUNDAWAT et al.** *Biotechnol. Bioeng.,* 2007, vol. 96, 219-231
- **ALIZADEH et al.** *Appl. Biochem. Biotechnol.,* 2005, vol. 121, 1133-1141
- **TEYMOURI et al.** *Bioresource Technol.,* 2005, vol. 96, 2014-2018
- **PAN et al.** *Biotechnol. Bioeng.,* 2005, vol. 90, 473-481
- **PAN et al.** *Biotechnol. Bioeng.,* 2006, vol. 94, 851-861
- **KURABI et al.** *Appl. Biochem. Biotechnol.,* 2005, vol. 121, 219-230
- **SCHELL et al.** *Appl. Biochem. and Biotechnol.,* 2003, vol. 105-108, 69-85
- **MOSIER et al.** *Bioresource Technology,* 2005, vol. 96, 673-686
- Pretreatment of biomass. **HSU, T.-A.** Handbook on Bioethanol: Production and Utilization. Taylor & Francis, 1996, 179-212
- **GHOSH ; SINGH.** Physicochemical and biological treatments for enzymatic/microbial conversion of cellulosic biomass. *Adv. Appl. Microbiol.,* 1993, vol. 39, 295-333
- Pretreating lignocellulosic biomass: a review, in Enzymatic Conversion of Biomass for Fuels Production. **MCMILLAN, J. D.** ACS Symposium. American Chemical Society, 1994
- Ethanol production from renewable resources. **GONG, C. S. ; CAO, N. J. ; DU, J. ; TSAO, G. T.** Advances in Biochemical Engineering/Biotechnology. Springer-Verlag, 1999, vol. 65, 207-241
- **OLSSON ; HAHN-HAGERDAL.** Fermentation of lignocellulosic hydrolysates for ethanol production. *Enz. Microb. Tech.,* 1996, vol. 18, 312-331
- **VALLANDER ; ERIKSSON.** Production of ethanol from lignocellulosic materials: State of the art. *Adv. Biochem. Eng./Biotechnol.,* 1990, vol. 42, 63-95
- **PENTTILA et al.** *Gene,* 1986, vol. 45, 253-263
- **SALOHEIMO et al.** *Gene,* 1988, vol. 63, 11-22
- **OKADA et al.** *Appl. Environ. Microbiol.,* 1988, vol. 64, 555-563
- **SALOHEIMO et al.** *Molecular Microbiology,* 1994, vol. 13, 219-228
- **OOI et al.** *Nucleic Acids Research,* 1990, vol. 18, 5884
- **SAKAMOTO et al.** *Current Genetics,* 1995, vol. 27, 435-439
- **SAARILAHTI et al.** *Gene,* 1990, vol. 90, 9-14
- **KAWAGUCHI et al.** *Gene,* 1996, vol. 173, 287-288
- **DAN et al.** *J. Biol. Chem.,* 2000, vol. 275, 4973-4980
- More Gene Manipulations in Fungi. Academic Press, 1991
- **BAILEY, J.E. ; OLLIS, D.F.** Biochemical Engineering Fundamentals. McGraw-Hill Book Company, 1986
- **LIN et al.** *Appl. Microbiol. Biotechnol.,* 2006, vol. 69, 627-642
- **CHEN ; HO.** Cloning and improving the expression of Pichia stipitis xylose reductase gene. *Saccharomyces cerevisiae, Appl. Biochem. Biotechnol.,* 1993, vol. 39-40, 135-147
- **HO et al.** Genetically engineered Saccharomyces yeast capable of effectively cofermenting glucose and xylose. *Appl. Environ. Microbiol.,* 1998, vol. 64, 1852-1859
- **KOTTER ; CIRIACY.** Xylose fermentation by Saccharomyces cerevisiae. *Appl. Microbiol. Biotechnol.,* 1993, vol. 38, 776-783
- **WALFRIDSSON et al.** Xylose-metabolizing Saccharomyces cerevisiae strains overexpressing the TKL1 and TAL1 genes encoding the pentose phosphate pathway enzymes transketolase and transaldolase. *Appl. Environ. Microbiol.,* 1995, vol. 61, 4184-4190
- **KUYPER et al.** Minimal metabolic engineering of Saccharomyces cerevisiae for efficient anaerobic xylose fermentation: a proof of principle. *FEMS Yeast Research,* 2004, vol. 4, 655-664
- **BEALL et al.** Parametric studies of ethanol production from xylose and other sugars by recombinant. *Escherichia coli, Biotech. Bioeng.,* 1991, vol. 38, 296-303
- **INGRAM et al.** Metabolic engineering of bacteria for ethanol production. *Biotechnol. Bioeng.,* 1998, vol. 58, 204-214
- **ZHANG et al.** Metabolic engineering of a pentose metabolism pathway in ethanologenic. *Zymomonas mobilis, Science,* 1995, vol. 267, 240-243
- **DEANDA et al.** Development of an arabinose-fermenting Zymomonas mobilis strain by metabolic pathway engineering. *Appl. Environ. Microbiol.,* 1996, vol. 62, 4465-4470
- The Alcohol Textbook. Nottingham University Press, 1999
- **ALFENORE et al.** Improving ethanol production and viability of Saccharomyces cerevisiae by a vitamin feeding strategy during fed-batch process. Springer-Verlag, 2002
- **SILVEIRA, M. M. ; JONAS, R.** The biotechnological production of sorbitol. *Appl. Microbiol. Biotechnol.,* 2002, vol. 59, 400-408
- **NIGAM, P. ; SINGH, D.** Processes for fermentative production of xylitol - a sugar substitute. *Process Biochemistry,* 1995, vol. 30 (2), 117-124

- **EZEJI, T. C. ; QURESHI, N. ; BLASCHEK, H. P.** Production of acetone, butanol and ethanol by Clostridium beijerinckii BA101 and in situ recovery by gas stripping. *World Journal of Microbiology and Biotechnology,* 2003, vol. 19 (6), 595-603
- **RICHARD, A. ; MARGARITIS, A.** Empirical modeling of batch fermentation kinetics for poly(glutamic acid) production and other microbial biopolymers. *Biotechnology and Bioengineering,* 2004, vol. 87 (4), 501-515
- **KATAOKA, N. ; A. MIYA ; K. KIRIYAMA.** Studies on hydrogen production by continuous culture system of hydrogen-producing anaerobic bacteria. *Water Science and Technology,* 1997, vol. 36 (6-7), 41-47
- **GUNASEELAN V.N.** *Biomass and Bioenergy,* 1997, vol. 13 (1-2), 83-114
- **CHEN, R. ; LEE, Y. Y.** Membrane-mediated extractive fermentation for lactic acid production from cellulosic biomass. *Appl. Biochem. Biotechnol.,* 1997, vol. 63-65, 435-448
- **EWING et al.** *Genome Research,* 1998, vol. 8, 175-185
- **EWING ; GREEN.** *Genome Research,* 1998, vol. 8, 186-194
- *Build linux-x86,* 30 July 1998, vol. 18 (51), 44
- **GISH et al.** *Nat. Genet.,* 1993, vol. 3, 266-72